(19) Europäisches Patentamt
European Patent Office
Office européen des brevets

(11) EP 2 075 245 B1

(12) **EUROPEAN PATENT SPECIFICATION**

(45) Date of publication and mention of the grant of the patent:
**31.12.2014 Bulletin 2015/01**

(51) Int Cl.:
***C07C 225/22*** *(2006.01)* ***C07C 237/04*** *(2006.01)*
***G01N 21/64*** *(2006.01)* ***G01N 33/58*** *(2006.01)*

(21) Application number: **08163077.4**

(22) Date of filing: **27.08.2008**

(54) **Two-photon probe for real-time monitoring of intracellular calcium ions, method for preparing the probe and method for real-time monitoring of intracellular calcium ions using the probe**

Zweiphotonensonde zur Echtzeitüberwachung intrazellulärer Calciumionen, Verfahren zur Herstellung der Sonde und Verfahren zur Echtzeitüberwachung der intrazellulären Calciumionen unter Verwendung der Sonde

Sonde à deux photons pour la surveillance en temps réel d'ions calcium intracellulaires, procédé de préparation de la sonde et procédé pour la surveillance en temps réel d'ions calcium intracellulaires à l'aide de la sonde

(84) Designated Contracting States:
**DE FR GB**

(30) Priority: **07.09.2007 KR 20070090726**

(43) Date of publication of application:
**01.07.2009 Bulletin 2009/27**

(73) Proprietor: **Korea University Industry and Academy Cooperation Foundation**
**Seoul 136-701 (KR)**

(72) Inventor: **Cho, Bong-Rae**
**138-170 Seoul (KR)**

(74) Representative: **Grosse Schumacher Knauer von Hirschhausen**
**Patent- und Rechtsanwälte**
**Nymphenburger Strasse 14**
**80335 München (DE)**

(56) References cited:
**US-A1- 2006 024 833 US-A1- 2008 293 088**

- **HWAN MYUNG KIM ET AL: "A Two-Photon Fluorecent Probe for Calcium Waves in Living" ANGEW. CHEM. IND. ED., vol. 46, no. 39, 2007, pages 7445-7448, XP002554198**
- **HWAN MYUNG KIM ET AL: "Environment-Sensitive Two-Photon Probe for Intracellular Free Magnesium Ions in Live tissue" ANGEW. CHEM. INT. ED., vol. 46, no. 19, 2007, pages 3460-3469, XP002554199**

EP 2 075 245 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention relates to a two-photon probe for real-time monitoring of intracellular calcium ions, a method for preparing the two-photon probe, and a method for real-time monitoring of intracellular calcium ions using the two-photon probe. More particularly, the present invention relates to a two-photon probe suitable for real-time imaging of intracellular calcium ions due to its high two-photon absorption efficiency, ability to selectively recognize the membrane and calcium ions and high photostability, a method for preparing the two-photon probe, and a method for real-time monitoring of intracellular calcium ions using the two-photon probe.

Calcium is a versatile intracellular signal messenger controlling numerous cellular functions. The $Ca^{2+}$-signalling system operates in many different ways to regulate various cellular processes that function over a wide dynamic range. Calcium triggers exocytosis within microseconds and drives the gene transcription and proliferation in minutes to hours.

To understand these functions, fluorescence imaging with fluorescent probes such as Oregon Green 488 BAPTA-1 (OG1) and fura-2 have most often been used. However, use of these probes with one-photon microscopy requires excitation with short wavelength light (-350-500 nm) that limits their application in tissue imaging owing to shallow penetration depth (< 100 $\mu$m), photobleaching, photodamage, and cellular auto fluorescence.

Two-photon microscopy (TPM) overcomes these shortcomings. One-photon microscopy (OPM) employs one high-energy photon for excitation, whereas TPM employs two lower energy, near-infrared photons to produce an excited fluorescent substance. TPM has the advantages of localized excitation, increased penetration depth (> 500 $\mu$m), lower cellular autofluorescence and self-absorption, as well as reduced photodamage and photobleaching, when compared to OPM. Thus, TPM allows imaging deep inside tissues for a long period of time without interference from artifacts of surface preparation that can extend > 70 $\mu$m into the tissue slice interior.

However, most of the fluorescent probes presently used for TPM have small TP action cross sections ($\Phi\delta$), demanding impractically high concentrations of probe and/or laser power. Furthermore, the fluorescence signals from membrane-bound probes can cause significant errors such as mistargeting because the fluorescence quantum yield is higher in the membrane than in the cytosol.

Hwan Myung Kim et al., "Environment-Sensitive Two-Photon Pobe for Intercellular Free Magnesium Ions in Live tissue" Angew. Chem. Int. Ed., vol. 46, No. 19, 2007, pages 3460-3469, describes a TPM employing two lower energy, near infrared photons for excitation for detecting magnesium ions.

US 2006/024833 describes a two photon probe for real-time monitoring of intracellular magnesium ions, a method for preparing the two-photon probe and a method for real-time monitoring of intracellular magnesium ions using the two photon probe. Hwan Myung Kim et al., "A Two-Photon Fluorescent probe for Calcium Waves in Living", Angew. Chem. Int. Ed., vol. 46, No. 39, 2007, pages 7445-7448, describe a TPM, namely ACa1 for intracellular free Calcium Ions.

There is a need to develop an efficient TP probe that can visualize the calcium waves deep inside the live tissue without photobleaching or mistargeting problems.

Therefore, it is a first object of the present invention to provide a two-photon (TP) probe that has the advantages of significant TP cross section for bright TPM image at low probe concentration, high selectivity for $Ca^{2+}$ ions, possible discrimination between the cytosolic and membrane-bound probes due to different emission spectra arising from the polarity of environments, and high photostability, thus being suitable for real-time imaging of intracellular calcium ions.

It is a second object of the present invention to provide a method for preparing the two-photon probe.

It is a third object of the present invention to provide a method for real-time monitoring of intracellular calcium ions using the two-photon probe.

In accordance with one aspect of the present invention, the first object can be accomplished by the provision of a two-photon probe for real-time monitoring of intracellular calcium ions, represented by Formula 1:

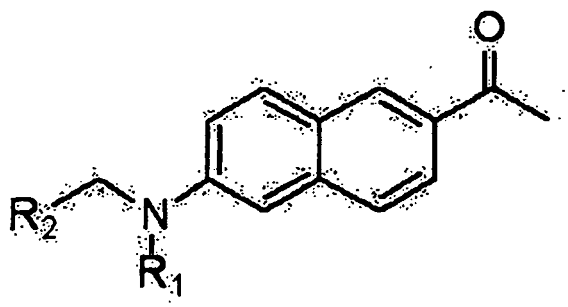

**(1)**

wherein $R_1$ is $CH_3$ or H, and

$R_2$ is

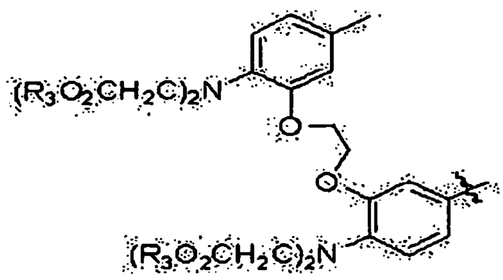

($R_3$ = H or $CH_2OCOCH_3$).

In accordance with another aspect of the present invention, the second object can be accomplished by the provision of a method for preparing the two-photon probe for real-time monitoring of intracellular calcium ions, the method comprising reacting a compound of Formula 2:

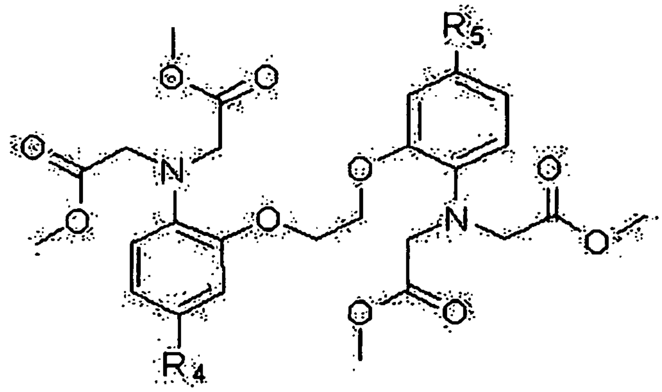

**(2)**

wherein $R_4$ is $NH_2$ or CHO and $R_5$ is H or $CH_3$, with a compound of Formula 3:

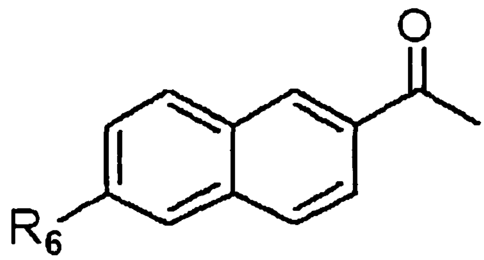

**(3)**

wherein $R_6$ is $NH_2$ or

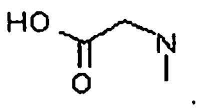

In accordance with yet another aspect of the present invention, the third object can be accomplished by the provision of a method for real-time monitoring of intracellular calcium ions, the method comprising the steps introducing the two-photon probe into cells of interest and imaging two-photon excited fluorescence emitted from the two-photon probe.

In an embodiment, the intracellular calcium ion concentration may be quantitatively determined by Equation 1:

$$[Ca^{2+}] = K_d[(F - F_{min})/(F_{max} - F)] \qquad (1)$$

where $K_d$ is the dissociation constant of the two-photon probe for $Ca^{2+}$, F is the observed two-photon fluorescence intensity, $F_{min}$ is the minimum fluorescence intensity, and $F_{max}$ is the maximum fluorescence intensity.

In another embodiment, the two-photon excited fluorescence images may be collected at wavelengths ranging from 500 to 620 nm.

The two-photon probe of the present invention is suitable for real-time imaging of intracellular calcium ions because it has the advantages of significant TP cross section for bright TPM image at low probe concentration, high selectivity for $Ca^{2+}$ ions, possible discrimination between the cytosolic and membrane-bound probes due to different emission spectra with the polarity of environments (*e.g.*, hydrophilic and hydrophobic environments), and high photostability.

The two-photon probe of the present invention uses 2-acetyl-6-(dimethylamino)naphthalene as the TP chromophore and O,O'-bis(2-aminophenyl)ethyleneglycol-N,N,N',N'-tetraacetic acid (BAPTA) as the $Ca^{2+}$ ion chelator.

The two-photon probe of the present invention shows significant TP cross section and large spectral shifts with the solvent polarity, allowing the detection of the two-photon excited fluorescence (TPEF) of the probe-$Ca^{2+}$ complex separately from that of membrane-bound probes. In addition, the two-photon probe of the present invention is capable of imaging the calcium waves in live cells and living tissue at > 100 $\mu$m depth for a long period of time without mistargeting and photobleaching problems.

Considering the cell permeability of the two-photon probe according to the present invention, $R_3$ in Formula 1 is preferably $CH_2OCOCH_3$. The replacement of the hydrogen atoms with $CH_2OCOCH_3$ in $R_3$ is performed by reacting bromomethyl acetate and triethylamine with the compound of Formula 1 ($R_3$ = H).

In the real-time monitoring method of the present invention, the two-photon excited fluorescence images can be collected using wavelengths between 500 nm and 620 nm and intracellular free $Ca^{2+}$ only can be selectively detected with minimum contribution from the membrane-bound two-photon probes, as will be described below.

Unlike prior art methods, the intracellular calcium ions can be quantitatively detected as well as qualitatively analyzed by the real-time monitoring method of the present invention.

The two-photon probe of the present invention is very suitable for real-time imaging of intracellular calcium ions, shows 44-fold TPEF enhancement in response to $Ca^{2+}$, has a dissociation constant ($K_d^{TP}$) of 0.25 $\pm$ 0.03 $\mu$M, and emits 5-fold stronger TPEF than Oregon Green 488 BAPTA-1 (OG1) upon complexation with $Ca^{2+}$. Unlike the previously available probes, the two-photon probe of the present invention can selectively detect dynamic levels of intracellular free $Ca^{2+}$ in live cells and living tissues without interference from other metal ions and from the membrane-bound probes. Moreover, the two-photon probe of the present invention is capable of monitoring the calcium waves at a depth of 100-300 $\mu$m in live tissues for longer than 1,100 s using TPM with no artifacts of photobleaching.

FIG. 1 shows absorption and emission spectra of two-photon probes ACa1 (1a, 1b), ACa2 (1c, 1d) and ACa3 (1e, 1f) in 1,4-dioxane, DMF, ethanol and $H_2O$;

FIG. 2 shows one-photon emission and absorption spectra of two-photon probes ACa1 (2a, 2b), ACa2 (2c, 2d) and ACa3 (2e, 2f) in the presence of free $Ca^{2+}$ at various concentrations;

FIG. 3 shows two-photon action spectra of two-photon probes ACa1 (3a) and ACa2 (3b) in the presence of free $Ca^{2+}$ at various concentrations;

FIG. 4 shows two-photon emission spectra of two-photon probes ACa1 (4a), ACa2 (4b) and ACa3 (4c) in the presence of free $Ca^{2+}$ at various concentrations;

FIG. 5 shows linear Hill plots for the complexation of two-photon probes ACa1 (5a), ACa2 (5b) and ACa3 (5c) with $Ca^{2+}$;

FIG. 6 shows titration curves of two-photon probes ACa1 (6a), ACa2 (6b) and ACa3 (6c), which were fitted to Equation 5;

FIG. 7 shows one-photon emission spectra of ACa1 in the presence of free $Mg^{2+}$ at various concentrations;

FIG. 8 is a linear Hill plot for the complexation of ACa1 with free $Mg^{2+}$ at various concentrations;

FIG. 9 is a one-photon fluorescence titration curve for the complexation of ACa1 with free $Mg^{2+}$ at various concentrations;

FIG. 10 shows graphs illustrating the reactivity of ACa1 (10a), ACa2 (10b) and ACa3 (10c) for various metal ions;

FIG. 11 shows graphs illustrating the effect of pH on the reactivity of two-photon probes ACa1 (11a), ACa2 (11b) and

ACa3 (11c);

FIG. 12 shows pseudocolored TPM images of cultured astrocytes labeled with two-photon probes ACa1-AM (12a), ACa2-AM (12b), and two-photon excited fluorescence spectra from the hydrophobic and hydrophilic domains of the respective astrocytes;

FIG. 13 shows a one-photon fluorescence intensity image (13a) and a pseudocolored lifetime image (13b) of ACa1-AM-labeled astrocytes, lifetime distribution (13c) of the astrocytes, and results (13d) of single-point analysis of the region indicated by the white arrow;

FIG. 14 shows TPEF images of ACa1-AM collected at 500-620 nm (14a) and 360-460 nm (14b);

FIG. 15 shows pseudocolored TPM Images of astrocytes labeled with the compound of Formula 7, which were taken after 110s (15a) and 220s (15b), and time courses (15c, 15d) of the calcium waves in different locations of the astrocytes;

FIG. 16 shows pseudocolored TPM images of an acute rat hypothalamic slice stained with ACa1-AM, which were taken after 195 s (16a) and 214 s (16b);

FIG. 17 shows spontaneous $Ca^{2+}$ transients recorded in soma (1), astrocyte process (2), and neighboring cell (3);

FIG. 18 shows a one-photon fluorescence intensity image (18a) and a pseudocolored lifetime image (18b) of ACa2-AM-labeled astrocytes, lifetime distribution (18c) of the astrocytes, and results (18d) of single-point analysis of the region indicated by the white arrow;

FIG. 19 shows pseudocolored TPM images of ACa2-AM-labeled astrocytes collected at 360-460 nm (19a) and 500-620 nm (19b);

FIG. 20 shows a pseudocolored TPM image (20a) of ACa2-AM-labeled astrocytes taken after 20s, and time courses of the calcium waves in different locations showing the spontaneous intracellular (20b) and intercellular (20c) $Ca^{2+}$ propagation; and

FIG. 21 shows pseudocolored TPM images of an acute rat hypothalamic slice stained with ACa2-AM taken after 40s (21a) and 1460s (21b), and spontaneous $Ca^{2+}$ transients recorded in the cells (21c).

Hereinafter, the present invention will be explained in more detail with reference to the following examples. However, these examples serve to provide further appreciation of the invention but are not meant in any way to restrict the scope of the invention.

EXAMPLES

Preparative Example 1: Synthesis of two-photon probe

In this example, the compound of Formula 4 was synthesized by the following procedure.

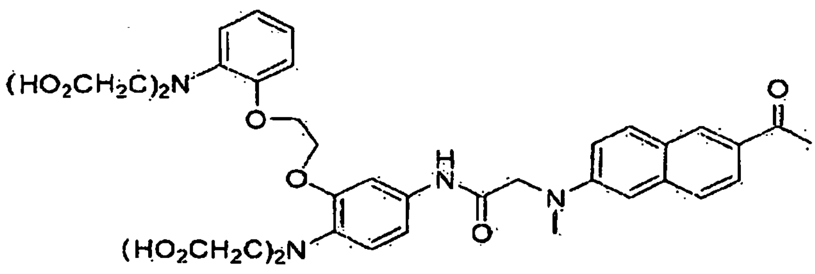

(4)

Preparative Example 1.1: Preparation of 5-nitro-BAPTA-tetramethyl ester (Formula 5) and 6-acetyl-2-[N-methyl-N-(carboxymethyl)amino]naphthalene (Formula 6)

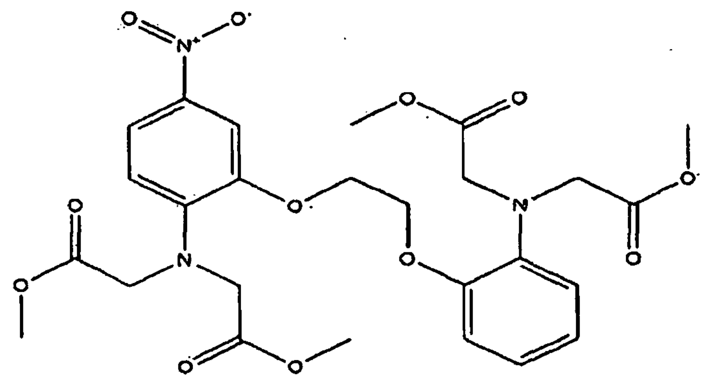

(5)

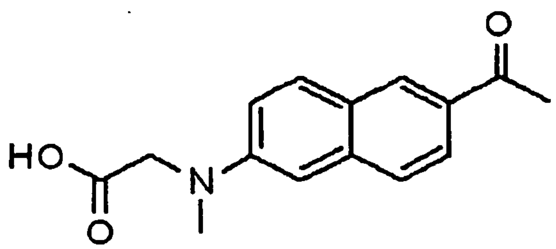

(6)

The compounds were prepared by the literature methods (R. Pethig, M. Kuhn, R. Payne, E. Adler, T.-H. Chen, L. F. Jaffe, Cell Calcium 1989, 10, 491-498 and H. M. Kim, C. Jung, B. R. Kim, S.-Y. Jung, J. H. Hong, Y.-G. Ko, K. J. Lee, B. R. Cho, Angew. Chem. Int. Ed. 2007, 46, 3460-3463).

Preparative Example 1.2: Preparation of 5-amino-BAPTA-tetramethyl ester (Formula 2)

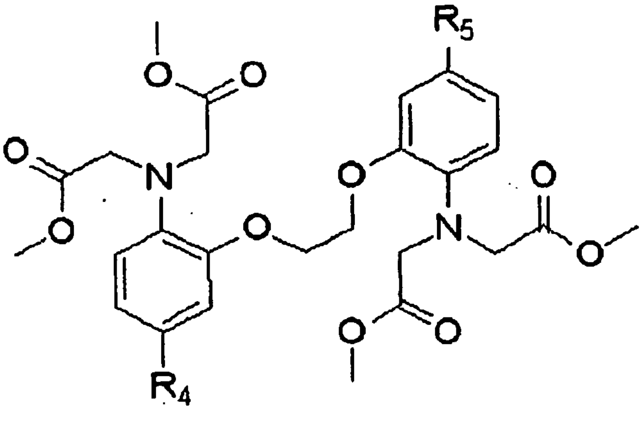

(2)

($R_4 = NH_2$, $R_5$ = H).

A mixture of the compound (2.2g, 3.8 mmol) of Formula 5 and 5% Pd on carbon (90 mg) in ethanol was shaken under hydrogen for 5 h. The reaction mixture was filtered and washed with hot ethanol, and the solvent was removed in vacuo. The product was purified by column chromatography using ethyl acetate/hexane (2:1) as the eluent.

Yield: 1.1g (53%); mp 121°C; IR (KBr): 3438, 3346, 1753 $cm^{-1}$; $^1H$ NMR (300 MHz, $CDCl_3$):

δ 6.87 (m, 4H), 6.77 (d, 1H, *J* = 9 Hz), 6.28 (d, 1H, *J* = 3.0 Hz), 6.22 (dd, 1H, *J* = 9, *J* = 3 Hz), 4.26 (m, 4H), 4.15 (s, 4H), 4.06 (s, 4H), 3.59 (s, 6H), 3.56 (s, 6H), 3.51 (br s, 2H); $^{13}C$ NMR (100 MHz, $CDCl_3$): δ = 172.4, 172.3, 152.2, 150.6, 142.7, 139.4, 131.5, 122.5, 121.6, 119.2, 113.3, 113.2, 107.8. 101.8, 67.3. 67.2, 53.9, 53.5, 51.9, 51.7 ppm; Anal. Calcd for $C_{26}H_{33}N_3O_{10}$: C, 57.03; H, 6.07; N, 7.67. Found: C, 57.11; H, 6.05; N, 7.60.

Preparative Example 1.3: Preparation of compound of Formula 4

A mixture of the compound (0.38g, 1.48 mmol) of Formula 6 and 1-ethyl-3-(3-dimethylaminopropyl)-carbodiimide HCl (0.34g, 1.80 mmol) in DMF (5 mL) was stirred for 20 min. To this mixture, the compound (0.90g, 1.64 mmol) of Formula 2 and 4-dimethylaminopyridine (26 mg, 0.22 mmol) were added and stirred for 12 h under $N_2$. The product was extracted with chloroform, dried over $MgSO_4$, and the solvent was removed in vacuo. The product was purified by column chromatography using hexane/ethyl acetate (2:3) as the eluent. It was further purified by recrystallization from EtOH.

Yield: 0.62g (53%); mp 148°C; IR (KBr): 3260, 1755, 1662 $cm^{-1}$; $^1H$ NMR (400 MHz, $CDCl_3$): $\delta$ 8.35 (d, 1H, *J* = 2 Hz), 8.18 (s, 1H), 7.98 (dd, 1H, *J* = 9, *J* = 2 Hz), 7.87 (d, 1H, *J* = 9 Hz), 7.70 (d, 1H, *J* = 9 Hz), 7.27 (d, 1H, *J* = 2 Hz), 7.16 (dd, 1H, *J* = 9, *J* = 2 Hz), 7.06 (d1H, *J* = 2.0 Hz), 6.88 (m, 6H), 4.25 (s, 4H), 4.12 (s, 4H), 4.10 (s, 6H), 3.56 (s, 6H), 3.53 (s, 6H), 3.24 (s, 3H), 2.68 (s, 3H); $^{13}C$ NMR (100 MHz, $CDCl_3$): $\delta$ = 198.0, 172.2, 172.0, 168.0, 150.8, 150.5, 149.3, 139.4, 137.3, 136.4, 132.3, 132.2, 131.6, 130.4, 127.0, 126.6, 125.2, 122.5, 121.7, 119.4, 119.2, 116.7, 113.3, 112.9, 107.7, 106.1, 77.4, 67.5, 67.1, 59.5, 53.6, 51.9, 51.8, 40.4, 26.8 ppm; Anal. Calcd for $C_{41}H_{46}N_4O_{12}$: C, 62.59; H, 5.89; N, 7.12. Found: C, 62.52; H, 5.93; N, 7.07.

This ester (0.50g, 0.64 mmol) was hydrolyzed by the method as described above. The resulting precipitate was collected, washed with distilled water, and purified by crystallization from $MeOHCHCl_3$-petroleum ether.

Yield: 0.27g (58%); mp 145°C; IR (KBr): 3250, 2910, 1745, 1660 $cm^{-1}$; H NMR (400 MHz, $CD_3OD$): $\delta$ 8.39 (d, 1H, *J* = 2 Hz), 7.86 (d, 1H, *J* = 9 Hz), 7.85 (dd, 1H, *J* = 9, *J* = 2 Hz), 7.65 (d, 1H, *J* = 9Hz), 7.46 (d, 1H, *J* = 2 Hz), 7.24 (dd, 1H, *J* = 9, *J* = 2 Hz), 6.96 (m, 7H), 4.31 (s, 4H), 4.30 (s, 2H), 3.91 (s, 8H), 3.24 (s, 3H), 2.63 (s, 3H); $^{13}C$ NMR (100 MHz, $CD_3OD$): $\delta$ = 199.2, 173.9, 173.8, 170.0, 150.6, 150.5, 149.9, 149.8, 138.7, 138.0, 137.9, 135.3, 134.2, 130.8, 130.7, 126.3, 126.2, 125.8, 124.1, 123.6, 121.3, 118.7, 118.4, 116.2, 113.0, 112.5, 105.8, 66.8, 66.5, 56.3, 54.5, 39.2, 25.3 ppm. Anal. Calcd for $C_{37}H_{38}N_4O_{12}$: C, 60.82; H, 5.24; N, 7.67. Found: C, 60.72; H, 5.34; N, 7.59.

Preparative Example 2: Synthesis of two-photon probe

In this example, the compound of Formula 7 was synthesized by the following procedure.

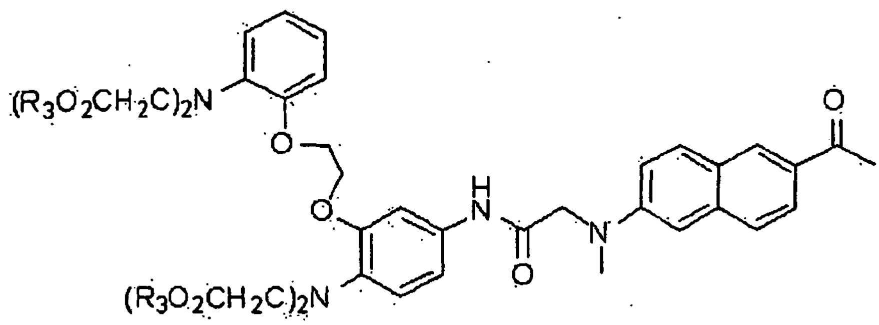

**(7)**

($R_3 = CH_2OCOCH_3$).

A mixture of the compound (0.11g, 0.15 mmol) of Formula 4, bromomethyl acetate (0.24g, 1.55 mmol), and $Et_3N$ (0.14g, 1.05 mmol) in $CHCl_3$ (5 mL) was stirred under $N_2$ for 24 h. The solution was removed in vacuo and the crude product was purified by column chromatography using ethyl acetate/hexane (3:1) as the eluent. It was further purified by recrystallization from MeOH to obtain a pale yellow solid.

Yield: 85 mg (56%); mp 137°C; IR (KBr): 1759, 1710, 1665 $cm^{-1}$; $^1H$ NMR (400 MHz, $CDCl_3$): $\delta$ 8.36 (d, 1H, *J* = 2 Hz), 8.22 (s,1 H), 7.98 (dd, 1 H, *J* = 9, *J* = 2 Hz), 7.88 (d, 1H, *J* = 9 Hz), 7.71 (d, 1H, *J* = 9 Hz), 7.31 (d, 1H, *J* = 2 Hz), 7.17 (dd, 1H, *J* = 9, *J* = 2 Hz), 7.07 (d, 1H, *J* = 2 Hz), 6.88 (m, 6H), 5.62 (s, 4H), 5.60 (s, 4H), 4.29 (s, 4H), 4.18 (s, 4H), 4.15 (s, 4H), 4.12 (s, 2H), 3.25 (s, 3H), 2.69 (s, 3H), 2.05 (s, 6H), 2.04 (s, 6H); $^{13}C$ NMR (100 MHz, $CDCl_3$): $\delta$ = 198.0, 170.4, 170.2, 169.7, 168.1, 151.0, 150.6, 149.3, 138.8, 137.3, 135.7, 132.9, 132.3, 131.5, 130.3, 127.0, 126.6, 125.2, 123.2, 121.9, 120.4, 120.0, 116.7, 113.8, 113.1, 107.7, 106.5, 79.5, 79.4, 77.5, 77.4, 77.1, 67.5, 67.2, 59.5, 53.5, 40.4, 26.7, 20.9 ppm; Anal. Calcd for $C_{49}H_{54}N_4O_{20}$: C, 57.76; H, 5.34; N, 5.50. Found: C, 57.70; H, 5.31; N, 5.52.

Preparative Example 3: Synthesis of two-photon probe

Preparative Example 3.1: Synthesis of 6-acyl-2-naphthylamine

A mixture of 6-acyl-2-hydroxynaphthalene (5.2g, 28 mmol), $Na_2S_2O_5$ (13.3g, 70 mmol) and $NH_4OH$ (150 mL) in a steel-bomb reactor was stirred at 140°C for 96 h. The product was collected by filtration, washed with water, and

purified by flash-column chromatography by using hexane/ethyl acetate as the eluent. It was further purified by recrystallization from MeOH.

Yield: 3.9g (75%); m.p. 170°C; $^1$H NMR (400 MHz, $CDCl_3$): d = 8.30 (d, J = 1.6 Hz, 1H), 7.91 (dd, J = 8.8,2.0 Hz, 1 H), 7.74 (d, J = 8.6 Hz, 1H), 7.58 (d, J = 9.2 Hz, 1H), 6.97 (dd, J = 8.6, 2.2 Hz, 1H), 6.95 (d, J = 2.2 Hz, 1H), 4.05 (br s, 2 H), 2.66 ppm (s, 3H); $^{13}$C NMR (100 MHz, $CDCl_3$): d = 198.1, 147.0, 137.8, 131.6, 131.4, 130.7, 126.7, 126.2, 124.9, 118.9, 108.1, 26.7 ppm; IR (KBr): $\overline{v}$ = 3436, 3345, 1662 $cm^{-1}$; elemental analysis calcd. (%) for $C_{12}H_{11}NO$: C 77.81, H 5.99, N 7.56; found: C 77.78, H 5.86, N 7.59.

Preparative Example 3.2

Sodium triacetoxyborohydride (1.24 g, 5.85 mmol) was added to a mixture of 6-acyl-2-naphthylamine (0.64 g, 3.46 mmol) prepared in Preparative Example 3.1 and 5-methyl-5'-formyl-BAPTA-tetramethyl ester (1.68g, 2.92 mmol) in dichloroethane (30 mL), and the solution was stirred for 12 h under $N_2$. The solvent was removed in vacuo and the product was purified by column chromatography by using chloroform/ethyl acetate/hexane (3:1:1) as the eluent.

Yield: 1.6 g (74%); m.p. 86°C; $^1$H NMR (400 MHz, $CDCl_3$): d = 8.29 (d, J = 1.8 Hz, 1H), 7.91 (dd, J = 8.8, 2.0 Hz, 1H), 7.71 (d, J = 8.6 Hz, 1H), 7.58 (d, J = 8.6 Hz, 1H), 6.92 (m, 3H), 6.73(m, 5H), 4.51 (br s, 1H), 4.36 (s, 2H), 4.25 (m, 4H), 4.15 (s, 4H), 4.10 (s,4H), 3.58 (s, 6H), 3.52 (s, 6H), 2.66 (s, 3H), 2.25 ppm (s, 3H); $^{13}$C NMR (100 MHz, $CDCl_3$): d = 198.0, 172.2, 172.1, 150.8, 150.5, 138.8, 138.1, 136.9, 132.6, 132.4, 131.2, 131.1, 130.6, 126.3, 125.0, 122.0, 120.7, 119.4, 119.1, 118.7, 114.3, 114.2, 112.7, 112.6, 104.8, 67.4, 67.1, 53.6, 53.5, 51.9, 51.7, 47.9, 26.7, 21.2 ppm; IR (KBr): $\overline{v}$ = 3343, 1756, 1663 $cm^{-1}$; elemental analysis calcd. (%) for $C_{40}H_{45}N_3O_{11}$: C 64.59, H 6.10, N 5.65; found: C 64.49, H 6.15, N 5.54.

Preparative Example 3.3: Preparation of two-photon probe

In this example, the two-photon probe of Formula 8 was prepared by the following procedure.

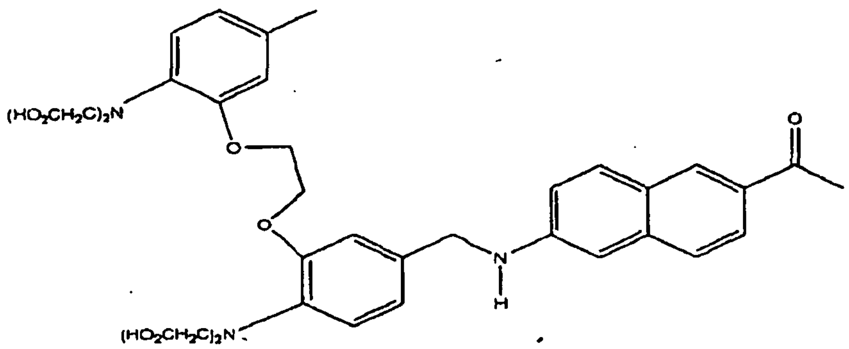

(8)

A mixture of the compound (0.50 g, 0.67 mmol) prepared in Preparative Example 3.2 and KOH (0.30 g, 5.36 mmol) in EtOH/$H_2O$ (50/10 mL) was stirred for 6 h. The ethanol was evaporated in vacuo, the residue was diluted with ice-water (30 mL), and concentrated HCl (aq.) was added slowly until pH 3 had been reached. The resulting precipitate was collected and washed with distilled water.

Yield: 0.27 g (59%); m.p. 151°C; $^1$H NMR (400 MHz, $CD_3OD$): d = 8.33 (d, J = 1.6 Hz, 1H), 7.80 (dd, J = 8.8, 1.6 Hz, 1H), 7.71 (d, J = 9.2 Hz, 1 H), 7.50(d, J = 8.6 Hz, 1H), 7.09 (d, J = 2.0 Hz, 1 H), 7.06 (dd, J = 9.0, 2.0 Hz, 1H), 6.94 (dd, J = 8.8, 2.0 Hz, 1H), 6.88 (d, J = 8.8 Hz, 1H), 6.82 (d, J = 9.0 Hz, 1H), 6.77 (d, J = 1.6 Hz, 1H), 6.76 (d, J = 2.0 Hz, 1H), 6.67 (dd, J = 9.0, 2.0 Hz, 1H), 4.37 (s, 2H), 4.32 (t, 2H), 4.26 (t, 2H), 4.02 (s, 4H), 3.96 (s, 4H), 2.62 (s, 3 H), 2.42 ppm (s, 3H); $^{13}$C NMR (100 MHz, $CD_3OD$): d = 199.2, 174.8, 150.7, 149.4, 138.6, 138.0, 136.3, 134.1, 133.1, 130.9, 130.6, 130.1, 126.1, 125.8, 125.7, 124.0, 123.9, 121.5, 120.2, 119.4, 118.8, 118.2, 114.2, 112.7, 67.2, 66.9, 55.2, 55.0, 46.7, 25.3, 25.2, 19.8 ppm; IR (KBr): $\overline{v}$ = 3350, 2915, 1750, 1662 $cm^{-1}$; elemental analysis calcd.(%) for $C_{36}H_{37}N_3O_{11}$: C 62.87, H 5.42, N 6.11; found: C 62.64, H 5.90, N 6.02.

Preparative Example 4: Preparation of two-photon probe

In this example, the two-photon probe of Formula 9 was prepared by the following procedure.

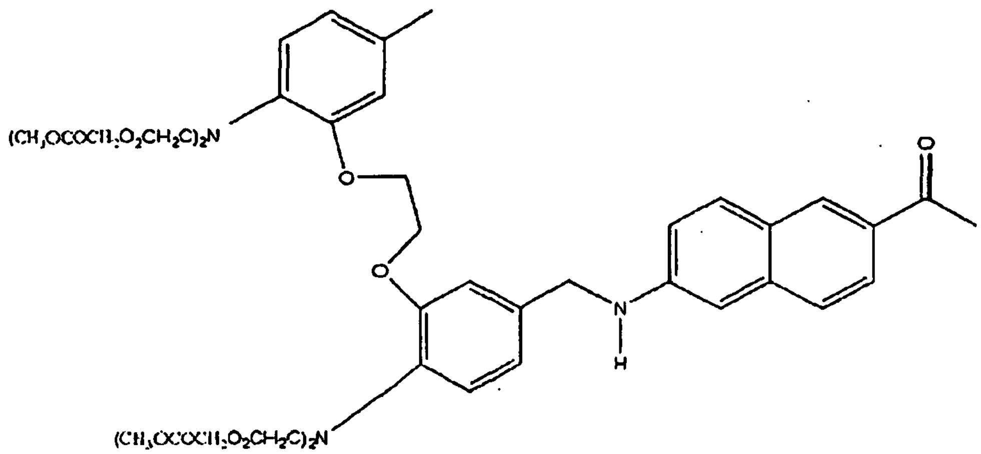

(9)

A mixture of the compound of Formula 8 (0.12 g, 0.17 mmol), bromomethyl acetate (0.21 g, 1.4 mmol), and $Et_3N$ (0.14 g, 1.4 mmol) in $CHCl_3$ (5 mL) was stirred under $N_2$ for 24 h. The solvent was removed in vacuo and the crude product was purified by column chromatography by using ethyl acetate/hexane (3:1) as the eluent. It was further purified by recrystallization from MeOH.

Yield: 85 mg (51%); m.p. 78°C; $^1H$ NMR (400 MHz, $CDCl_3$): d = 8.30 (d, J = 1.6 Hz, 1H), 7.91 (dd, J = 8.8, 1.6 Hz, 1 H), 7.72 (d, J = 8.6 Hz, 1 H), 7.59 (d, J = 9.0 Hz, 1H), 6.95 (m, 3H), 6.75 (m, 5H), 5.61 (s, 4H), 5.56 (s, 4 H), 4.65 (br s, 1 H), 4.36 (s, 2 H), 4.27 (s, 4H), 4.20 (s, 4H), 4.15 (s, 4 H), 2.67 (s, 3 H), 2.27 (s, 3H), 2.07 (s, 6H), 2.05 ppm (s, 6H); $^{13}C$ NMR (100 MHz, $CDCl_3$): d = 198.1, 170.5, 170.4, 169.8, 169.7, 150.9, 150.6, 148.4, 138.2, 136.3, 135.4, 133.4, 133.1, 131.2, 131.1, 130.6, 130.4, 126.3, 125.0, 122.2, 121.1, 119.8, 118.8, 114.5, 114.4, 113.0, 104.0, 79.5, 79.4, 79.3, 67.4, 67.1, 53.6, 53.5, 47.9, 26.7, 22.9, 20.9 ppm; IR (KBr): $\bar{v}$ = 3345, 1750, 1710, 1662 $cm^{-1}$; elemental analysis calcd.(%) for $C_{48}H_{53}N_3O_{19}$: C 59.07, H 5.47, N 4.31; found: C 58.80, H 5.78, N 4.12.

Preparative Example 5: Preparation of two-photon probe

In this example, the two-photon probe of Formula 10 was prepared by the following procedure.

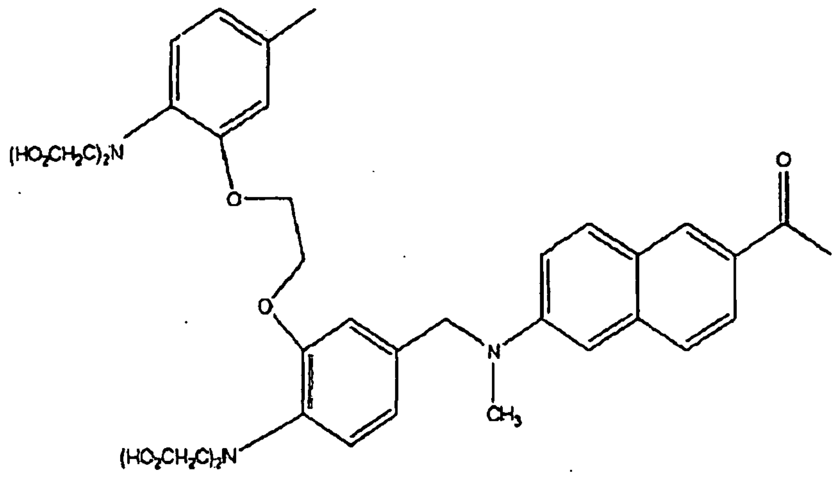

(10)

The compound (0.80 g, 1.08 mmol) prepared in Preparative Example 3.2 was dissolved in MeCN (30 mL) and proton sponge (0.46 g, 2.15 mmol), NaI (0.032 g, 0.22 mmol), and $CH_3I$ (0.46 g, 3.24 mmol) were added. The reaction mixture was refluxed for 12 h under $N_2$. The solvent was removed in vacuo and the product was purified by column chromatography by using chloroform/ethyl acetate/hexane (3:1:1) as the eluent.

Yield: 0.73 g (90%); m.p. 82°C; $^1H$ NMR (400 MHz, $CDCl_3$): d = 8.30 (d, J = 1.6 Hz, 1H), 7.91 (dd, J = 8.6, 1.8 Hz, 1 H), 7.76 (d, J = 8.8 Hz, 1H), 7.60 (d, J = 9.0 Hz, 1 H), 7.16 (dd, J = 9.0, 2.0 Hz, 1H), 6.90 (d, J = 2.0 Hz, 1H), 6.66 (m, 6H), 4.60 (s, 2H), 4.20 (s, 4H), 4.13 (s, 4 H), 4.08 (s, 4 H), 3.55 (s, 6H), 3.51 (s, 6H), 3.14 (s, 3H), 2.67 (s, 3 H), 2.25

ppm (s, 3H); $^{13}C$ NMR (100 MHz, $CDCl_3$): d = 198.0, 172.3, 172.2, 150.8, 150.4, 138.5, 137.9, 136.9, 132.4, 132.2, 131.0, 130.6, 126.4, 125.4, 124.8, 121.9, 119.7, 119.3, 119.1, 116.6, 114.2, 111.4, 105.6, 67.2, 67.0, 58.7, 56.3, 53.6, 53.5, 51.8, 51.7, 39.0, 26.7, 21.2, 18.7 ppm; IR (KBr): $\bar{v}$ = 1756, 1662 $cm^{-1}$; elemental analysis calcd.(%) for $C_{41}H_{47}N_3O_{11}$: C 64.98, H 6.25, N 5.54; found: C 65.04, H 6.19, N 5.59.

This ester (0.50 g, 0.66 mmol) was hydrolyzed by the method described above.

Yield: 0.24 g (52%); m.p. 141°C; $^{1}H$ NMR (400 MHz, $CD_3OD$): d = 8.35 (d, J = 1.8 Hz, 1 H), 7.82 (dd, J = 8.8, 1.6 Hz, 1H), 7.79 (d, J = 8.8 Hz, 1H), 7.59 (d, J = 9.0 Hz, 1H), 7.26 (dd, J = 9.0, 2.0 Hz, 1 H), 6.98 (d, J = 2.0 Hz, 1H), 6.82 (m, 6H), 4.67 (s, 2H), 4.25 (m, 4H), 3.96 (s, 4H), 3.90 (s, 4H), 3.16 (s, 3H), 2.63 (s, 3H), 2.24 ppm (s, 3H); $^{13}C$ NMR (100 MHz, $CD_3OD$): d = 199.0, 172.6, 172.5, 150.4, 150.2, 138.4, 138.1, 137.6, 135.3, 134.2, 130.8, 130.7, 125.1, 124.7, 121.3, 119.4, 119.1, 118.7, 118.4, 116.2, 114.3, 112.7, 112.1, 105.8, 67.3, 67.0, 58.3, 56.3, 54.5, 51.6, 39.2, 25.3, 19.0 ppm; IR (KBr): $\bar{v}$ = 2915, 1748, 1663 $cm^{-1}$; elemental analysis calcd.(%) for $C_{37}H_{39}N_3O_{11}$: C 63.33, H 5.60, N 5.99; found: C 63.22, H 5.75, N 5.91.

Preparative Example 6: Preparation of two-photon probe

In this example, the two-photon probe of Formula 11 was prepared from the compound of Preparative Example 5 by using the procedure described in Preparative Example 4.

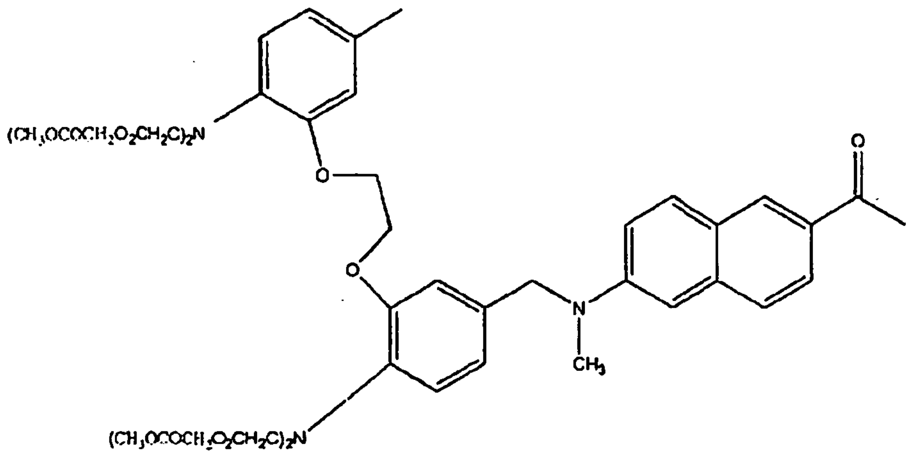

(11)

Yield: 54%; m.p. 102°C; $^{1}H$ NMR (400 MHz, $CDCl_3$): d = 8.30 (d, J = 1.6 Hz, 1H), 7.92 (dd, J = 8.6, 1.6 Hz, 1 H), 7.77 (d, J = 8.8 Hz, 1H), 7.62 (d, J = 9.0 Hz, 1 H), 7.17 (dd, J = 9.0, 2.0 Hz, 1H), 6.93 (d, J = 2.0 Hz, 1 H), 6.74 (m, 6 H), 5.60 (s, 4 H), 5.58 (s, 4 H), 4.60 (s, 2H), 4.22 (s, 4H), 4.17 (s, 4 H), 4.14 (s, 4 H), 3.15 (s, 3H), 2.67 (s, 3H), 2.26 (s, 3 H), 2.05 (s, 6H), 2.04 ppm (s, 6H); d = 8.0, 171.1, 170.8, 169.7, 169.6, 150.5, 150.2, 148.7, 138.6, 136.3, 135.4, 133.3, 133.2, 131.7, 131.6, 130.8, 130.5, 126.5, 125.2, 122.7, 121.1, 119.8, 118.8, 114.7, 114.6, 113.2, 104.0, 79.7, 79.5, 79.4, 68.4, 68.1, 54.6, 54.5, 47.9, 42.8, 26.7, 23.2, 21.0 ppm; IR (KBr): $\bar{v}$ = 1750, 1710, 1660 $cm^{-1}$; elemental analysis calcd.(%) for $C_{49}H_{55}N_3O_{19}$: C 59.45, H 5.60, N 4.24; found: C 59.42, H 5.54, N 4.30.

Example 1: Measurements of absorption and emission spectra

The absorption spectra of the compounds of Formula 4 ('ACa1'), Formula 7 ('ACa1-AM), Formula 8('ACa2'), Formula 9 ('ACa2-AM'), Formula 10 ('ACa-3') and Formula 11 ('ACa-3-AM') were recorded on a Hewlett-Packard 8453 diode array spectrophotometer, and the fluorescence spectra of the compounds were obtained with Amico-Bowman series 2 luminescence spectrometer with a 1 cm standard quartz cell. The fluorescence quantum yields of the compounds were determined by using Coumarin 307 as the reference by the literature method (J. N. Demas, G. A. Crosby, J. Phys. Chem. 1971, 75, 991-1024.). The obtained photophysical data for the compounds are shown in Table 1.

[Table 1]

| Compound[a] | $\lambda_{max}^{(1)}$ [b] | $\lambda_{max}^{fl}$ [c] | Φ[d] | $K_d^{OP}/K_d^{TP}$ [e] | *FEF/TFEF*[f] | $\lambda_{max}^{(2)}$ [g] | □□h] | Φ□□i] |
|---|---|---|---|---|---|---|---|---|
| ACa1-AM | 362 | 495 | 0.060 | | | nd[j] | nd[j] | nd[j] |
| ACa1 | 365 | 498 | 0.012 | | | nd[j] | nd[j] | nd[j] |
| ACa1+$Ca^{2+}$ | 365 | 498 | 0.49 | 0.27[k]/0.25 | 40/44 | 780 | 230 | 110 |
| ACa2-AM | 362 | 494 | 0.014 | | | nd[j] | nd[j] | nd[j] |
| ACa2 | 362 | 495 | 0.010 | | | nd[j] | nd[j] | nd[j] |
| ACa2+$Ca^{2+}$ | 362 | 495 | 0.42 | 0.14[k]/0.16 nM | 42/50 | 780 | 210 | 90 |
| ACa3-AM | 370 | 499 | 0.032 | | | nd[j] | nd[j] | nd[j] |
| ACa3 | 375 | 500 | 0.015 | | | nd[j] | nd[j] | nd[j] |
| ACa3+$Ca^{2+}$ | 375 | 517 | 0.38 | 0.13[k]/0.14 nM | 25/23 | 780 | 250 | 95 |
| OG1+$Ca^{2+}$ | 494 | 523 | 0.66 | 0.17[l]/nd | 14[l]/nd | 800 | 37 | 24 |

[a] All data were measured in 30 mM 3-(N-morpholino)propanesulfonic acid (MOPS), 100 mM KCl, 10 mM EGTA, pH 7.2 in the absence and presence of free $Ca^{2+}$.
[b,c] $\lambda_{max}$ of the one-photon (OP) absorption and emission spectra, respectively, in nm.
[d] Fluorescence quantum yield, ±10%.
[e] Dissociation constants for $Ca^{2+}$ in μM measured by OP ($K^{OP}{}_d$) and TP ($K^{TP}{}_d$) processes, ±4%.
[f] Fluorescence enhancement factor, $(F-F_{min})/F_{min}$, measured by OP (FEF) and TP (TFEF) processes.
[g] $\lambda_{max}$ of the TP excitation spectra in nm.
[h] The peak TP cross section in $10^{-50}$ $cm^4 s$ $photon^{-1}$ (GM), ±5%.
[i] TP action cross section.
[j] nd: not determined; the TPEF intensity was too weak to measure the cross section accurately.
[k] $K^{OP}{}_d$ values of ACa1, ACa2, and ACa3 for $Mg^{2+}$ are 6.8±0.7, 6.2±0.7 and 6.0±0.6 mM, respectively.

Referring to Table 1, the absorption and emission spectra of ACa1 and ACa2 are almost the same, probably because the electron-donating effect of the N-methyl group is nullified by the electron-withdrawing ability of the amide group between the fluorophore and BAPTA. ACa3 shows a slight bathochromic shift compared to ACa2, indicating an enhanced intramolecular charge transfer (ICT) by the N-methyl group.

FIG. 1 shows the absorption (1a, 1c, 1e) and emission (1b, 1d, 1f) spectra of the two-photon probes ACa1, ACa2 and ACa3 in 1,4-dioxane, DMF, ethanol and $H_2O$. The absorption and emission maxima of the two-photon probes in various solvents are shown in Table 2.

[Table 2]

| Cpd | Solvent ($E_T^N$)[a] | $\lambda_{max}^{(1)}$, nm[b] | $\lambda_{max}^{fl}$, nm[b] | Φ[c] |
|---|---|---|---|---|
| ACa1-AM | 1,4-dioxane (0.164) | 344 | 411 | |
| | DMF (0.386) | 353 | 442 | 0.27 |
| | EtOH (0.654) | 359 | 472 | |
| | $H_2O$ (1.00) | 360 | 495 | 0.060 |
| ACa2-AM | 1,4-dioxane (0.164) | 345 | 413 | |
| | DMF (0.386) | 355 | 440 (440)[d] | 0.37 |
| | EtOH (0.654) | 359 | 473 | |
| | $H_2O$ (1.00) | 362 | 494 | 0.014 |
| ACa3-AM | 1,4-dioxane (0.164) | 351 | 427 | |
| | DMF (0.386) | 357 | 452 | 0.22 |
| | EtOH (0.654) | 363 | 484 | |

(continued)

| Cpd | Solvent ($E_T^N$)[a] | $\lambda_{max}^{(1)}$, nm[b] | $\lambda_{max}^{fl}$, nm[b] | Φ[c] |
|---|---|---|---|---|
| | $H_2O$ (1.00) | 370 | 499 | 0.032 |

[a] The numbers in the parenthesis are normalized empirical parameter of solvent polarity.
[b] $\lambda_{max}$ of the one-photon absorption and emission spectra in nm.
[c] Fluorescence quantum yield, ±10%.

The absorption and emission spectra of all compounds showed bathochromic red shifts with the solvent polarity in the order 1,4-dioxane < DMF < ethanol < $H_2O$. The effects are greater for the emission (72-82 nm) than those for the absorption spectra (17-19 nm). This indicates the potential utility of these compounds as polarity probes. In addition, the $\lambda_{max}^{fl}$ values of the AM esters in DMF are similar to those of the membrane-bound probes (FIG. 4(b) and Table 2), indicating that they could be used as models for the membrane-bound probes.

FIG. 2 shows one-photon emission (2a, 2c, 2e) and absorption (2b, 2d, 2f) spectra of ACa1, ACa2 and ACa3 (1 μM, 30 mM MOPS, 100 mM KCl, 10 mM EGTA, pH 7.2) in the presence of free $Ca^{2+}$ at various concentrations (0-39 μM).

FIG. 3 shows two-photon action spectra of ACa1 and ACa2 in the presence of free $Ca^{2+}$ at various concentrations.

When $Ca^{2+}$ was added to ACa2 in MOPS buffer solution (30 mM, pH 7.2), the fluorescence intensity increased gradually with the metal-ion concentration (FIG. 2(c)) without affecting the absorption spectra (FIG. 2(d)), probably due to the blocking of the photoinduced electron-transfer (PET) process by the metal-ion complexation.

Similar results were observed for ACa1 (FIG. 2(a, b)) and ACa3 (FIG. 2(e, f)).

The fluorescence-enhancement factors [FEF = $(F - F_{min})/F_{min}$] of ACa1-ACa3 measured by the titration curves were in the range of 23-50 (Table 1). The FEF of ACa2 is nearly identical to that of ACa1, while that of ACa3 is appreciably smaller as a result of the larger fluorescence quantum yield (φ) in the absence, and smaller φ in the presence, of excess $Ca^{2+}$ than for the others. All compounds show larger FEFs than the value (14) reported for OG1, indicating a greater sensitivity of these probes to the change in the $Ca^{2+}$-ion concentration. Moreover, Hill plots for $Ca^{2+}$ binding, measured by OP and TP processes, showed good linear relationships with a slope of 1.0, indicating 1:1 complexation (FIG. 5(a, b, c)).

Example 2: Determination of Dissociation Constants (*Kd*)

A series of calibration solutions containing various [$Ca^{2+}$] was prepared by mixing two solutions (solution A containing 10 mM $K_2$EGTA and solution B containing 10 mM CaEGTA) in various ratios. Both solutions contained ACa1 (1 μM), 100 mM KCl, 30 mM MOPS, and they were adjusted to pH 7.2.

To determine the *Kd* for $Ca^{2+}$ complexes with the two-photon probes, the fluorescence spectrum was recorded with 2.0 mL of solution A (0 μM free $Ca^{2+}$) at 20°C. Then, 203 μℓ of this solution was discarded and replaced by 203 μℓ of solution B (39 μM free $Ca^{2+}$), and the spectrum was recorded. This brings the CaEGTA concentration to 1.00 mM and the $[Ca^{2+}]_{free}$ to about 0.017 μM with no change in the concentration of the probe or of the total EGTA. The $[Ca^{2+}]_{free}$ is calculated from the Kd of EGTA for $Ca^{2+}$ (150.5 nM) by using Equation 2:

$$[Ca^{2+}]_{free} = K_d^{EGTA} \times \frac{[CaEGTA]}{K_2EGTA} \qquad (2)$$

Further, iterations attained 0.038, 0.065, 0.101, 0.150, 0.230, 0.350, 0.601, 0.800, 1.00, 1.30, 2.50, 5.30, 10.0, and 20.0 μM free $Ca^{2+}$ by successively discarding 223, 251, 285, 327, 421, 479, 667, 420, 350, 412, 905, 1028, 926, and 992 μℓ of solution A and replacing each with an equal volume of solution B.

When a 1:1 metal-ligand complex is formed between the two-photon probe and $Ca^{2+}$, the equilibrium can be described by Equation 3:

$$[LM]^2 - ([L]_0 + [M]_0 + K_d)[LM] + [L]_0[M]_0 = 0 \quad (3)$$

where L and M represent the two-photon probe and $Ca^{2+}$, respectively.

The total probe and metal ion concentration are defined as $[L]_0 = [L] + [LM]$ and $[M]_0 = [M] + [LM]$, respectively. With $[L]_0$ and $[M]_0$, the value of *Kd* is given by:

$$[LM] = \frac{([L]_0 + [M]_0 + K_d) - \sqrt{([L]_0 + [M]_0 + K_d)^2 - 4[L]_0[M]_0}}{2} \quad (4);$$

$$(F - F_{min}) = \left(\frac{([L]_0 + [M]_0 + K_d) - \sqrt{([L]_0 + [M]_0 + K_d)^2 - 4[L]_0[M]_0}}{2[L]_0}\right)(F_{max} - F_{min}) \quad (5)$$

where F is the observed fluorescence intensity, $F_{min}$ is the minimum fluorescence intensity, and $F_{max}$ is the maximum fluorescence intensity. The *Kd* value that best fits the titration curve (FIG. 6) with Equation 5 was calculated by using the Excel program as reported (J. R. Long, R. S. Drago, J. Chem. Ed. 1982, 59, 1037; K. Hirose, J. Incl. Phenom. Macrocycl. Chem. 2001, 39, 193).

To determine the $K_d^{TP}$ value for the two-photon process, the TPEF spectra were obtained with a DM IRE2 Microscope (Leica) excited by a mode-locked titanium-sapphire laser source (Coherent Chameleon, 90 MHz, 200 fs) set at wavelength 780 nm and output power 1180 mW, which corresponded to approximately 10 mW average power in the focal plane. The TPEF titration curves (FIG. 6(a, b, c)) were obtained and fitted to Equation 5.

The dissociation constants ($K_d^{OP}$) were calculated from the fluorescence titration curves (FIG. 6), as reported (J. R. Long, R. S. Drago, J. Chem. Educ. 1982, 59, 1037-1039).

FIG. 6 shows fluorescence titration curves for the two-photon probes ACa1, ACa 2 and ACa3.

Referring to FIG. 6, the $K_d^{OP}$ values of ACa2 and ACa3 for $Ca^{2+}$ were approximately twofold smaller than that of ACa1 (Table 1), indicating higher affinity for $Ca^{2+}$, which can be attributed to the enhanced electron density at the metal-ion binding sites by the p-Me group. Similar values were determined by the TP process (Table 1). This negates the possibility of $Mg^{2+}$ interference.

One-photon emission spectra of ACa1 (30 mM MOPS, 100 mM KCl, 10 mM EGTA, pH 7.2) in the presence of free $Mg^{2+}$ (0-30 mM), a Hill plot for the complexation of ACa1 with free $Mg^{2+}$ (0-30 mM), and a one-photon fluorescence titration curve for the complexation of ACa1 with free $Mg^{2+}$ (0-30 mM) were obtained and are shown in FIGs. 7, 8 and 9, respectively.

The $K_d^{OP}$ values for $Ca^{2+}$ and $Mg^{2+}$ were 0.27 $\pm$ 0.01 μM and 6.8 $\pm$ 0.7 mM, respectively. A similar value was determined in the two-photon process [$K_d^{TP}$ ($Ca^{2+}$) = 0.25 $\pm$0.03 μM]. ACa1 showed modest response toward $Zn^{2+}$ and $Mn^{2+}$, a much weaker response toward $Mg^{2+}$, $Fe^{2+}$ and $Co^{2+}$, and no response toward $Cu^{2+}$ (FIG. 10(a)). Similarly, ACa2 and ACa3 showed a modest response toward $Zn^{2+}$ and $Mn^{2+}$, and a much weaker response toward $Mg^{2+}$, $Fe^{2+}$, and $Co^{2+}$ (FIG. 10(b, c)). Because the intracellular concentrations of free $Mn^{2+}$ is negligible and chelatable $Zn^{2+}$ is essentially nonexistent except in specialized areas such as the hippocampal CA3 region, the probes can selectively detect the intracellular $Ca^{2+}$ concentration ($[Ca^{2+}]_i$) without interference from other metal ions. Furthermore, the two-photon probes ACa1, ACa2 and ACa3 are pH-insensitive in the biologically relevant pH range (FIG. 11 (a, b, c)).

Example 3: Observations of two-photon (TP) action spectra

The TP action spectra of the $Ca^{2+}$ complexes with the two-photon probes and OG1 in buffer solutions indicated the a value $\Phi\delta$ of 110 GM at 780 nm, approximately four-fold larger than that of OG1-$Ca^{2+}$. Thus, TPM images for samples stained with ACa1-ACa3 would be much brighter than those stained with commercial probe. In addition, the two-photon fluorescence enhancement factors (TFEFs) estimated from the two-photon titration curves were in the range of 23-50 (Table 1), values that could allow detection of $Ca^{2+}$ by TPM.

Example 4: Observation of cells using the two-photon probes

Astrocytes were taken from cerebral cortices of one-day-old rats (Sprague-Dawley; SD). Cerebral cortices were dissociated in Hank's balanced salt solution (HBSS; Gibco BRL, Gaithersburg, MD, USA), containing 3 U/ml papain (Worthington Biochemical Corporation, NJ, USA) and plated in 75 mm flasks. To prepare purified astrocytes culture, flasks were shaken for 6 h on a shaker at 37°C and the floating cells that were displaced into the media were removed. Astrocytes were passaged with 5 min exposure to 0.25% trypsin and replated onto poly-D-lysin-coated glass coverslips at 50-100 cells per $mm^2$, and maintained in Dulbecco's modified Eagle's medium (DMEM; Gibco) supplemented with penicillin/streptomycin and 10% fetal bovine serum (FBS; Gibco) in a $CO_2$ incubator at 37°C. After 7-15 days in vitro, astrocytes were washed three times with serum-free medium, and then incubated with 2 $\mu$M of ACa1-AM in serum-free medium for 20 min at 37°C. The cells were washed three times with phosphate-buffered saline (PBS; Gibco) and then imaged after further incubation in colorless serum-free medium for 15 min.

The pseudocolored TPM images of cultured astrocytes labeled with ACa1-AM, ACa2-AM and ACa3-AM (2 $\mu$M) showed intense spots and homogeneous domains (FIG. 12(a, b, c). It has been estimated that the images were attributed to the TPEF emitted from the intracellular ACa1-, ACa2- and ACa3-$Ca^{2+}$ complexes and membrane-bound probes because the fluorescence quantum yields of ACa1-$Ca^{2+}$, ACa2-$Ca^{2+}$ and ACa3-$Ca^{2+}$ in MOPS buffer (0.42 for ACa2) and ACa1-AM, ACa2-AM and ACa3-AM in DMF (0.27 for ACa2-AM) are much higher than those of ACa1, ACa2 and ACa3 (0.010 for ACa2) and ACa1-AM, ACa2-AM and ACa3-AM (0.060) in MOPS buffer (Tables 1 and 2). Therefore, the two-photon probes in DMF have been assumed to be good models for the membrane-bound probes due to the similarity in $\lambda_{max}^{fl}$.

The TPEF spectra of the intense spots and homogeneous domains showed emission maxima at 445 (blue curve in the right side of FIG. 12(b)) and 500 nm (red curve in the right side of FIG. 12(b)), respectively. Moreover, the blue emission band was asymmetrical and could be fitted to two Gaussian functions with emission maxima at 445 and 488 nm (sky blue curves in the right side of FIG. 12(b)), respectively, whereas the red emission band could be fitted to a single Gaussian function with maximum at 500 nm (brown curve in the right side of FIG. 12(b)). Compared with the emission spectra recorded in MOPS buffer (FIG. 2(a)), the shorter-wavelength band of the dissected spectrum was significantly blue-shifted while the longer-wavelength band remained similar. The spectral shift suggests that the probes may be located in two regions of differing polarity.

To assess the polarity of environments, lifetime images of astrocytes labeled with the two-photon probes were observed.

Astrocytes were grown on coverslip in 10% FBS containing DMEM. Cells were washed briefly in PBS and incubated with 2 $\mu$M of each of the two-photon probes for 20 min at 37°C. The cells were washed with PBS three times, fixed with formaldehyde (3.7% in PBS) for 10 min, washed with PBS three times, and then mounted with mounting solution. The fluorescence decays were resolved by time-correlated single-photon counting (TCSPC) using an SPC830 acquisition board (Becker & Hickl, Berlin) synchronized with a Leica TSC-SP2-AOBS confocal microscope. The results are shown in FIG. 13(a-d)).

Referring to FIG. 13(a-d) for ACa1, the intense spot exhibited an excited state lifetime of 1.8 ns, more than 2-fold longer than the upper extreme of the lifetime distribution curve centered at -0.8 ns. This result may reflect two distinct environments populated by the probes, a hydrophilic one that is likely to be cytosolic, which emits red light with a shorter lifetime, and a hydrophobic one, likely to be membrane-associated, with longer-lived blue emission. Referring to FIG. 18 for ACa2, the intense spot exhibited an excited-state lifetime of 1.2 ns, which was much longer than the upper extreme of the lifetime distribution curve centered at 0.6 ns.

From these results, it is hypothesized that the two-photon probes are located in two different environments, the more polar one is cytosol (red emission with a shorter lifetime) and the less-polar one is membrane-associated (blue emission with a longer lifetime).

The intracellular $Ca^{2+}$ could be detected with minimum errors due to the membrane-bound probes. The spectrum of the shorter wavelength band in the dissected Gaussian function (sky blue curve in the right side of FIG. 12(b)) decreased to baseline at -500 nm, indicating that the TPEF emitted from the membrane-bound probes contributes negligible interference at $\lambda$ > 500 nm. Similarly, if it is considered that ACa2-AM in DMF is as a model for the membrane-bound probe, the fluorescence at $\lambda$ > 500 nm accounts for only 5% of the total emission band (FIG. 2). Consistently, the TPEF images collected at 500-620 nm are homogeneous without the intense spots (FIG. 14(b)), whereas those collected at 360-460 nm clearly show them (FIG. 14(a)). Therefore, the intracellular $Ca^{2+}$ can be selectively detected by using the detection window at 500-620 nm.

To demonstrate the utility of the probes, $[Ca^{2+}]_i$ waves in live cells and tissue were monitored. The TPM images of cultured astrocytes labeled with ACa1 (2 $\mu$M) revealed the spontaneous $Ca^{2+}$ signal propagation from the astrocytic process (1) to soma (2) to terminal (3) with a speed of 7.5 $\pm$2.2 $\mu$m/s (n = 5 astrocytes) (FIG. 15(a, c)). ACa2 showed

a speed of 7.2 ± 2.2 μm/s (*n* = 8 astrocytes) (FIG. 20(a, c)). The spontaneous increase in $[Ca^{2+}]_i$ also propagated between astrocytes, as indicated by the delayed activity in the neighboring astrocyte (FIG. 15(b, d) for ACa1, and FIG. 20(b, c) for ACa2). Particularly, a closer examination of FIG. 20(b, c) reveals that ACa2 can also detect $Ca^{2+}$ in the nucleus.

The speed of propagation of spontaneously occurring waves was 1.8 ±1.1 μm/s (n = 7 astrocytes) for ACa1 and 2.1 ± 1.1 μm/s for ACa2, demonstrating that the two-photon probe ACa2 is superior to ACa1 in terms of wave propagation. These experimental results clearly show that the two-photon probes are capable of visualizing the intra- and intercellular calcium waves in cultured astrocytes by using TPM.

Example 5: Observations of acute rat hypothalamic slices

Slices were prepared from the hippocampi and the hypothalmi of two-day-old rats (SD). Coronal slices were cut into 400 μm thick slices by using a vibrating-blade microtome in artificial cerebrospinal fluid (ACSF; 138.6 mM NaCl, 3.5 mM KCl, 21 mM $NaHCO_3$, 0.6 mM $NaH_2PO_4$, 9.9 mM D-glucose, 1 mM $CaCl_2$, and 3 mM $MgCl_2$). Slices were incubated with ACa1-AM (10 μM) in ACSF bubbled with 95% $O_2$ and 5% $CO_2$ for 30 min at 37°C. The slices were then washed three times with ACSF and transferred to glass-bottomed dishes (MatTek), and observed in a spectral confocal multiphoton microscope.

To obtain the TPM images of the CA1 region in the presence of 20 μM N,N,N',N'-terakis(2-pyridyl)ethylenediamine (TPEN), a membrane permeable $Zn^{2+}$ chelator that can effectively remove $Zn^{2+}$ by chelation without causing toxic effect (C. M. Matias, N. C. Matos, M. Arif, J. C. Dionisio, M. E. Quinta-Ferreira, Biol. Res. 2006, 39, 521-530), a 20 mM stock solution of TPEN was prepared by dissolving 8.5 mg of TPEN in 1.0 mL of ethanol (M. E. Quinta-Ferreira, C. M. Matias, Brain Res. 2004, 1004, 52-60). 1.0 μℓ of this solution was added to 1.0 mL of ACSF to prepare 20 μM TPEN in ACSF. After taking the TPM image of the hippocampal slice labeled with ACa1-AM of Formula 7, the ACSF solution in the glass-bottomed dish was removed with a micropipette, 1 mL of 20 μM TPEN in ACSF was added, and then TPM image was obtained.

FIG. 16 shows pseudocolored TPM images of an acute rat hypothalamic slice stained with ACa1-AM (10 μM) of Formula 7 taken after 195s (16a) and 214 s (16b), and FIG. 17 shows spontaneous $Ca^{2+}$ transients recorded in soma (1), astrocyte process (2), and neighboring cell (3) in FIG. 16.

Referring to FIG. 17, the spontaneous $Ca^{2+}$ waves in the soma could be clearly visualized with a frequency of about 16 mHz (n = 4 slices) for more than 1100 s without appreciable decay. Furthermore, the spikes at the astrocyte process appeared slightly before those in the soma, confirming the previous finding that the signals propagate progressively from the process to the soma (J. Y. Koh, S. W. Suh, B. J. Gwag, Y. Y. He, C. Y. Hsu, D. W. Choi, Science 1996, 272, 1013-1016).

Similar results were reported for TTX-treated thalamus slices stained with fura-2 (H. R. Parri, T. M. Gould, V. Crunelli, Nat. Neurosci. 2001, 4, 803-812) except that the image revealed damaged cells on the tissue surface and was not as clear as the TPM image presented here. Also, the TTX fluorescence intensity decayed appreciably after 500s.

The improved TPM images were observed at -170 μm depth for a prolonged time in the present invention, underlining high photo-stability and low photo-toxicity of the probes in addition to the capability of deep tissue imaging.

Finally, the spikes at the process became very weak after 700s, probably because it has moved away from the focal point under the microscope. As can be seen from the data in FIG. 17, a similar calcium wave was also observed in a different cell.

FIG. 21 shows TPM images (21a) and (21b) of a rat hypothalamic slice stained with 10μm ACa2-AM, and spontaneous $Ca^{2+}$ transients recorded in cells (21c).

Referring to FIG. 21, the spontaneous $Ca^{2+}$ waves in the somas could be simultaneously visualized with an average frequency of about 11 mHz (n = 4 slices) for more than 4000s without appreciable decay, which indicates that the operating time of ACa2 is three-fold or more than that of ACa1.

In contrast, OPM images of the tetrodotoxin-treated thalamus slice stained with fura-2 (H. R. Parri, T. M. Gould, V. Crunelli, Nat. Neurosci. 2001, 4, 803-812) revealed damaged cells on the tissue surface and was not as clear as the TPM image presented in the specification. Also, the fluorescence intensity decayed appreciably after 500s. The improved TPM image of ACa2-labeled cells was observed for a prolonged time (≥ 4000s), which underlines the high photostability and lowphototoxicity of this probe. A similar result was also observed for ACa3.

Furthermore, it has to be mentioned that the use of laser power higher than that of OP fluorescence microscopy (10 mW versus 20 uW) does not in any way cause appreciable damage to the cell and tissue. This can be attributed to the negligible absorption by the sample at 780 nm, which can only lead to vibrational and not the electronic excitation.

## Claims

1. A two-photon probe for real-time monitoring of intracellular calcium ions, represented by Formula 1:

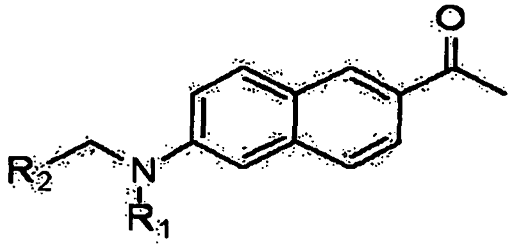

(1)

wherein $R_1$ is $CH_3$ or H, and
$R_2$ is

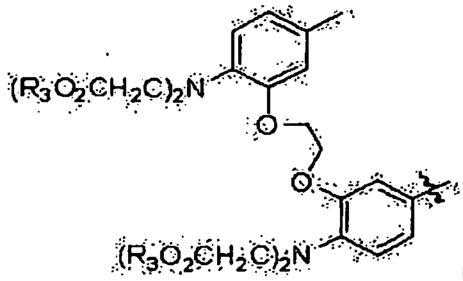

($R_3$ = H or $CH_2OCOCH_3$).

**2.** The two-photon probe according to claim 1, wherein $R_3$ is $CH_2OCOCH_3$.

**3.** A method for preparing a two-photon probe according to claim 1 for real-time monitoring of intracellular calcium ions, represented by Formula 1:

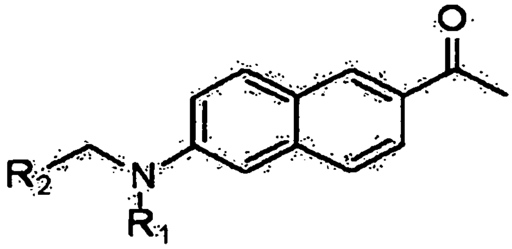

(1)

wherein $R_1$ is $CH_3$ or H, and $R_2$ is

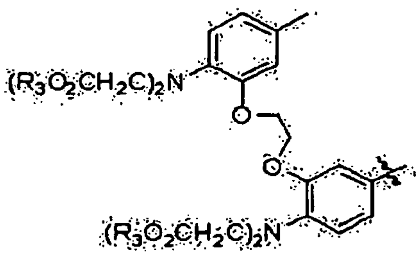

($R_3$ = H or $CH_2OCOCH_3$),

the method comprising reacting a compound of Formula 2:

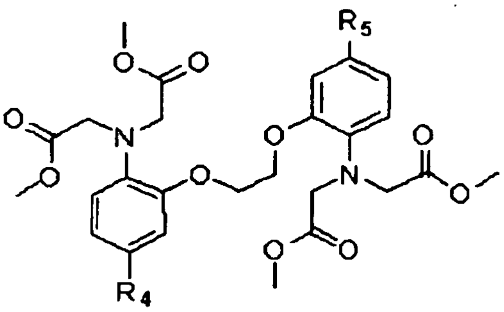

(2)

wherein $R_4$ is $NH_2$ or CHO and $R_5$ is H or $CH_3$, with a compound of Formula 3:

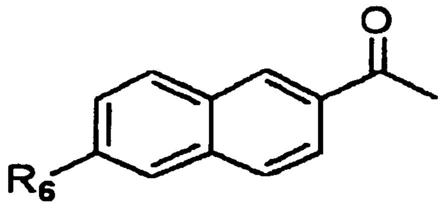

(3)

wherein $R_6$ is $NH_2$ or

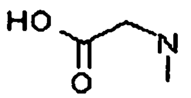

4. The method according to claim 3, wherein the hydrogen atoms in $R_3$ are replaced with $CH_2OCOCH_3$ by reacting bromomethyl acetate and triethylamine with the compound of Formula 1 ($R_3$ = H).

5. A method for real-time monitoring of intracellular calcium ions, the method comprising the steps introducing the two-photon probe according to claim 1 or 2 into cells of interest and imaging two-photon excited fluorescence emitted from the two-photon probe.

6. The method according to claim 5, wherein the intracellular calcium ion concentration is quantitatively determined by Equation 1:

$$[Ca^{2+}] = K_d[(F - F_{min})/(F_{max} - F)] \qquad (1)$$

where $K_d$ is the dissociation constant of the two-photon probe for $Ca^{2+}$, F is the observed two-photon fluorescence intensity, $F_{min}$ is the minimum fluorescence intensity, and $F_{max}$ is the maximum fluorescence intensity.

7. The method according to claim 5, wherein the two-photon excited fluorescence images are collected at wavelengths ranging from 500 to 620 nm.

**Patentansprüche**

1. Zweiphotonensonde zur Echtzeitüberwachung intrazellulärer Calziumionen, dargestellt durch Formel 1:

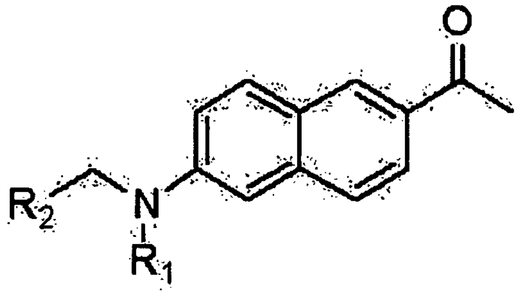

(1)

wobei $R_1 = CH_3$ oder H, und
$R_2$ =

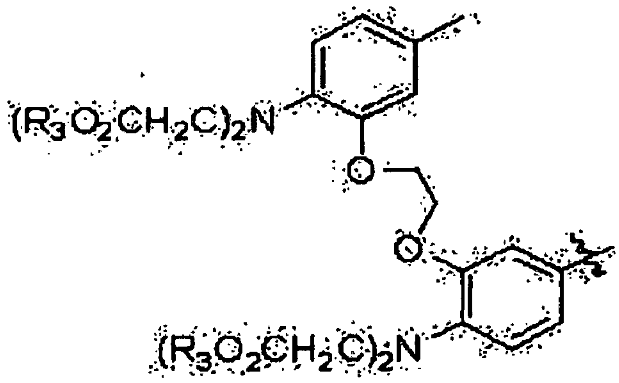

($R_3$ = H oder $CH_2OCOCH_3$).

2. Zweiphotonensonde nach Anspruch 1, wobei $R_3 = CH_2OCOCH_3$.

3. Verfahren zur Herstellung einer Zweiphotonensonde nach Anspruch 1 zur Echtzeitüberwachung intrazellulärer Calziumionen, dargestellt durch Formel 1:

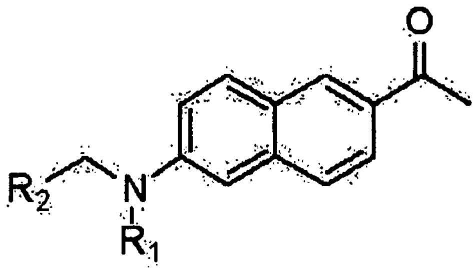

(1)

wobei $R_1 = CH_3$ oder H, und $R_2$ =

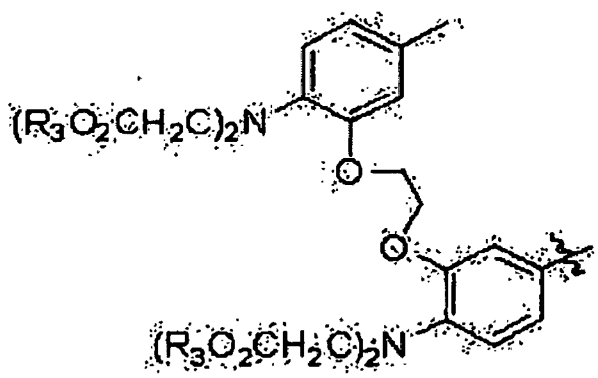

($R_3$ = H oder $CH_2OCOCH_3$).

wobei das Verfahren umfasst, dass eine Verbindung von Formel 2:

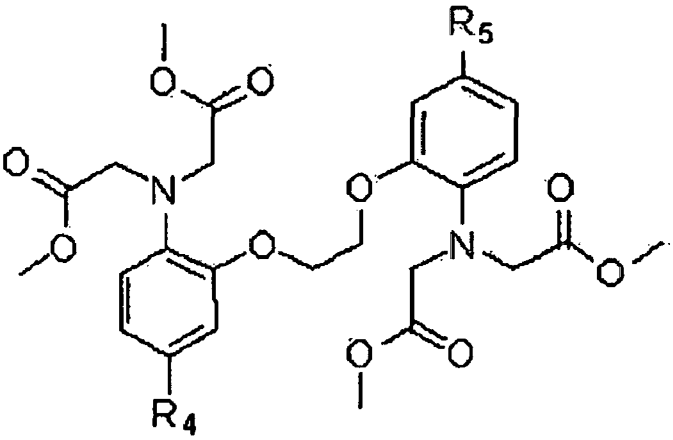

wobei $R_4$ = $NH_2$ oder CHO, und $R_5$ = H oder $CH_3$,
zur Reaktion gebracht wird mit einer Verbindung von Formel 3:

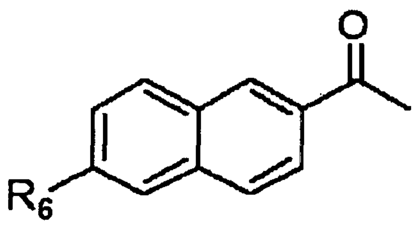

(3)

wobei $R_6$ = $NH_2$ oder

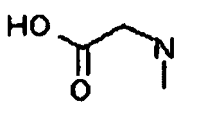

.

**4.** Verfahren nach Anspruch 3, wobei die Wasserstoffatome in $R_3$ ersetzt werden durch $CH_2OCOCH_3$, und zwar dadurch, dass Brom-Methylacetat und Triethylamin mit der Verbindung von Formel 1 ($R_3$ = H) zur Reaktion gebracht werden.

5. Verfahren zur Echtzeitüberwachung intrazellulärer Calziumionen, wobei das Verfahren die Schritte umfasst, dass die Zweiphotonensonde nach Anspruch 1 oder 2 in interessierende Zellen eingebracht wird und die durch Zweiphotonenanregung erzeugte Fluoreszenz, die von der Zweiphotonensonde emittiert wird, bildlich dargestellt wird.

6. Verfahren nach Anspruch 5, wobei die Konzentration intrazellulärer Calziumionen quantitativ bestimmt wird durch Gleichung 1:

$$[Ca^{2+}] = K_d[(F-F_{min})/(F_{max}-F)] \quad (1)$$

wobei $K_d$ die Dissoziationskonstante der Zweiphotonensonde für $Ca^{2+}$ ist, F die beobachtete Zweiphotonenfluoreszenzstärke ist, $F_{min}$ die minimale Fluoreszenzstärke ist, und $F_{max}$ die maximale Fluoreszenzstärke ist.

7. Verfahren nach Anspruch 5, wobei die Bilder einer durch Zweiphotonenanregung erzeugten Fluoreszenz bei Wellenlängen zwischen 500 bis 620 nm aufgefangen werden.

## Revendications

1. Sonde à deux photons pour la surveillance en temps réel d'ions calcium intracellulaires, représentée par la Formule 1 :

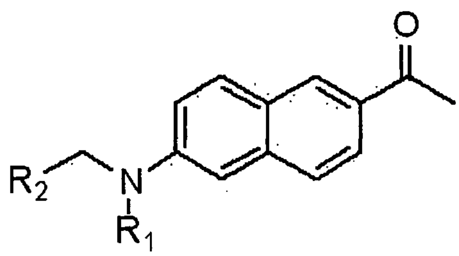

**(1)**

dans laquelle $R_1$ est $CH_3$ ou H, et
$R_2$ est

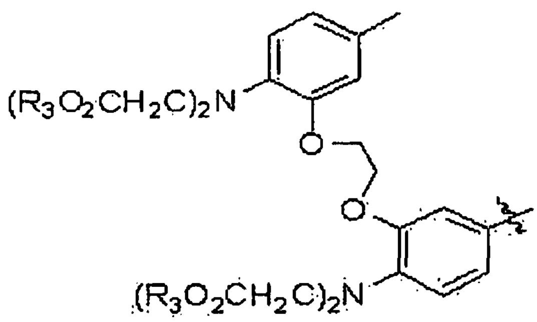

($R_3$ = H ou $CH_2OCOCH_3$).

2. Sonde à deux photons selon la revendication 1, dans laquelle $R_3$ est $CH_2OCOCH_3$.

3. Procédé pour préparer une sonde à deux photons selon la revendication 1 pour la surveillance en temps réel d'ions calcium intracellulaire, représentée par la Formule 1 :

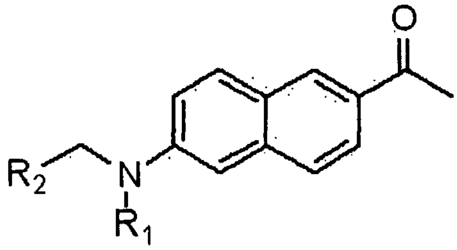

**(1)**

dans laquelle $R_1$ est $CH_3$ ou H, et $R_2$ est

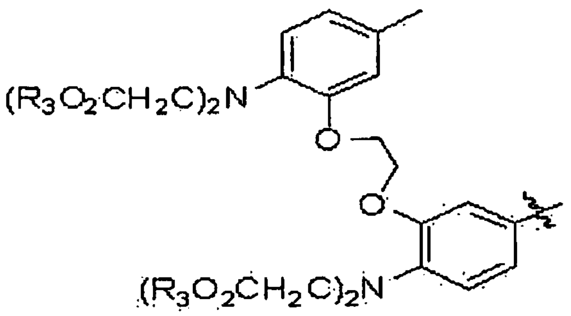

($R_3$ = H ou $CH_2OCOCH_3$),

le procédé comprenant la réaction d'un composé de Formule 2 :

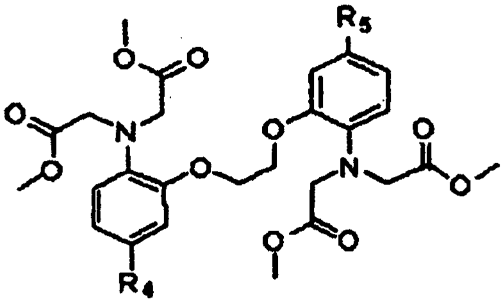

**(2)**

dans laquelle $R_4$ est $NH_2$ ou CHO et $R_5$ est H ou $CH_3$, avec un composé de Formule 3 :

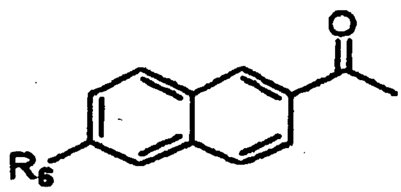

**(3)**

dans laquelle $R_6$ est $NH_2$ ou

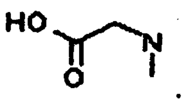

**4.** Procédé selon la revendication 3, dans lequel les atomes d'hydrogène dans $R_3$ sont remplacés par $CH_2OCOCH_3$ en faisant réagir de l'acétate de bromométhyle et de la triéthylamine avec le composé de Formule 1 ($R_3$ = H).

**5.** Procédé pour la surveillance en temps réel d'ions calcium intracellulaires, le procédé comprenant les étapes consistant à introduire la sonde à deux photons selon la revendication 1 ou 2 dans des cellules d'intérêt et à imager la fluorescence excitée à deux photons émise par la sonde à deux photons.

**6.** Procédé selon la revendication 5, dans lequel la concentration en ions calcium intracellulaires est déterminée quantitativement par l'Equation 1 :

$$[Ca^{2+}] = K_d[(F - F_{min})/(F_{max} - F)] \quad (1)$$

dans laquelle $K_d$ est la constante de dissociation de la sonde à deux photons pour $Ca^{2+}$, F est l'intensité de fluorescence à deux photons observée, $F_{min}$ est l'intensité de fluorescence minimale et $F_{max}$ est l'intensité de fluorescence maximale.

**7.** Procédé selon la revendication 5, dans lequel les images de fluorescence excitée à deux photons sont recueillies à des longueurs d'onde allant de 500 à 620 nm.

【Figure 1】

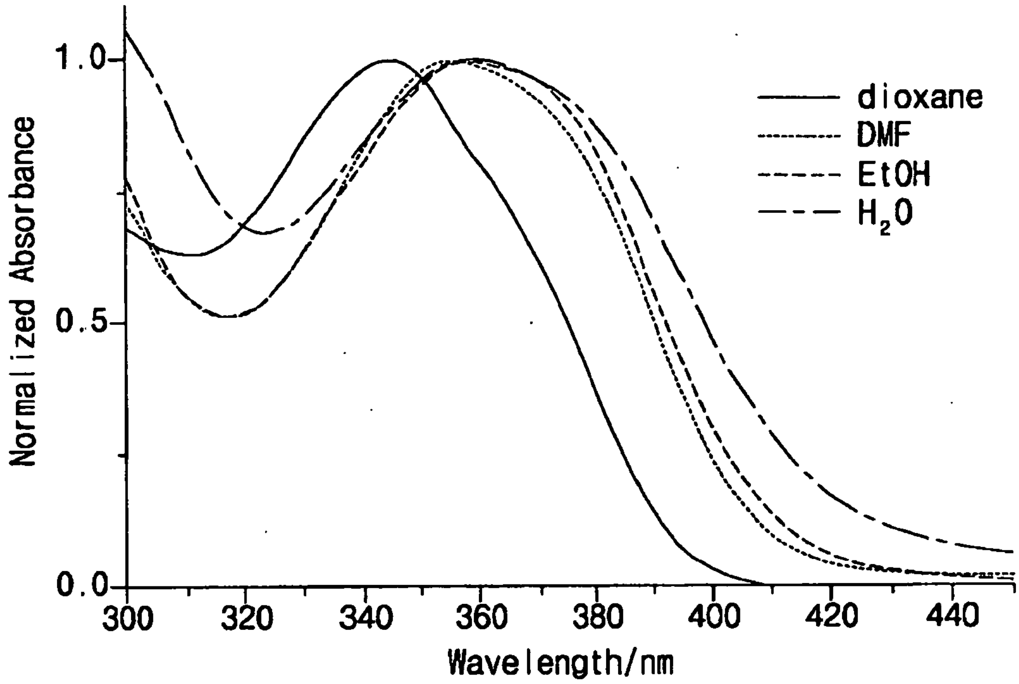
1.0
0.5
0.0
Normalized Absorbance
300
320
340
360
380
400
420
440
Wavelength/nm
dioxane
DMF
EtOH
H2O

(a)

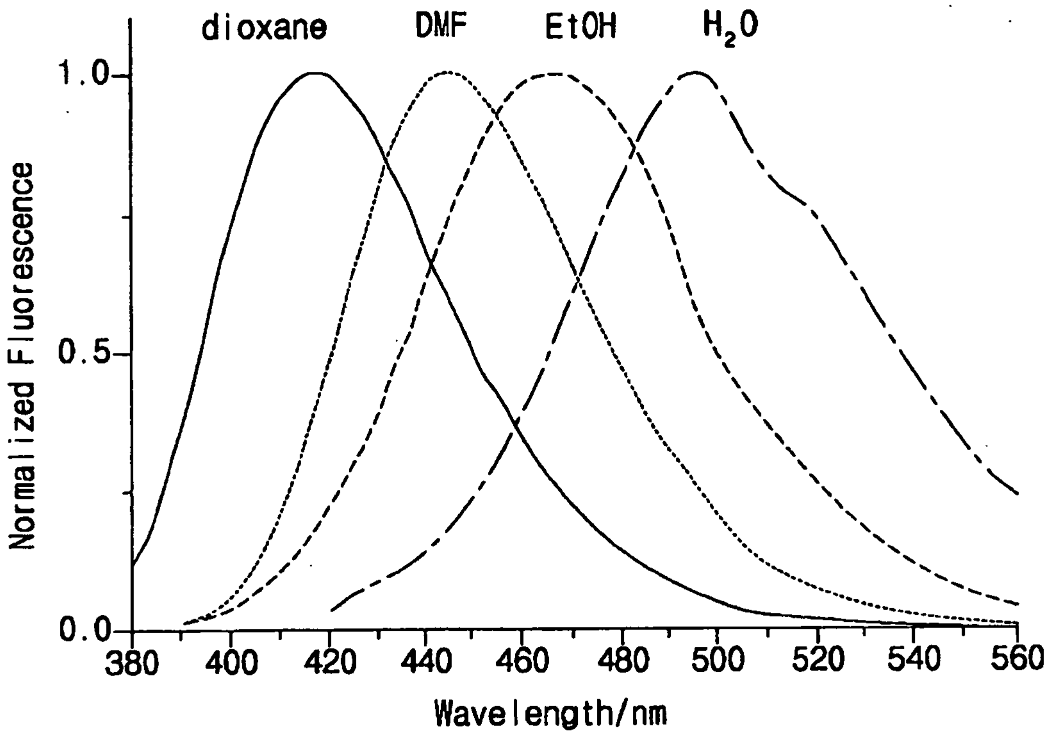
dioxane
DMF
EtOH
$H_2O$
1.0
0.5
0.0
Normalized Fluorescence
380
400
420
440
460
480
500
520
540
560
Wavelength/nm

(b)

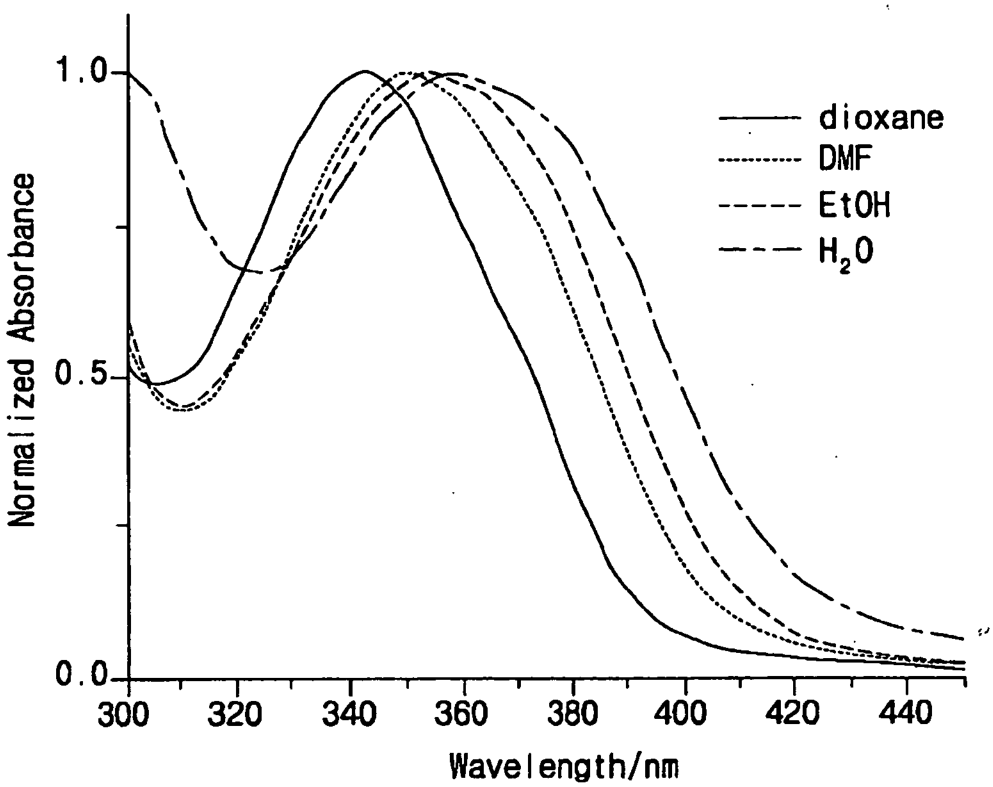
1.0
0.5
0.0
Normalized Absorbance
300
320
340
360
380
400
420
440
Wavelength/nm
dioxane
DMF
EtOH
H2O

(c)

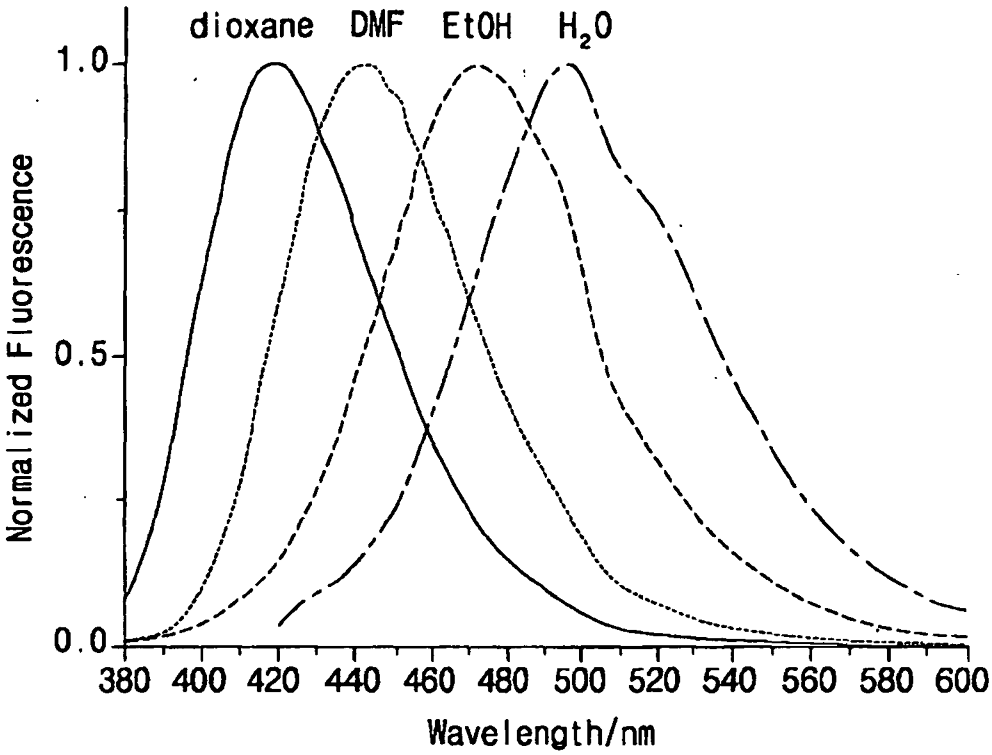
dioxane
DMF
EtOH
H2O
1.0
0.5
0.0
Normalized Fluorescence
380
400
420
440
460
480
500
520
540
560
580
600
Wavelength/nm

(d)

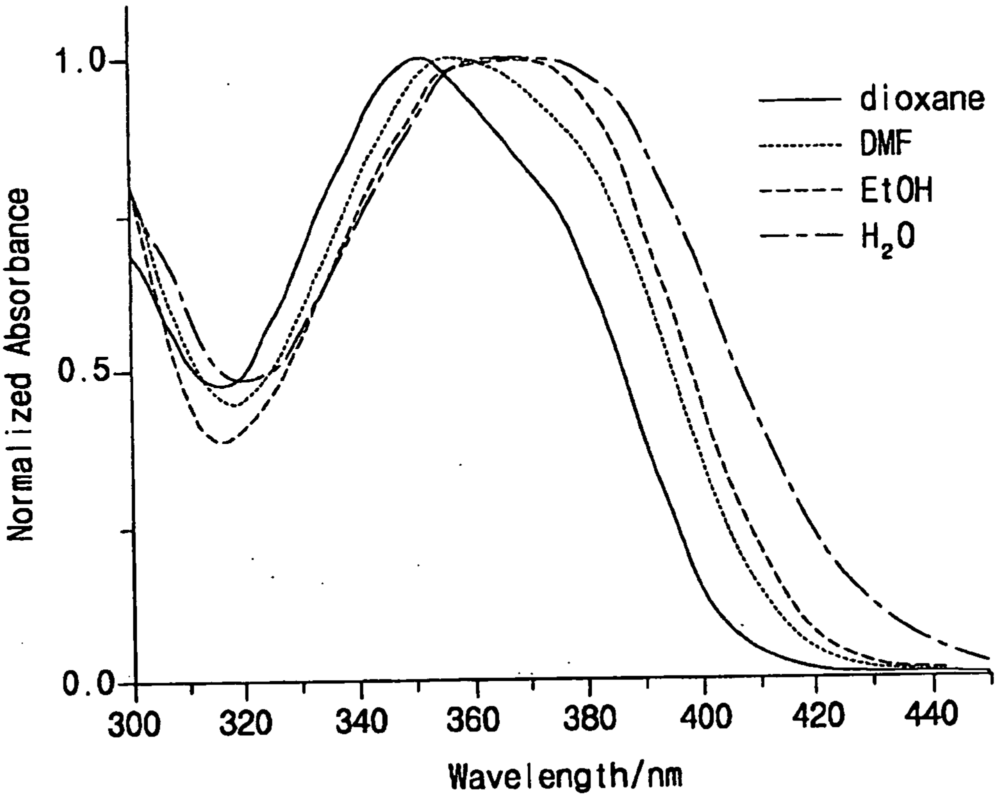
1.0
0.5
0.0
Normalized Absorbance
300
320
340
360
380
400
420
440
Wavelength/nm
dioxane
DMF
EtOH
H2O

(e)

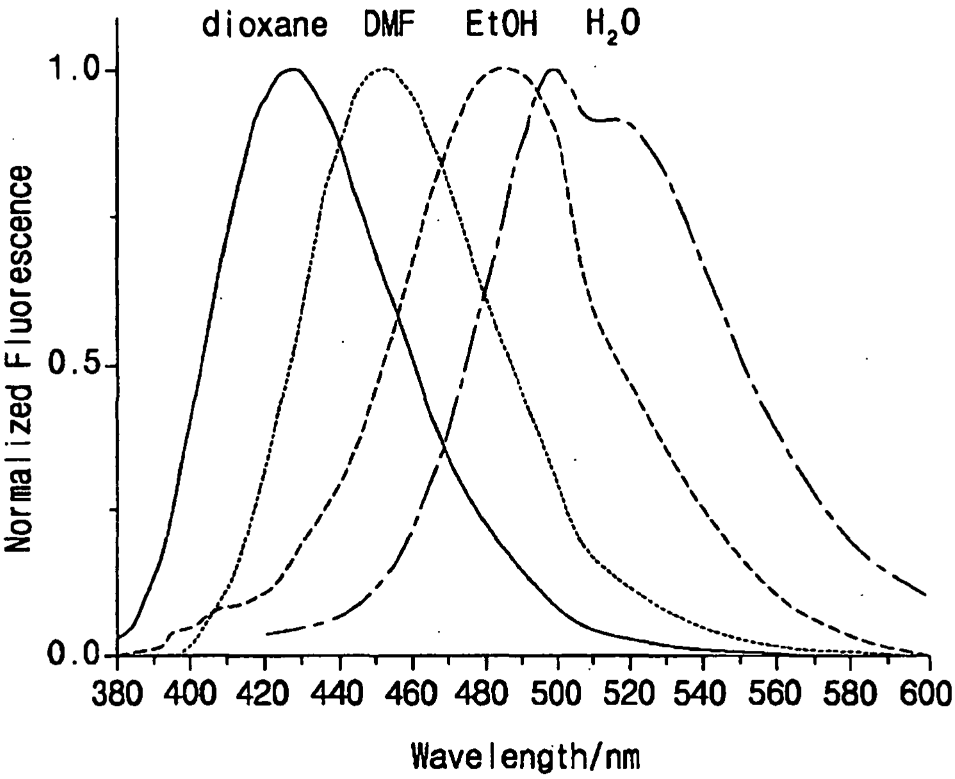
dioxane
DMF
EtOH
$H_2O$
1.0
0.5
0.0
380
400
420
440
460
480
500
520
540
560
580
600
Normalized Fluorescence
Wavelength/nm

(f)

【Figure 2】

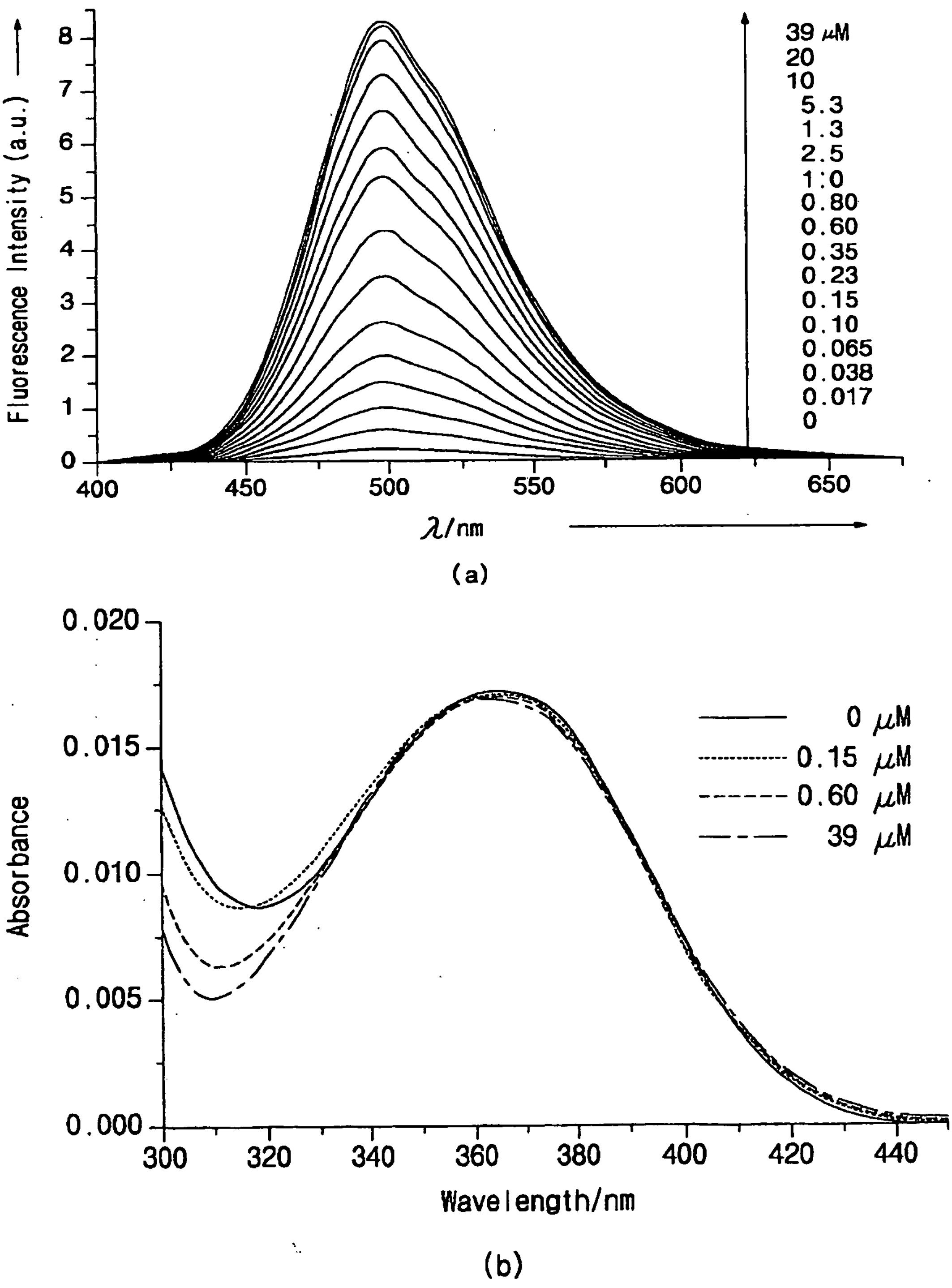
Fluorescence Intensity (a.u.)
0
1
2
3
4
5
6
7
8
400
450
500
550
600
650
λ/nm
39 μM
20
10
5.3
1.3
2.5
1.0
0.80
0.60
0.35
0.23
0.15
0.10
0.065
0.038
0.017
0
(a)
Absorbance
0.000
0.005
0.010
0.015
0.020
300
320
340
360
380
400
420
440
Wavelength/nm
0 μM
0.15 μM
0.60 μM
39 μM
(b)

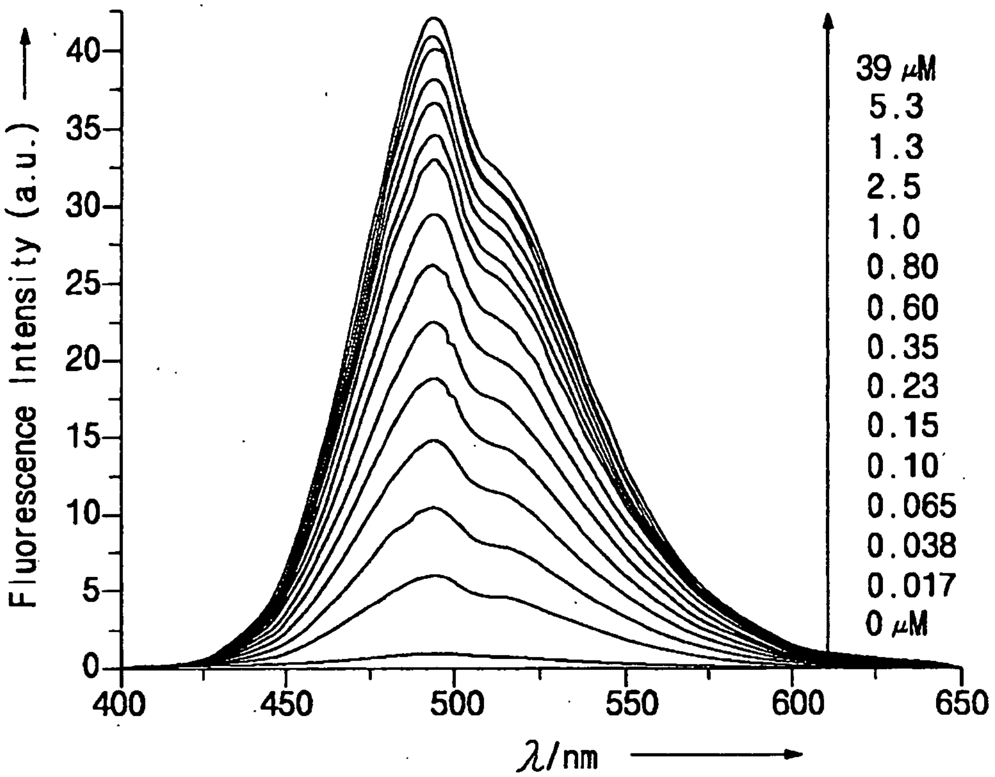
Fluorescence Intensity (a.u.)
0
5
10
15
20
25
30
35
40
400
450
500
550
600
650
λ/nm
39 μM
5.3
1.3
2.5
1.0
0.80
0.60
0.35
0.23
0.15
0.10
0.065
0.038
0.017
0 μM

(c)

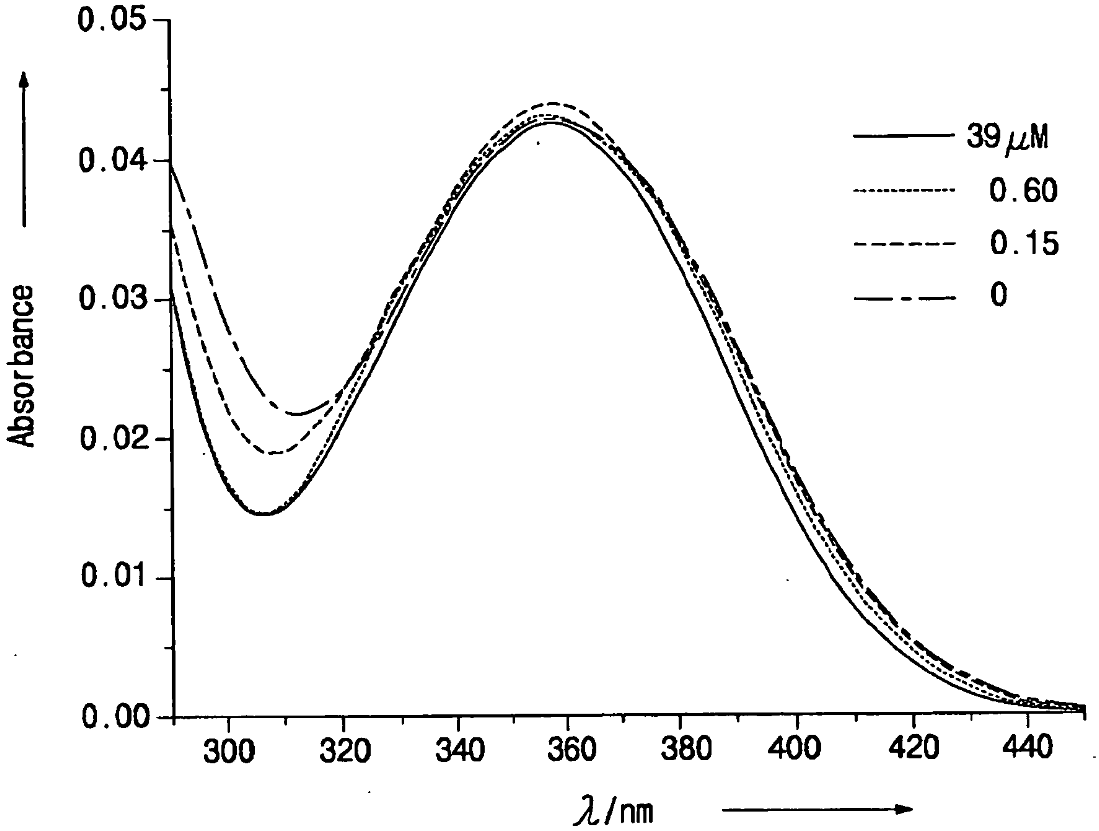
0.05
0.04
0.03
0.02
0.01
0.00
Absorbance
300
320
340
360
380
400
420
440
λ/nm
39 μM
0.60
0.15
0

(d)

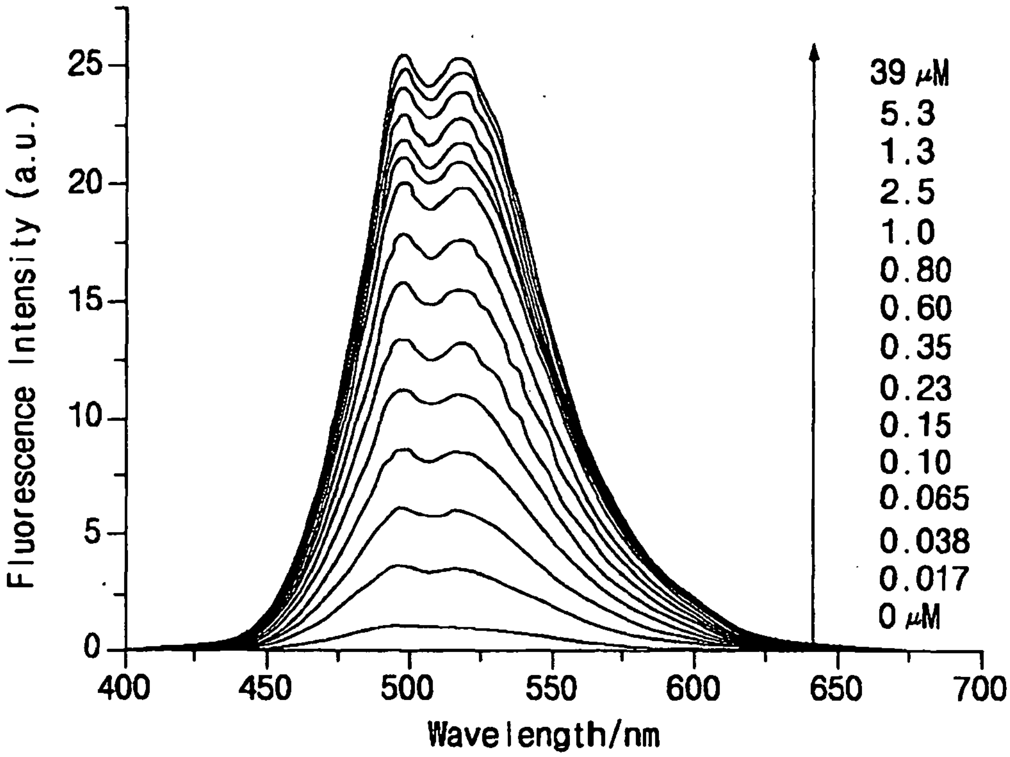

Fluorescence Intensity (a.u.)
Wavelength/nm
39 μM
5.3
1.3
2.5
1.0
0.80
0.60
0.35
0.23
0.15
0.10
0.065
0.038
0.017
0 μM


(e)

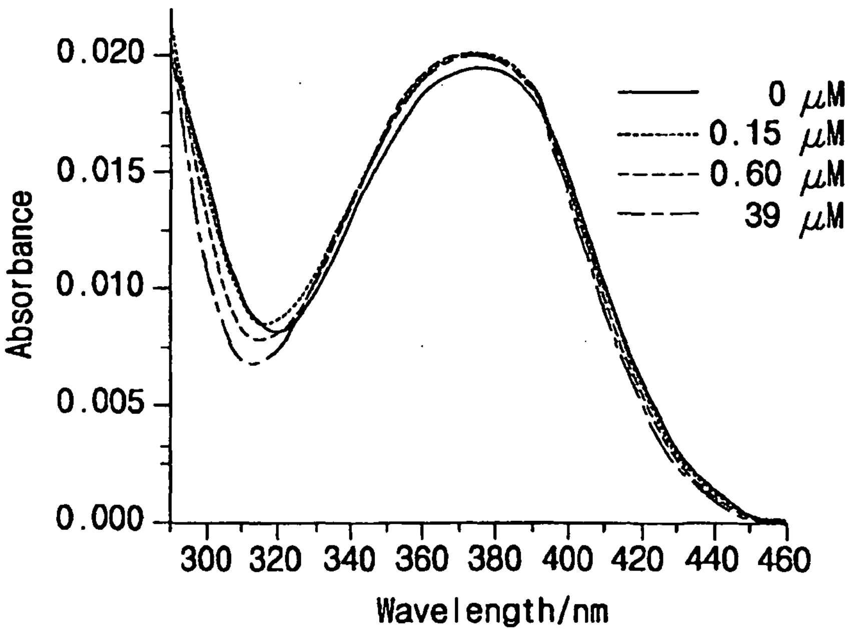

Absorbance
Wavelength/nm
0 μM
0.15 μM
0.60 μM
39 μM


(f)

【Figure 3】

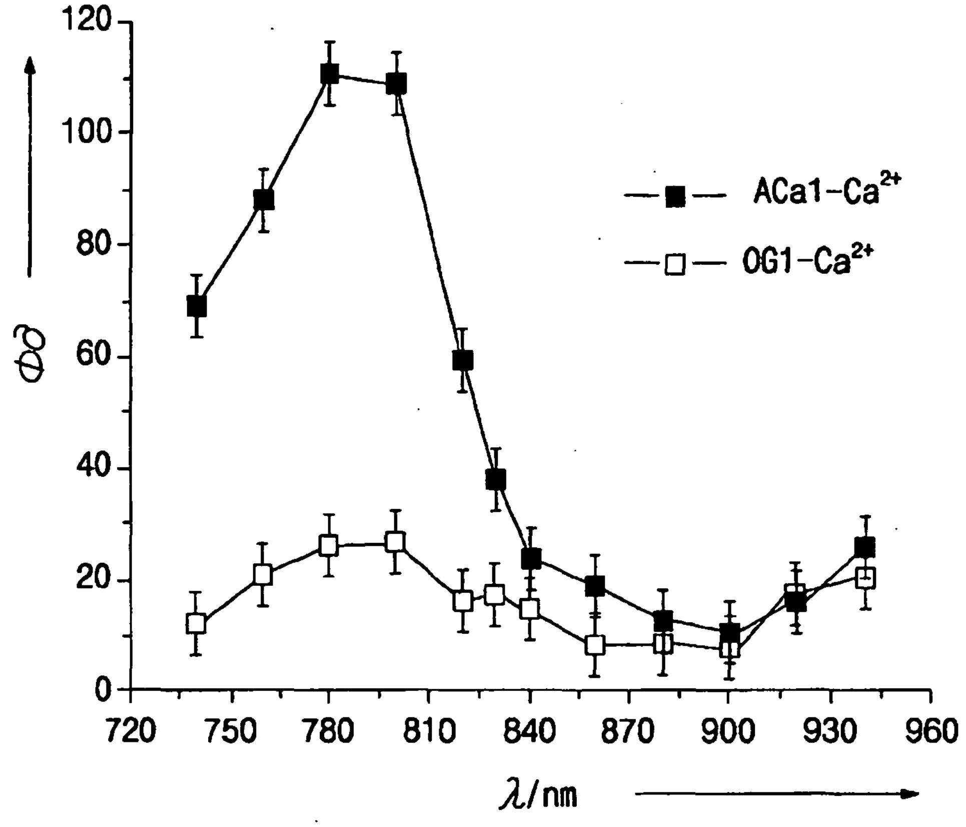
120
100
80
60
40
20
0
Φδ
720
750
780
810
840
870
900
930
960
λ/nm
ACa1-Ca2+
OG1-Ca2+

(a)

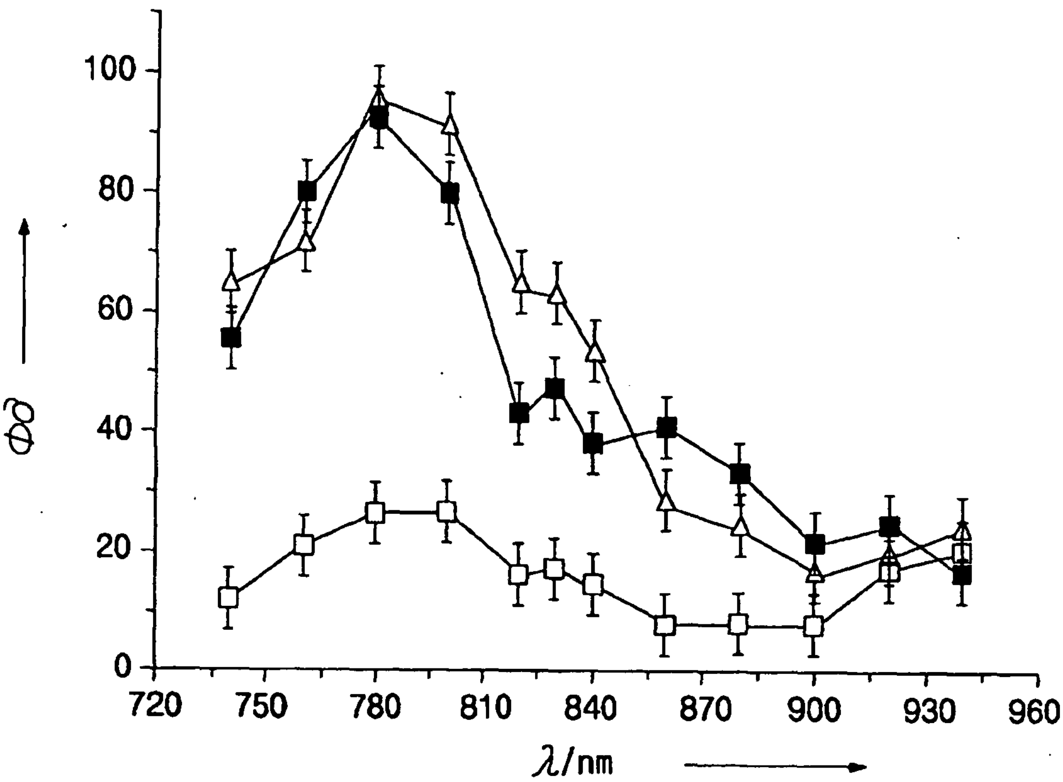


(b)

【Figure 4】

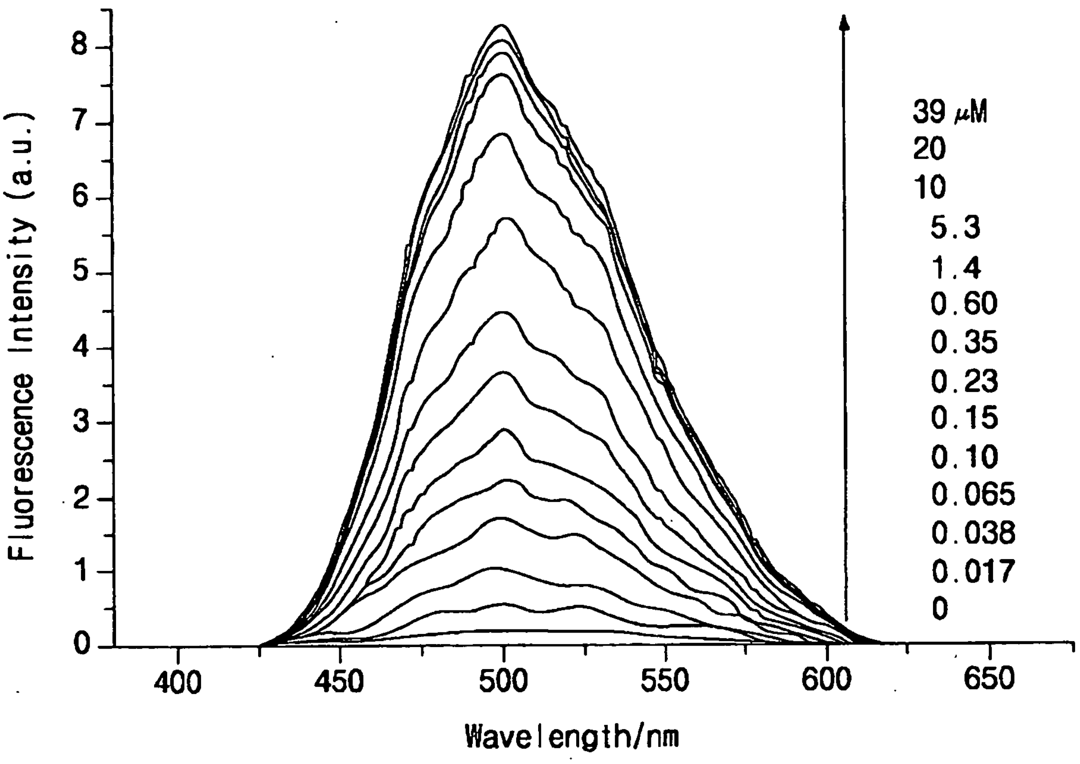

8
7
6
5
4
3
2
1
0
Fluorescence Intensity (a.u.)
400
450
500
550
600
650
Wavelength/nm
39 μM
20
10
5.3
1.4
0.60
0.35
0.23
0.15
0.10
0.065
0.038
0.017
0


(a)

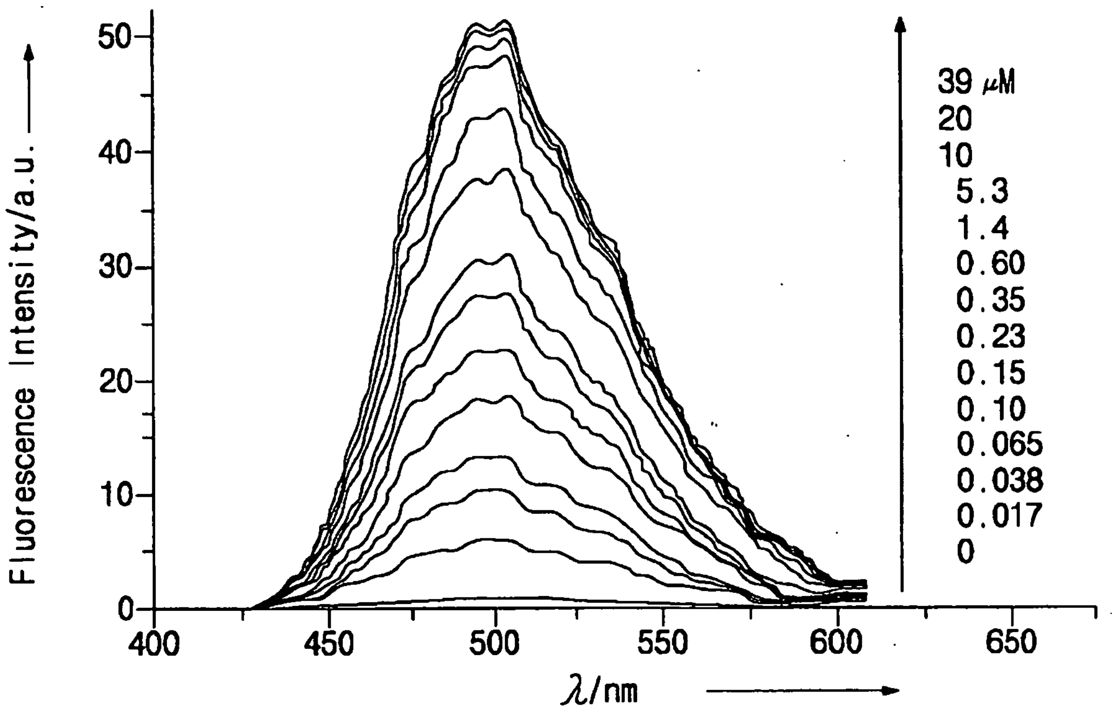
Fluorescence Intensity/a.u.
50
40
30
20
10
0
400
450
500
550
600
650
λ/nm
39 μM
20
10
5.3
1.4
0.60
0.35
0.23
0.15
0.10
0.065
0.038
0.017
0

(b)

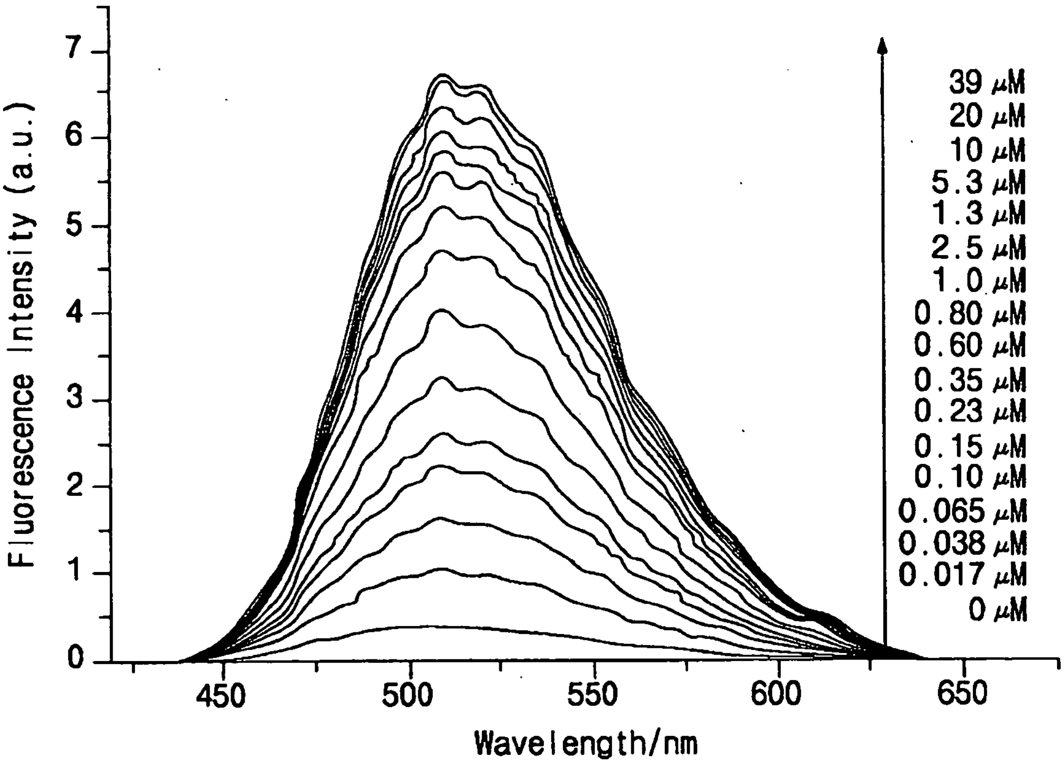
Fluorescence Intensity (a.u.)
0
1
2
3
4
5
6
7
450
500
550
600
650
Wavelength/nm
39 μM
20 μM
10 μM
5.3 μM
1.3 μM
2.5 μM
1.0 μM
0.80 μM
0.60 μM
0.35 μM
0.23 μM
0.15 μM
0.10 μM
0.065 μM
0.038 μM
0.017 μM
0 μM

(c)

【Figure 5】

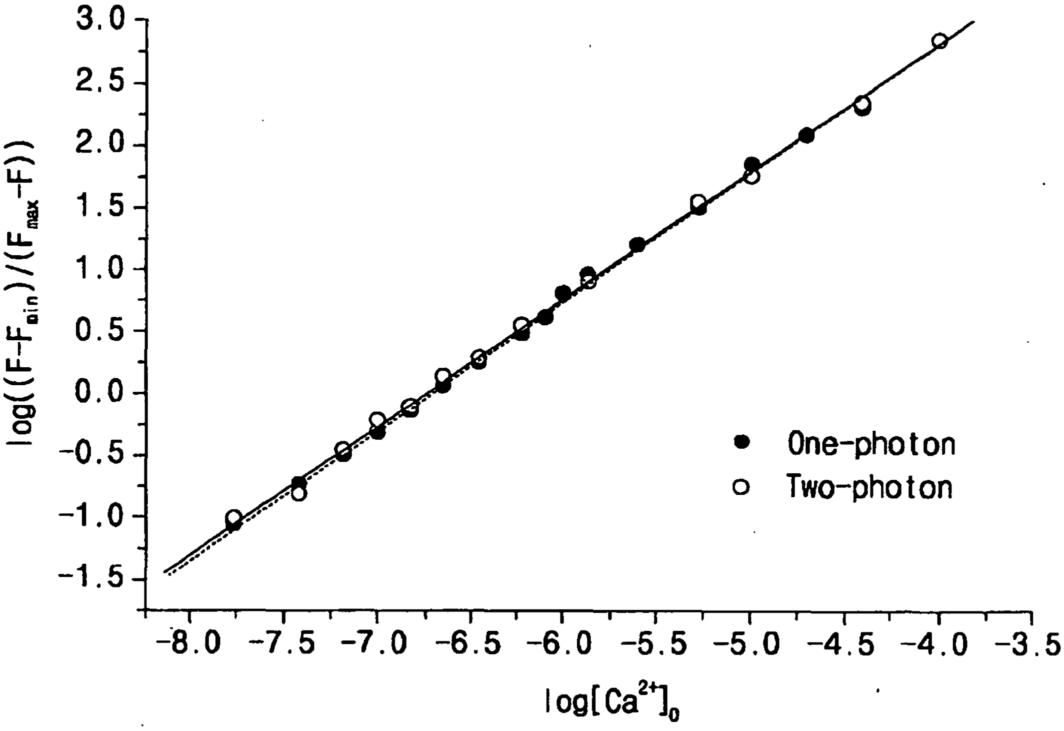
3.0
2.5
2.0
1.5
1.0
0.5
0.0
-0.5
-1.0
-1.5
log((F-F_min)/(F_max-F))
One-photon
Two-photon
-8.0
-7.5
-7.0
-6.5
-6.0
-5.5
-5.0
-4.5
-4.0
-3.5
log[Ca2+]0

(a)

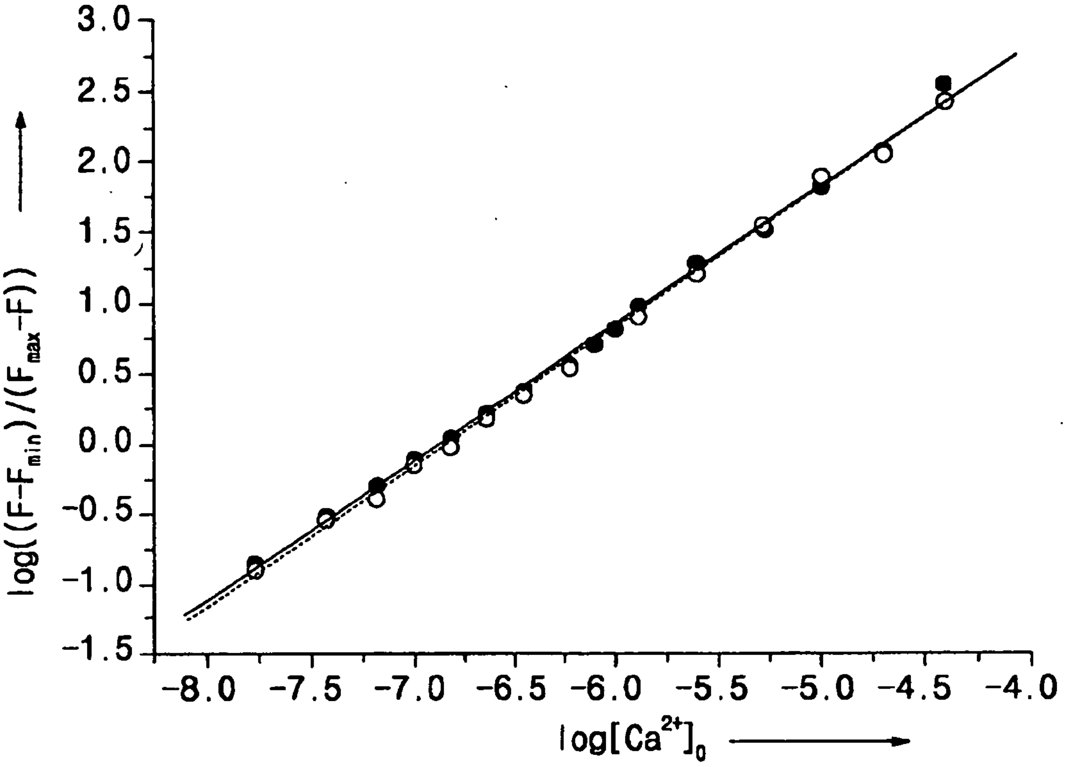
3.0
2.5
2.0
1.5
1.0
0.5
0.0
-0.5
-1.0
-1.5
log((F-F_min)/(F_max-F))
-8.0
-7.5
-7.0
-6.5
-6.0
-5.5
-5.0
-4.5
-4.0
log[Ca2+]0

(b)

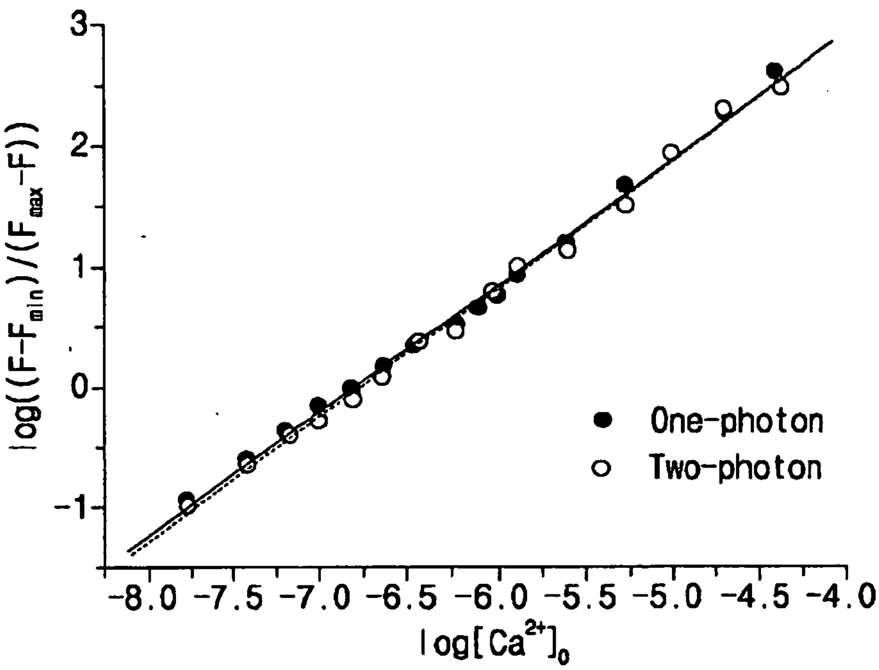
3
2
1
0
-1
log((F-F$_{min}$)/(F$_{max}$-F))
One-photon
Two-photon
-8.0 -7.5 -7.0 -6.5 -6.0 -5.5 -5.0 -4.5 -4.0
log[Ca$^{2+}$]$_0$

(c)

【Figure 6】

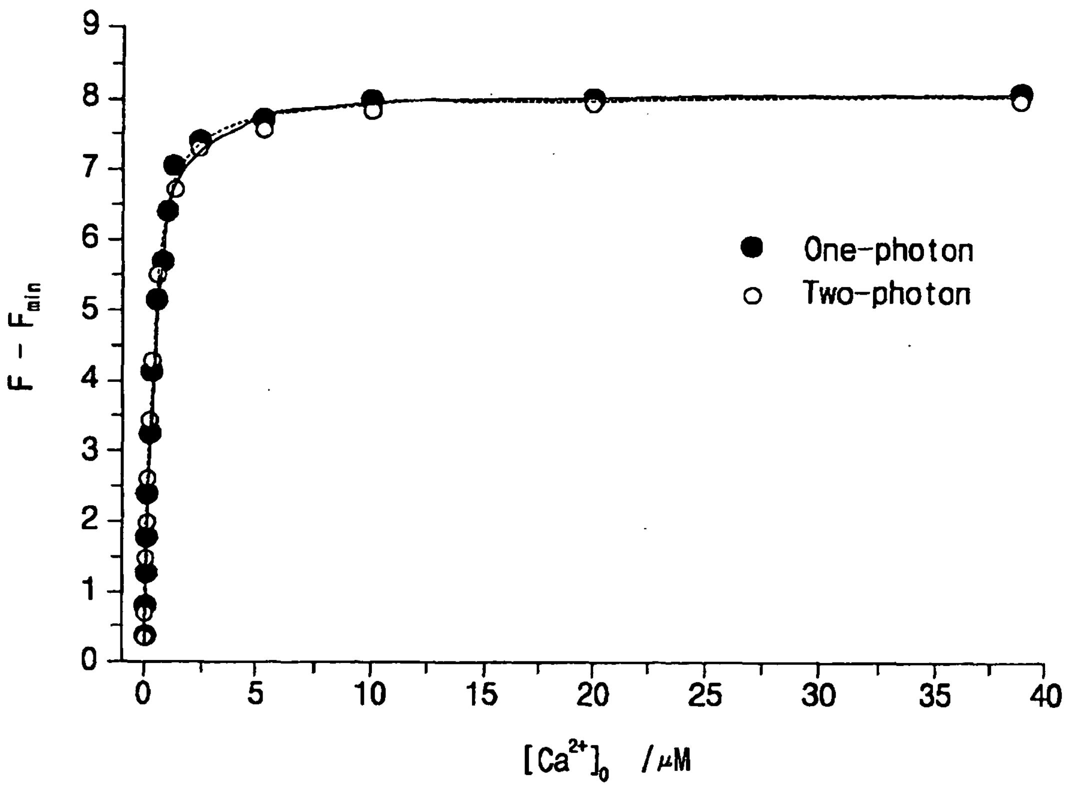
9
8
7
6
5
4
3
2
1
0
F - Fmin
0
5
10
15
20
25
30
35
40
[Ca2+]0 /μM
One-photon
Two-photon

(a)

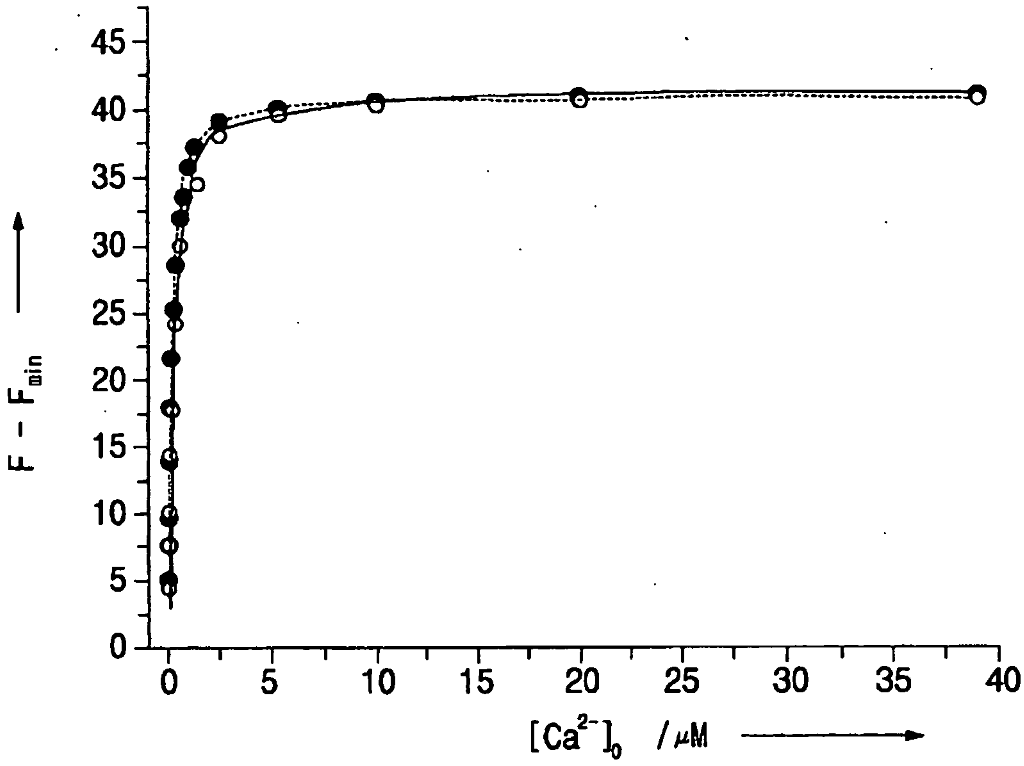


(b)

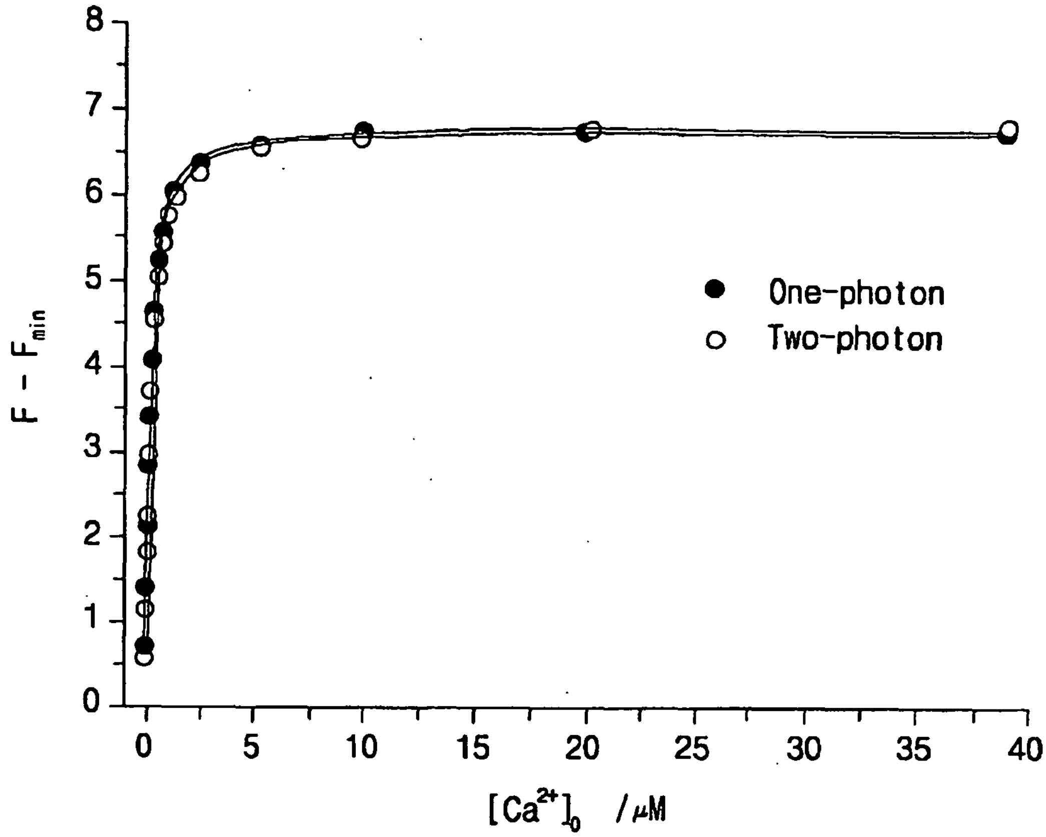
8
7
6
5
4
3
2
1
0
F - $F_{min}$
0
5
10
15
20
25
30
35
40
$[Ca^{2+}]_0$ /μM
One-photon
Two-photon

(c)

【Figure 7】

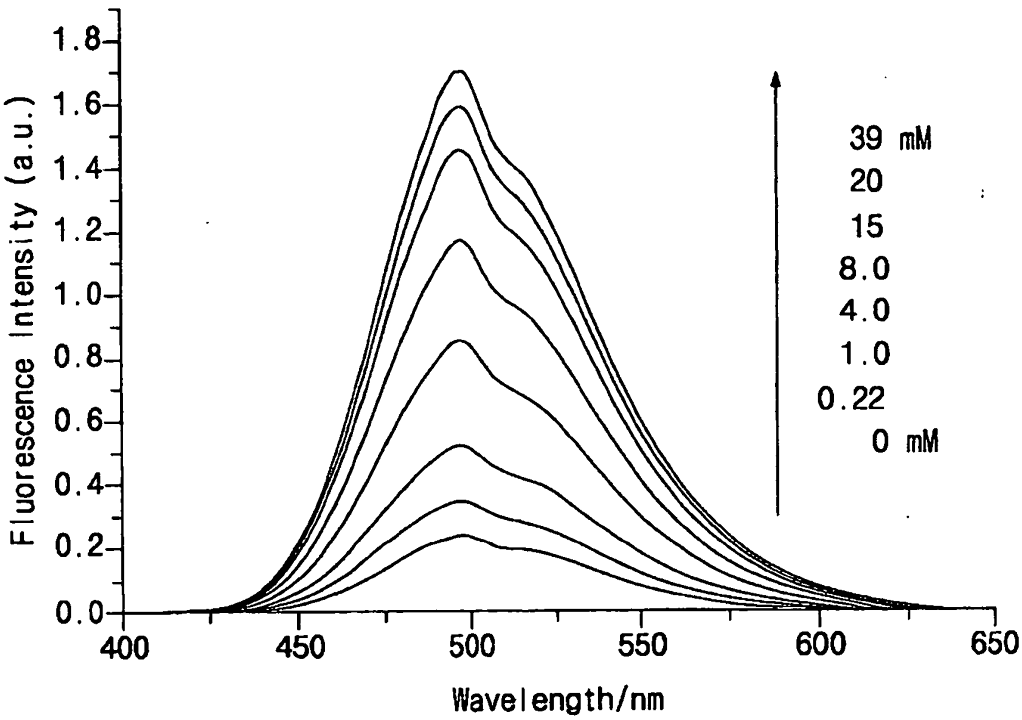
1.8
1.6
1.4
1.2
1.0
0.8
0.6
0.4
0.2
0.0
Fluorescence Intensity (a.u.)
400
450
500
550
600
650
Wavelength/nm
39 mM
20
15
8.0
4.0
1.0
0.22
0 mM

【Figure 8】

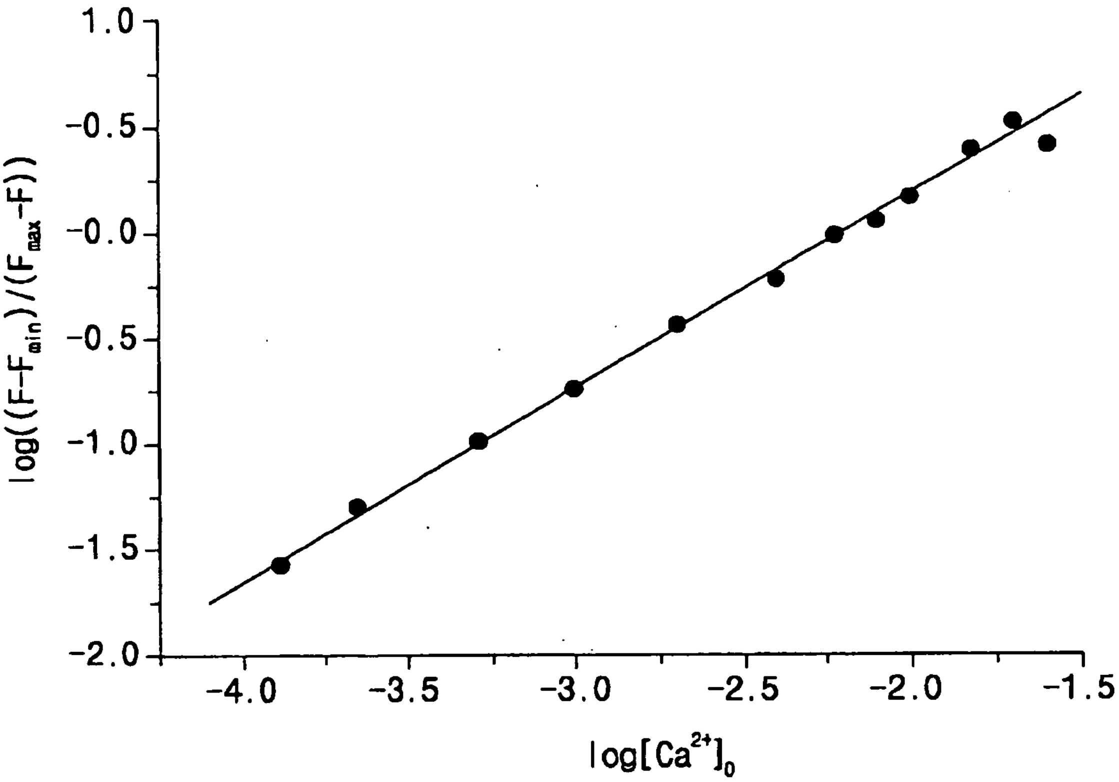

【Figure 9】

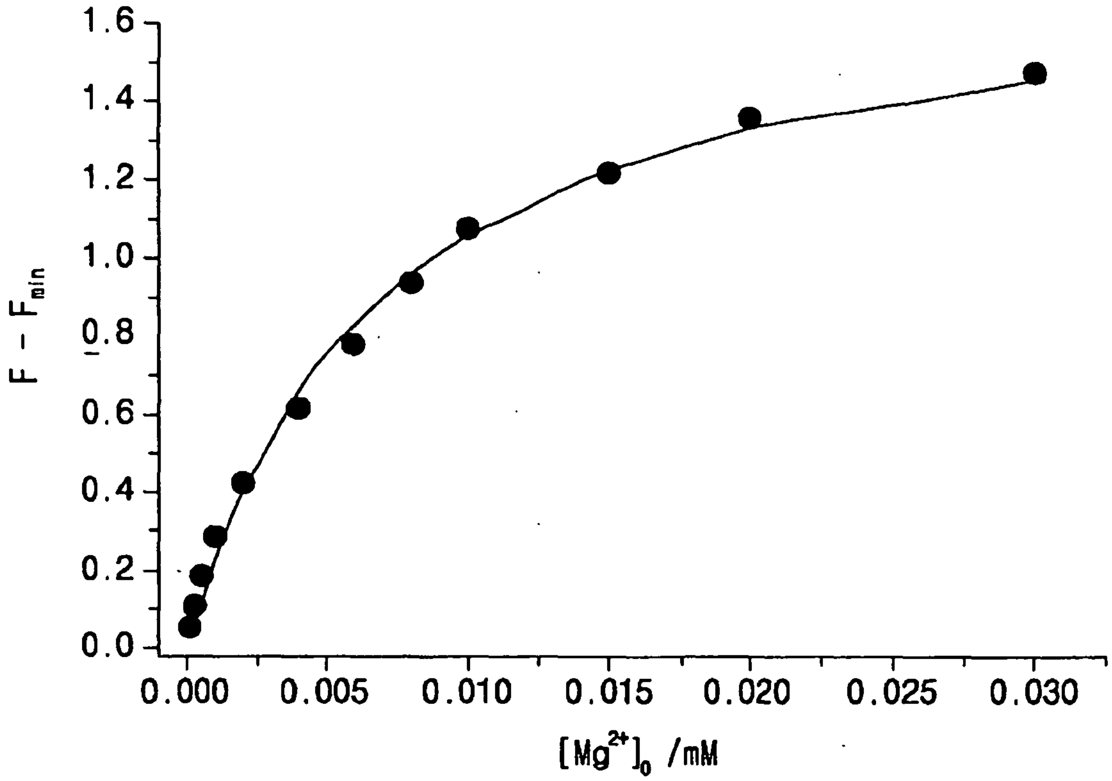
1.6
1.4
1.2
1.0
0.8
0.6
0.4
0.2
0.0
F - $F_{min}$
0.000
0.005
0.010
0.015
0.020
0.025
0.030
$[Mg^{2+}]_0$ /mM

【Figure 10】

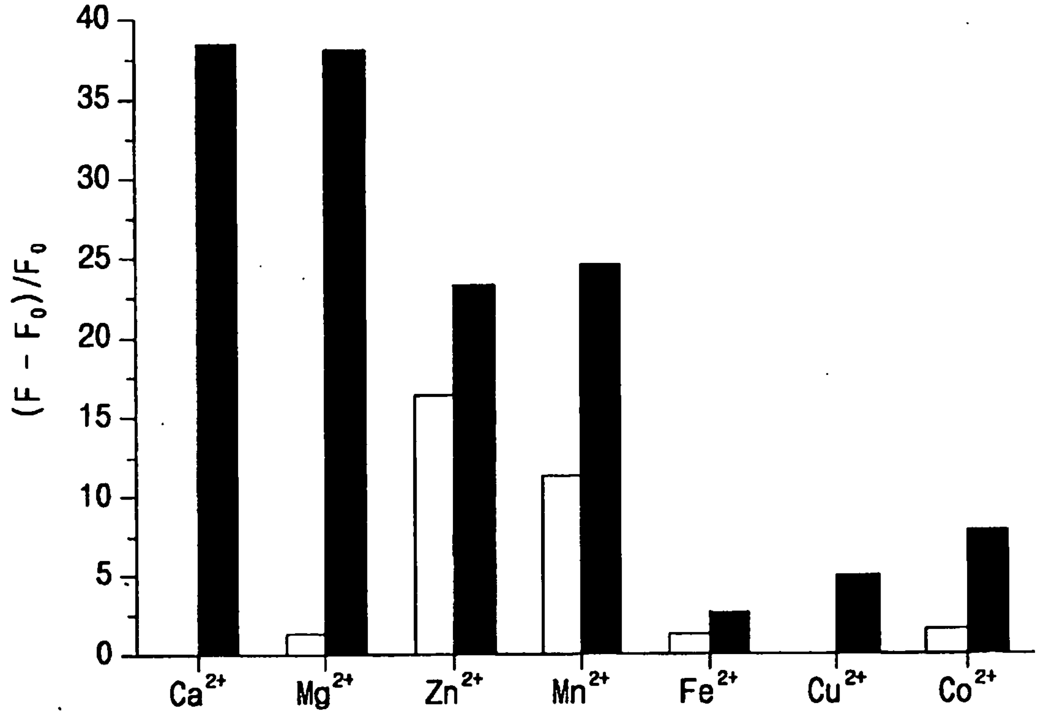


(a)

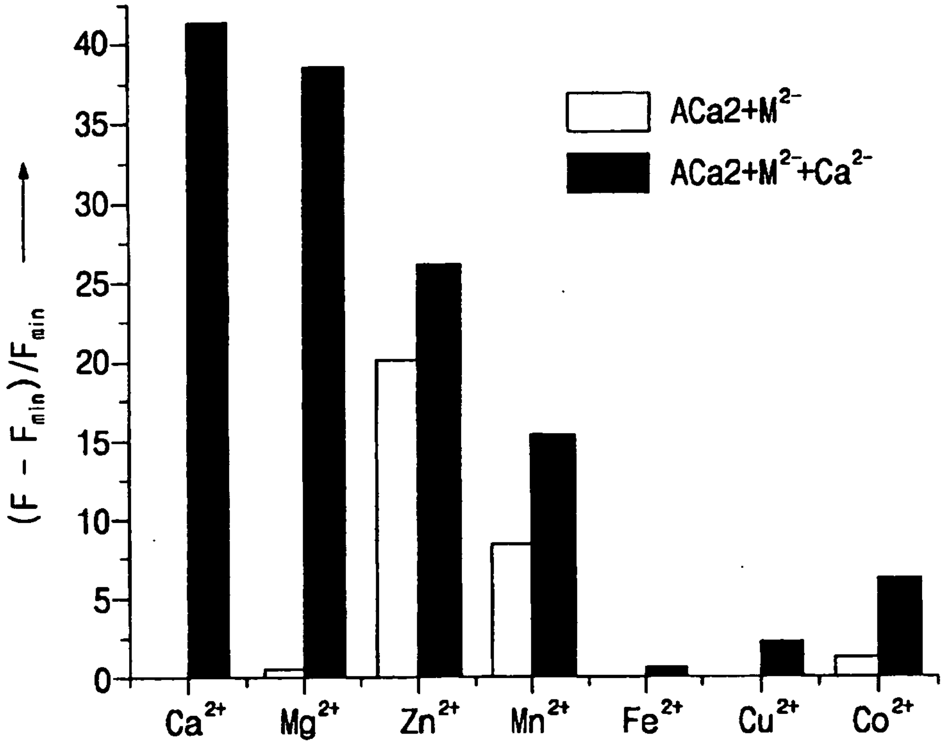

40
35
30
25
20
15
10
5
0
(F - F_min)/F_min
ACa2+M2-
ACa2+M2-+Ca2-
Ca2+
Mg2+
Zn2+
Mn2+
Fe2+
Cu2+
Co2+


(b)

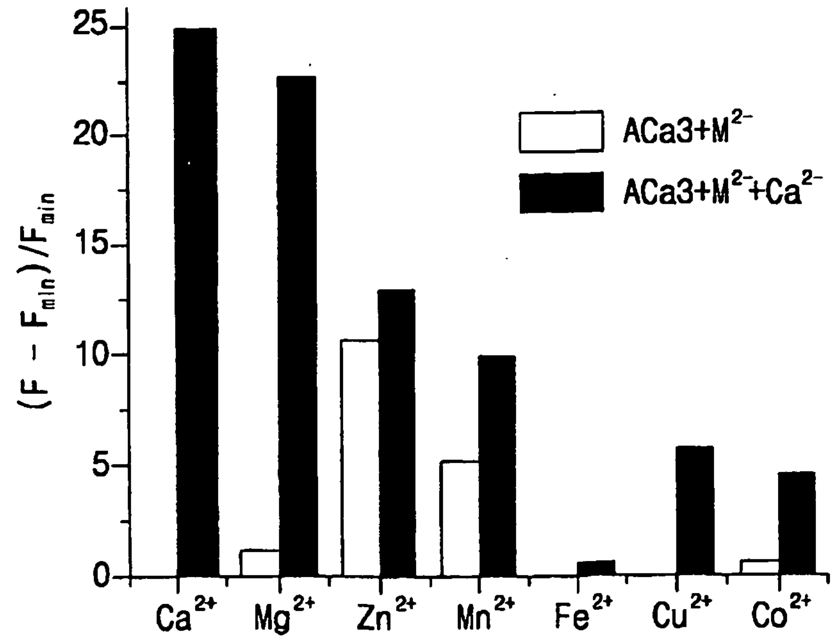

25
20
15
10
5
0
(F - F_min)/F_min
ACa3+M2-
ACa3+M2-+Ca2-
Ca2+
Mg2+
Zn2+
Mn2+
Fe2+
Cu2+
Co2+


(c)

【Figure 11】

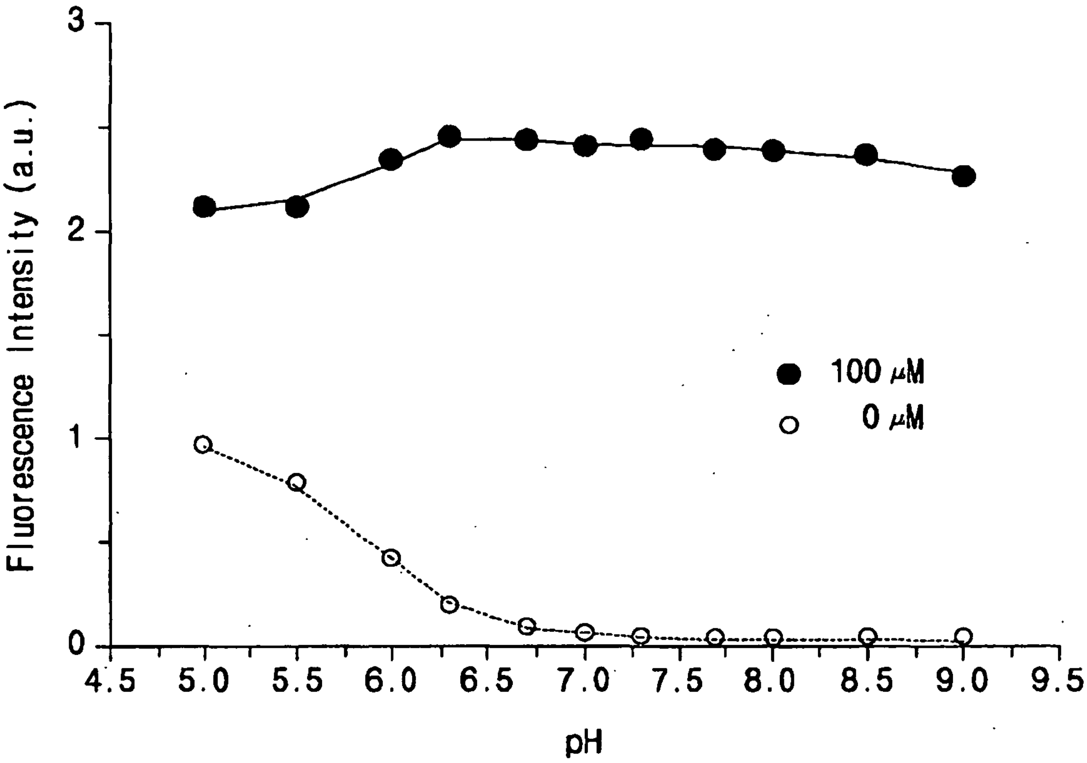
3
2
1
0
Fluorescence Intensity (a.u.)
4.5
5.0
5.5
6.0
6.5
7.0
7.5
8.0
8.5
9.0
9.5
pH
100 μM
0 μM

(a)

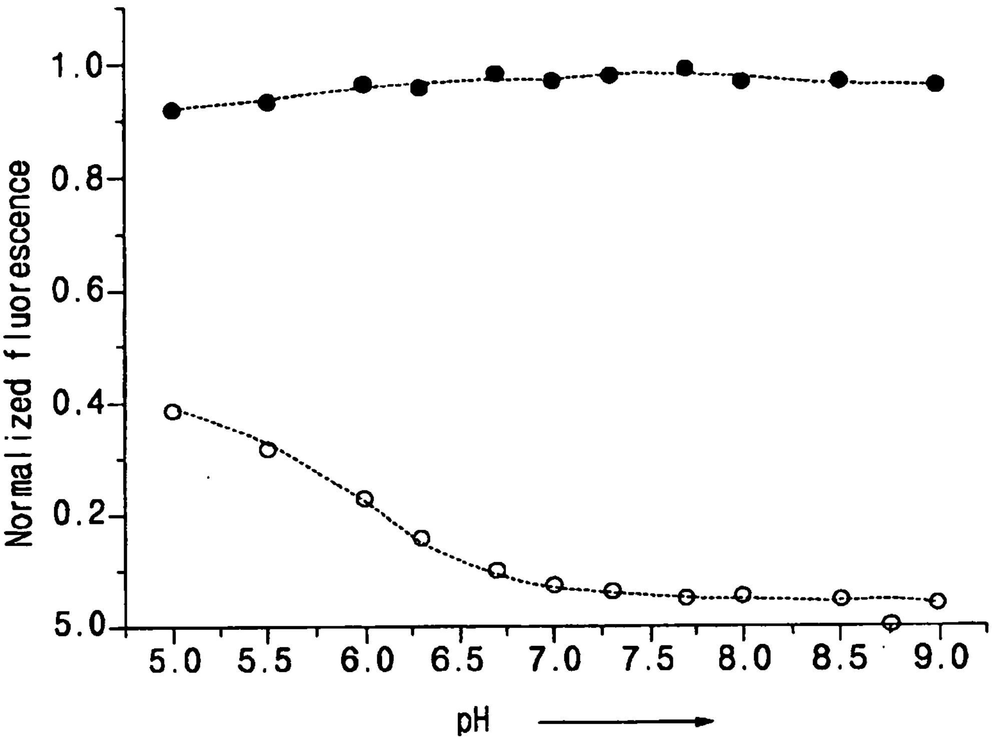
1.0
0.8
0.6
0.4
0.2
5.0
Normalized fluorescence
5.0
5.5
6.0
6.5
7.0
7.5
8.0
8.5
9.0
pH

(b)

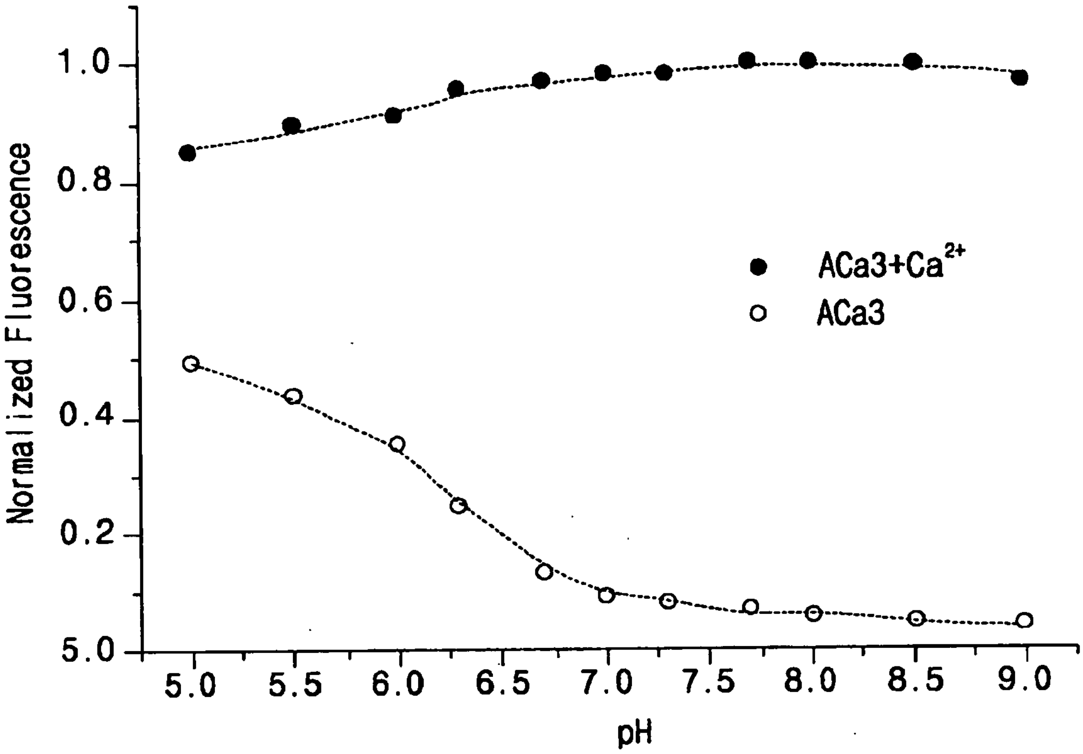
1.0
0.8
0.6
0.4
0.2
5.0
Normalized Fluorescence
ACa3+Ca2+
ACa3
5.0
5.5
6.0
6.5
7.0
7.5
8.0
8.5
9.0
pH

(c)

【Figure 12】

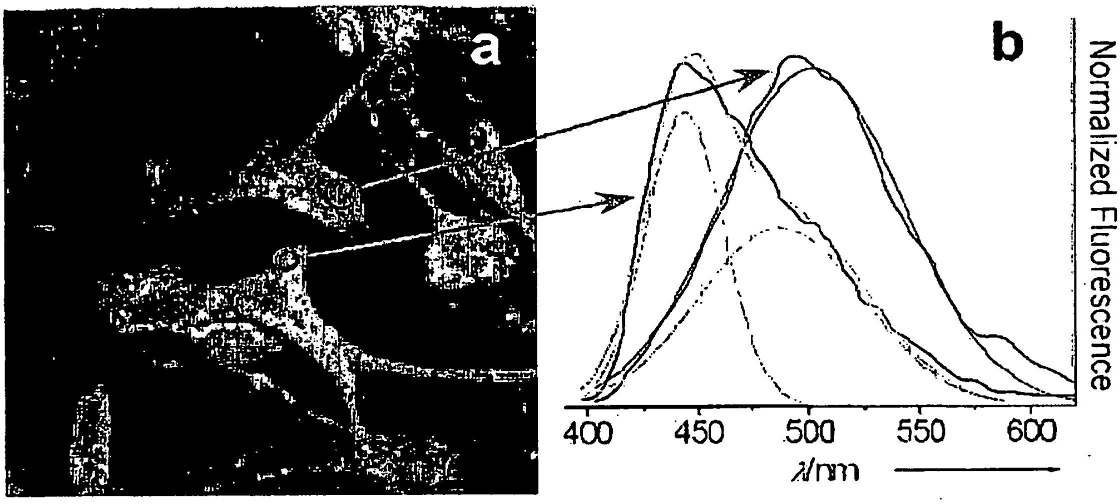
a
b
Normalized Fluorescence
400
450
500
550
600
λ/nm

(a)

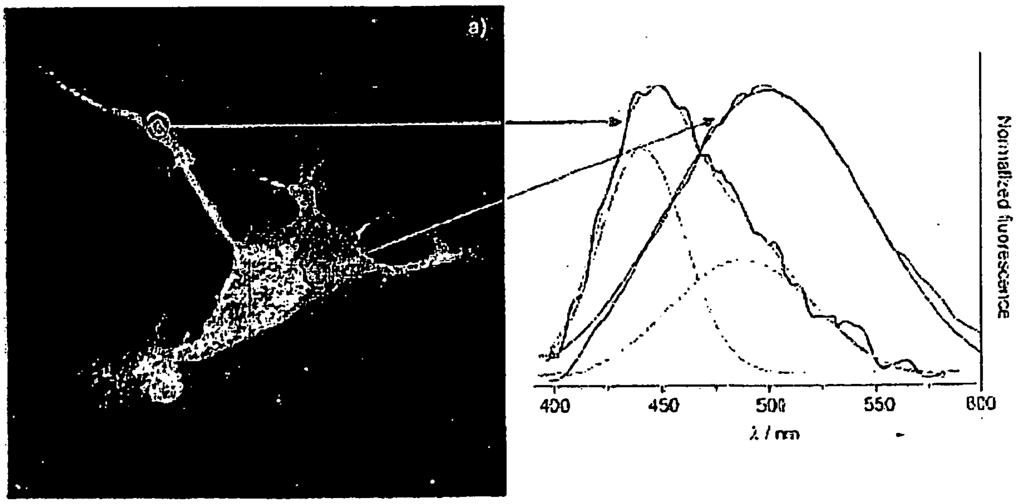
a)
Normalized fluorescence
400
450
500
550
600
λ / nm

(b)

【Figure 13】

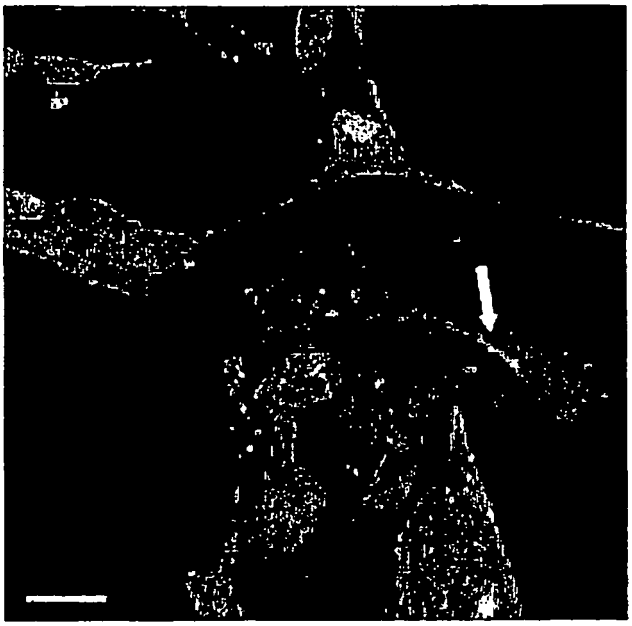

(a)

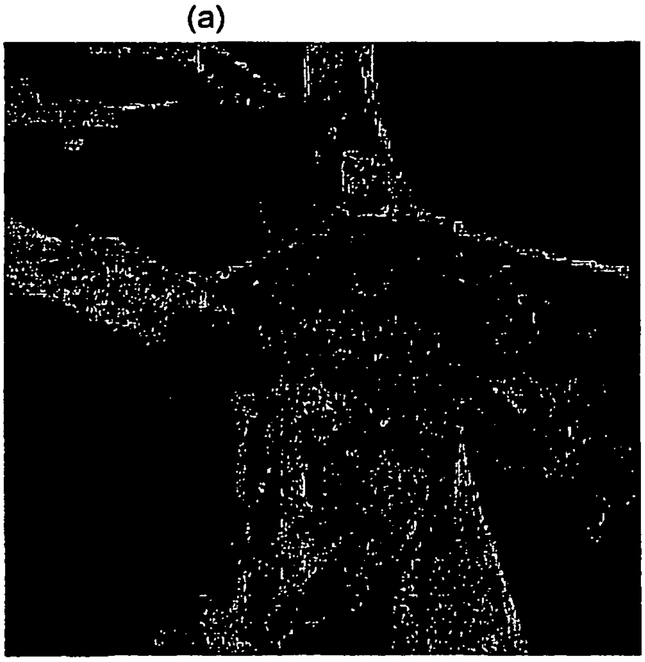

(b)

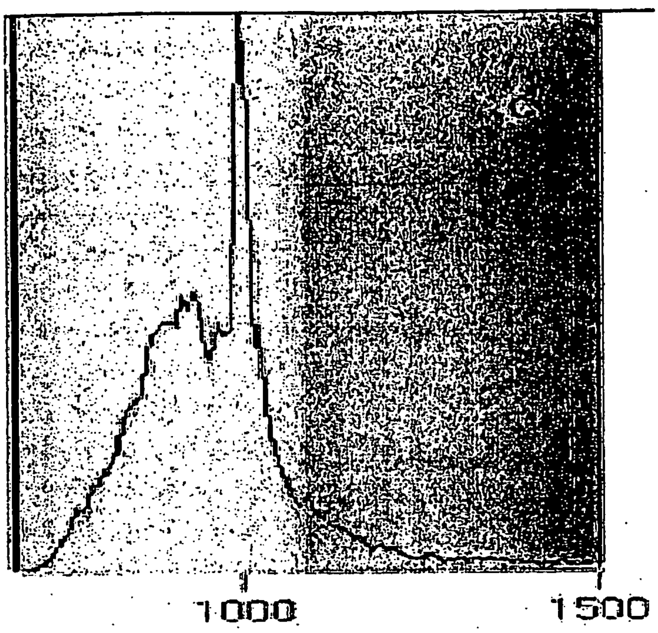

1000
1500


(c)

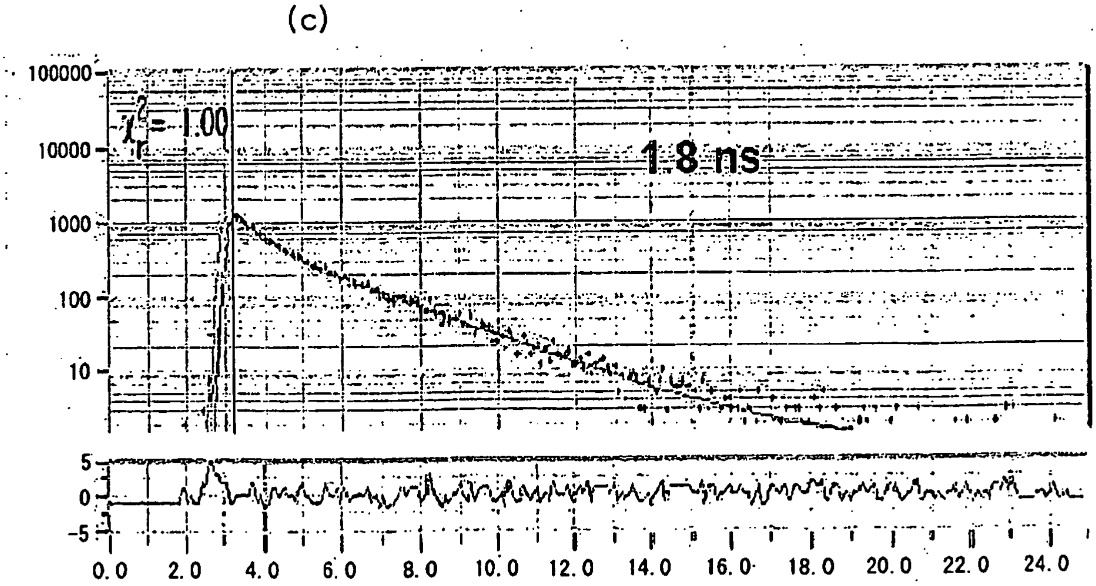

100000
10000
1000
100
10
$\chi_r^2$ = 1.00
1.8 ns
5
0
-5
0.0
2.0
4.0
6.0
8.0
10.0
12.0
14.0
16.0
18.0
20.0
22.0
24.0


(d)

【Figure 14】

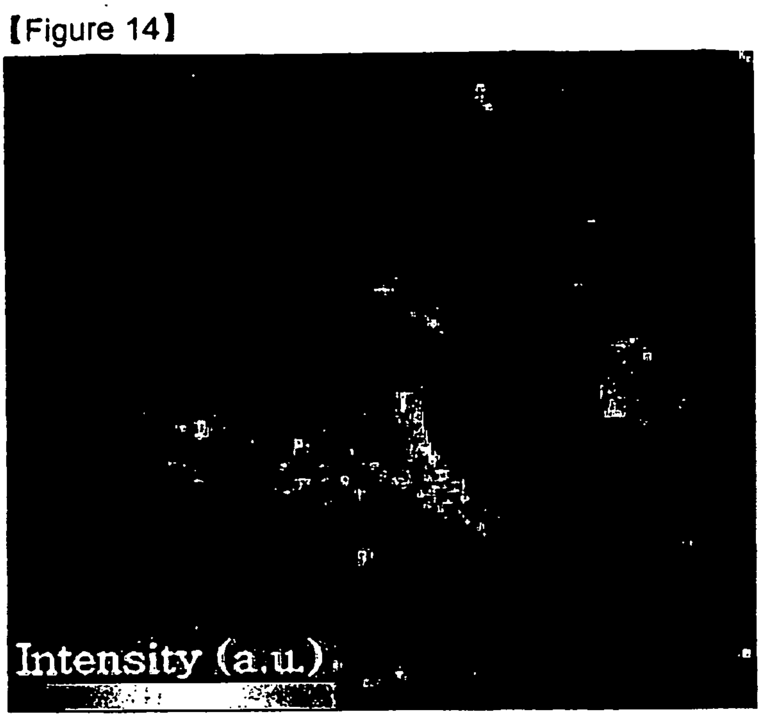


(a)

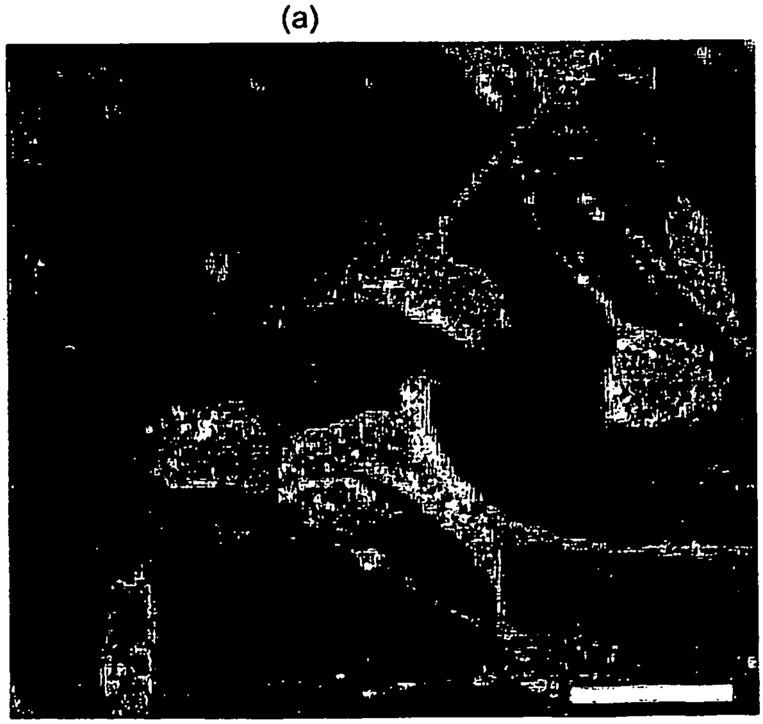

(b)

【Figure 15】

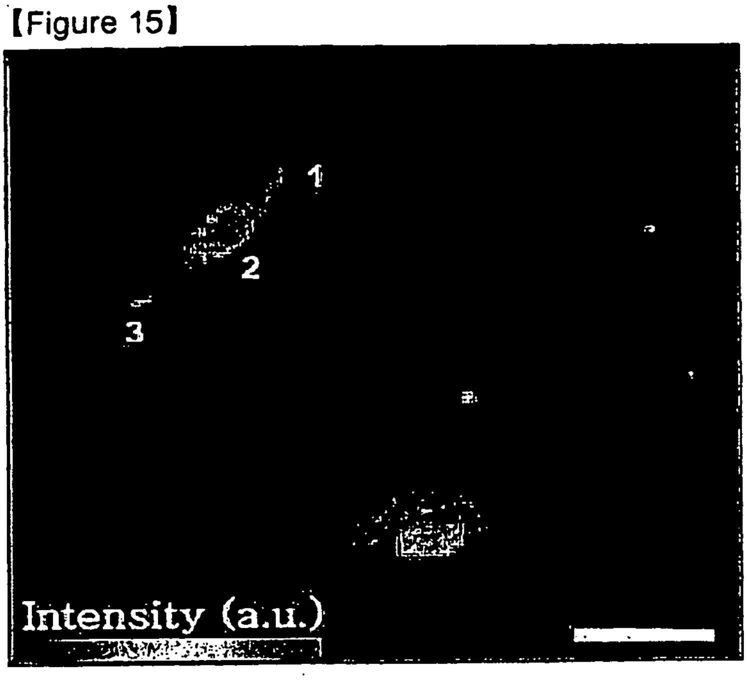


(a)

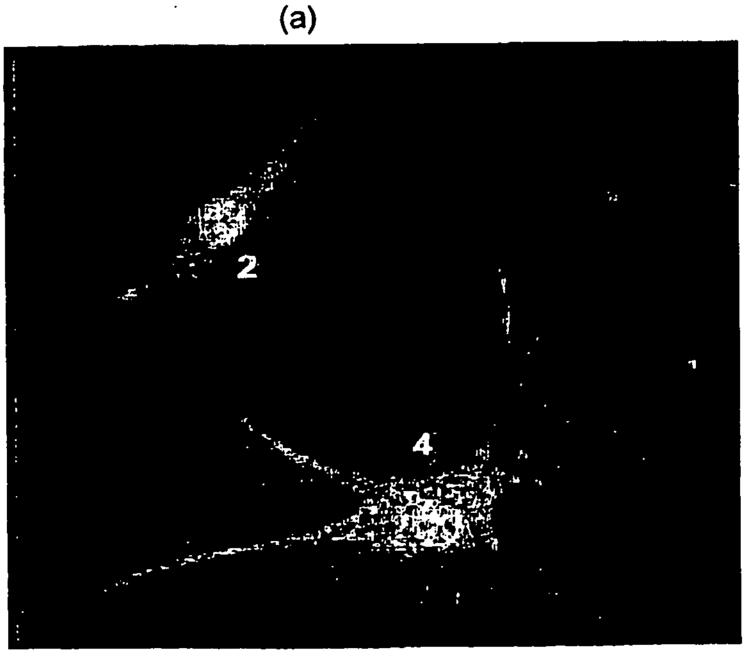


(b)

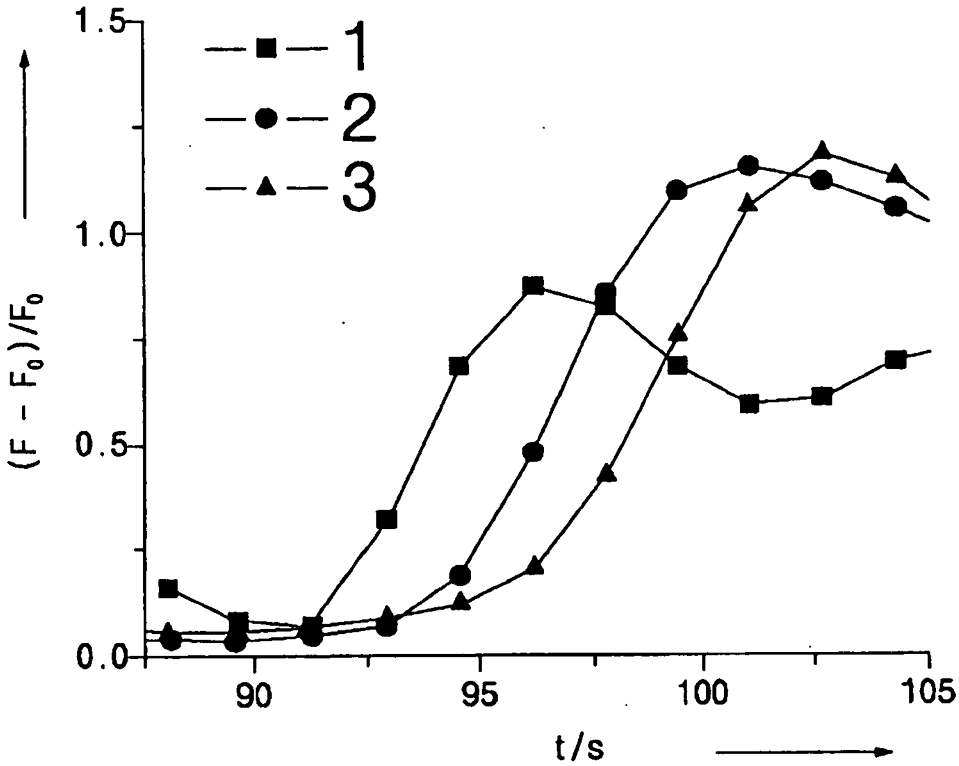


(c)

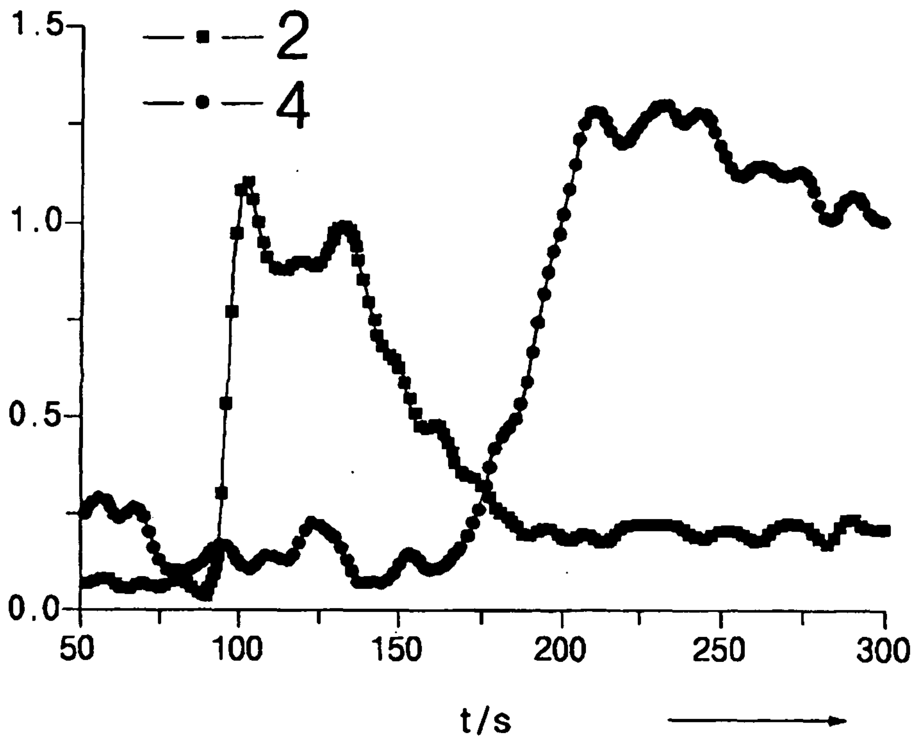


(d)

【Figure 16】

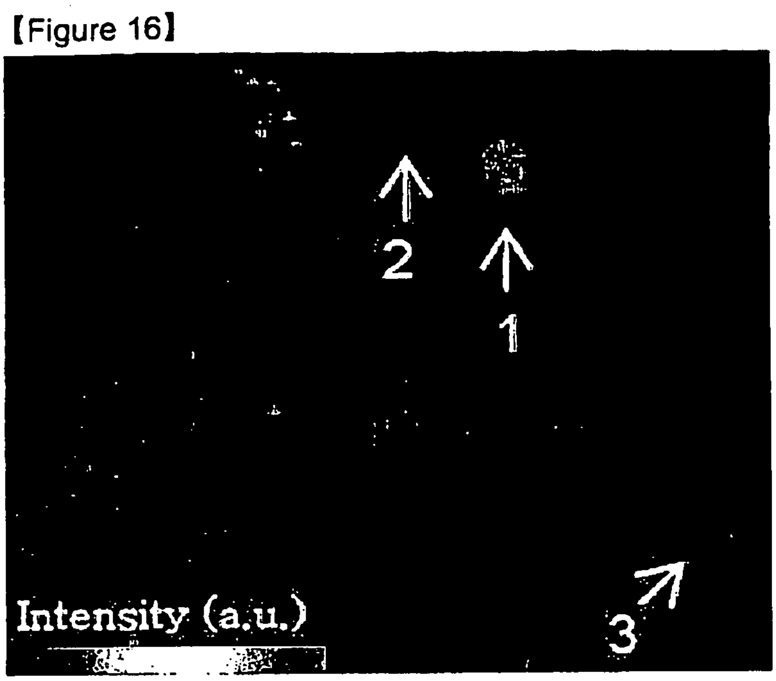


(a)

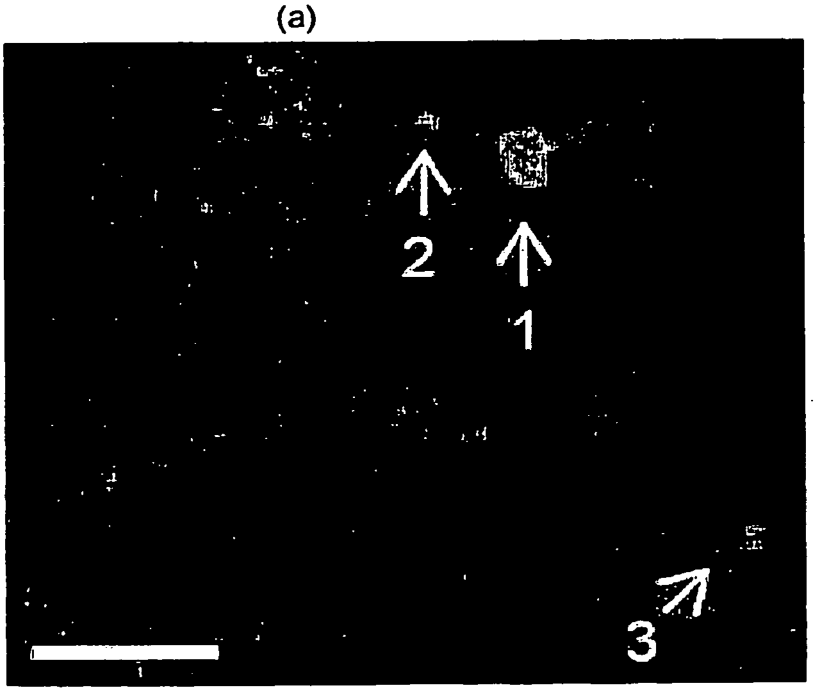


(b)

【Figure 17】

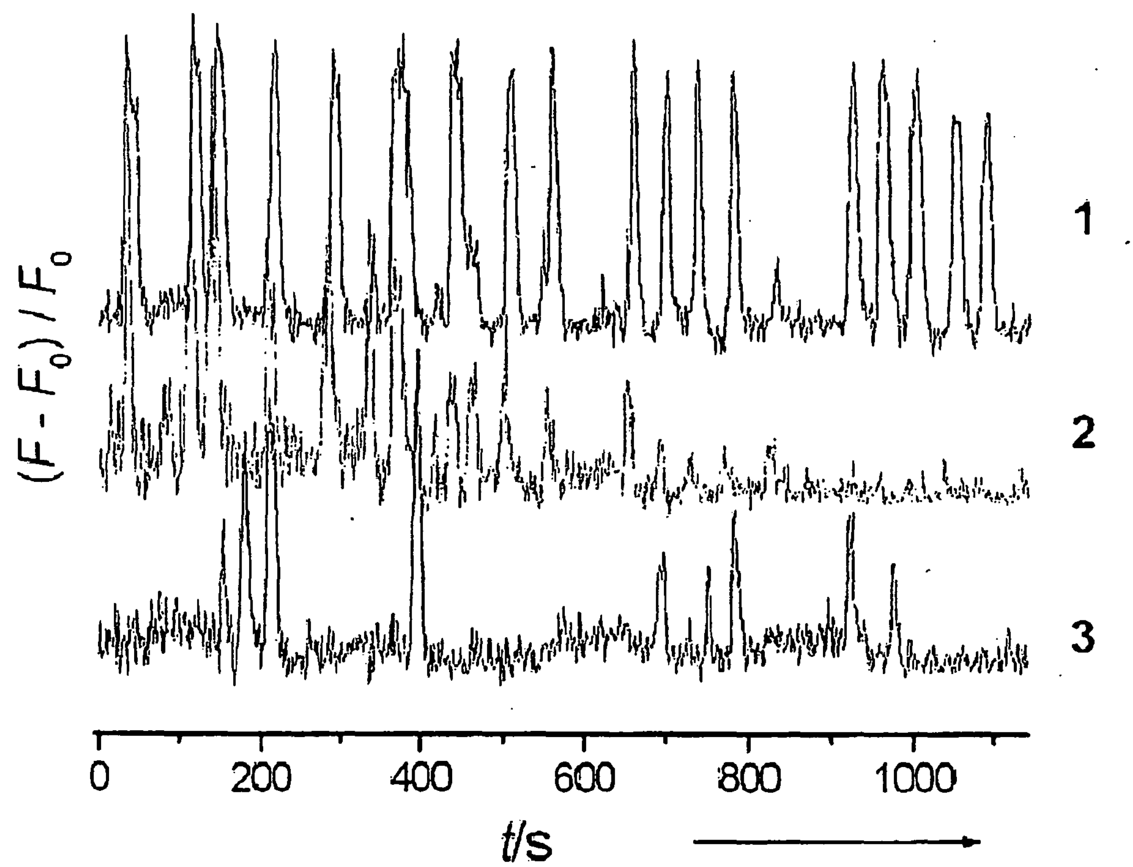

【Figure 18】

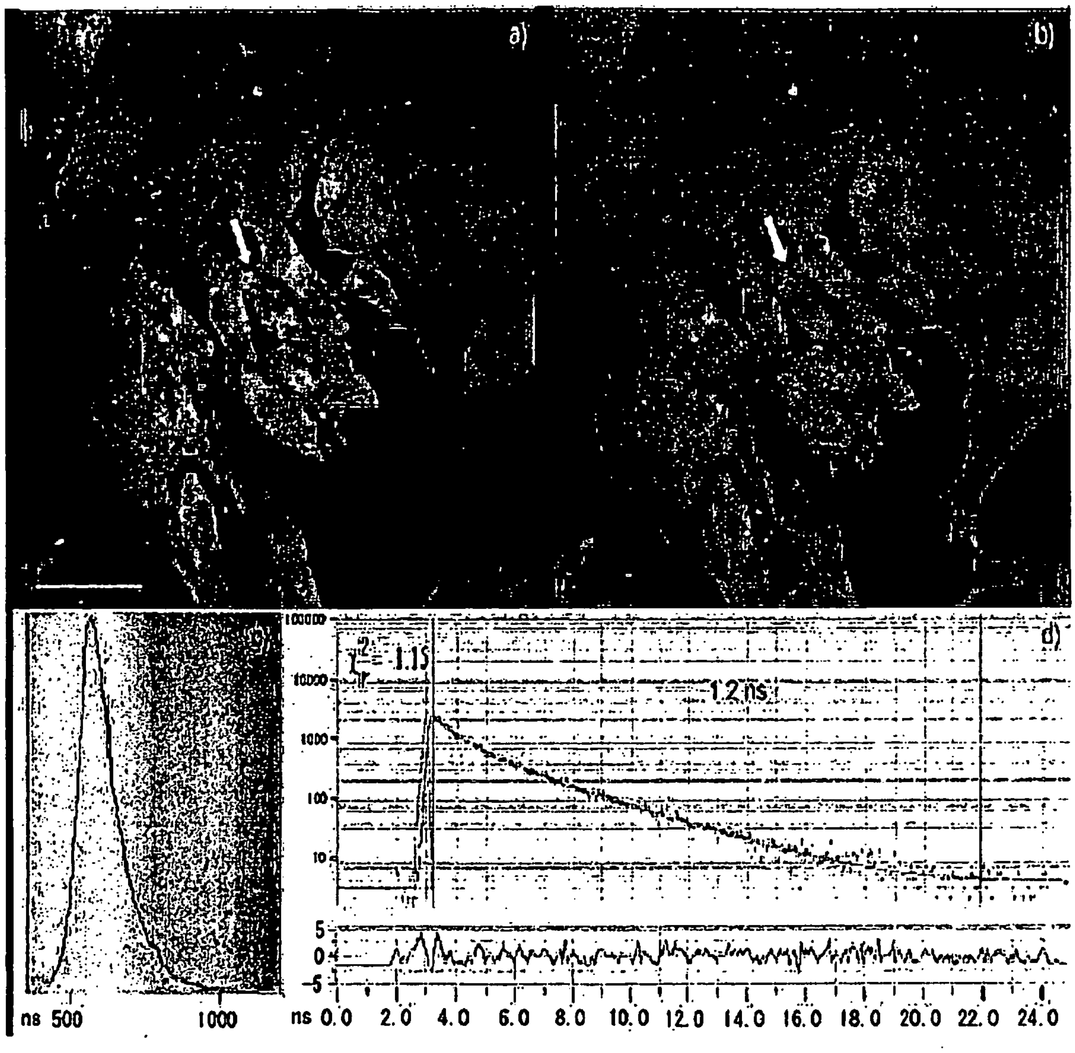
a)
b)
d)
1.2 ns
ns 500
1000
ns 0.0 2.0 4.0 6.0 8.0 10.0 12.0 14.0 16.0 18.0 20.0 22.0 24.0
5
0
-5

【Figure 19】

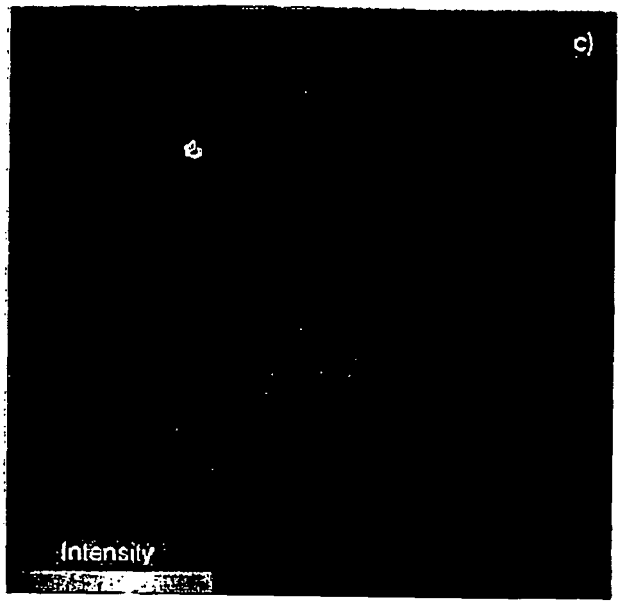


(a)

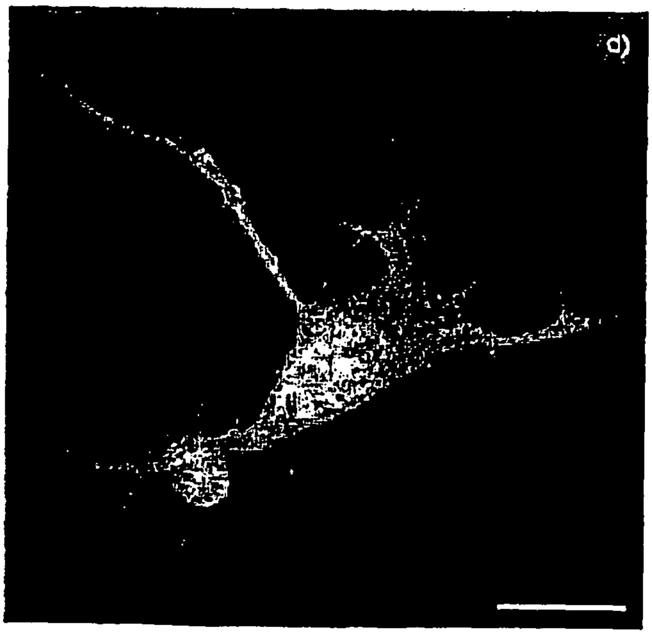


(b)

【Figure 20】

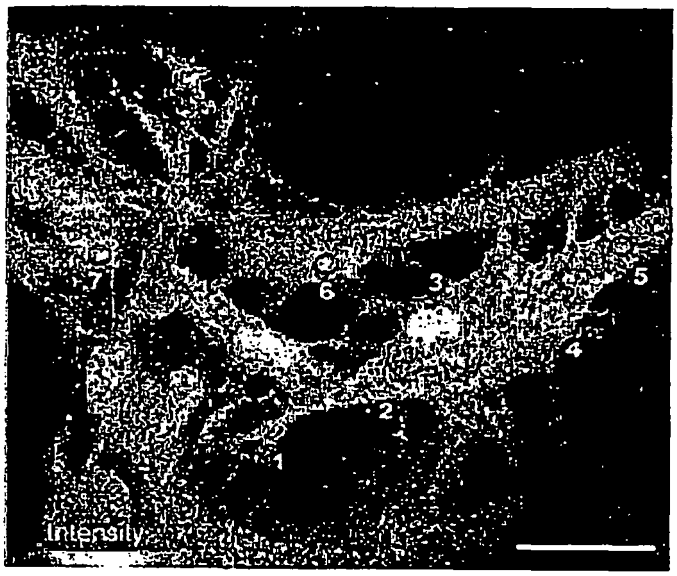


(a)

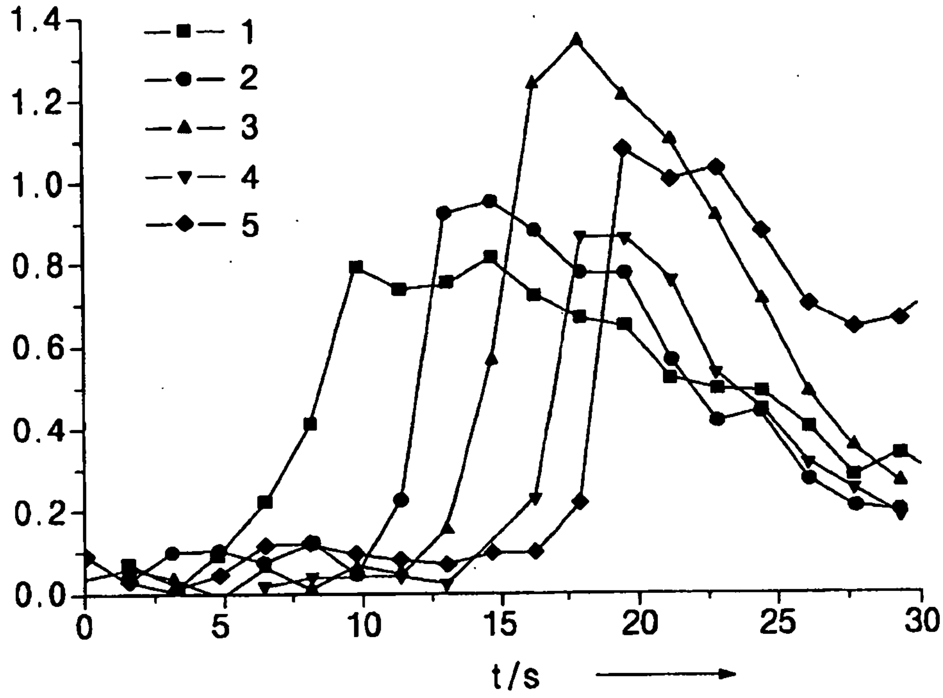


(b)

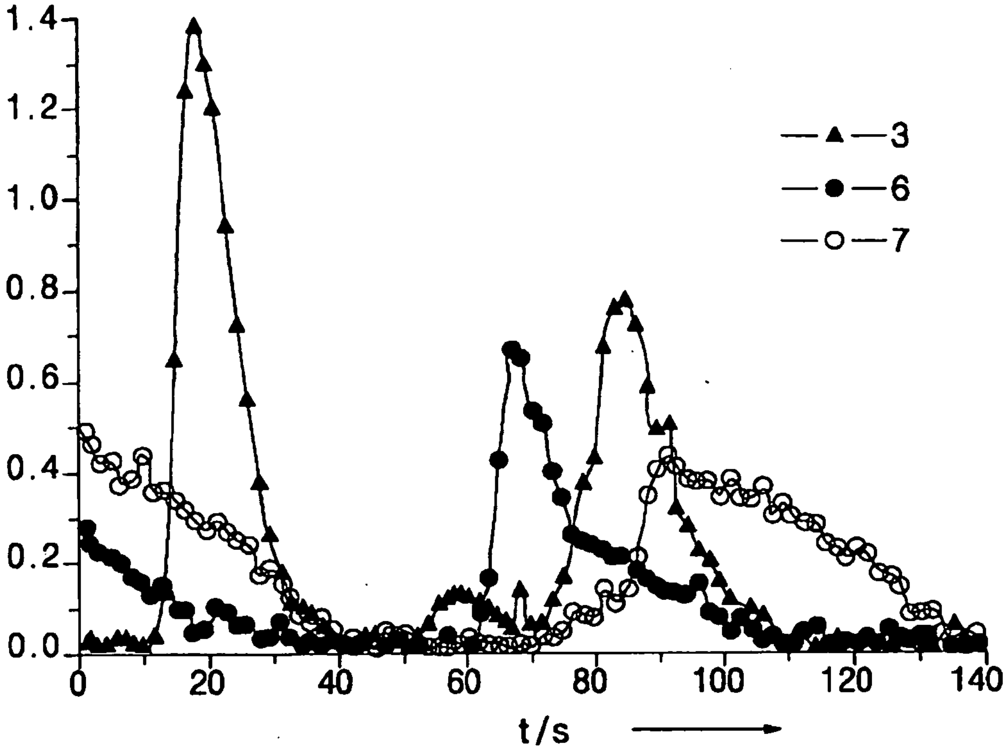


(c)

【Figure 21】

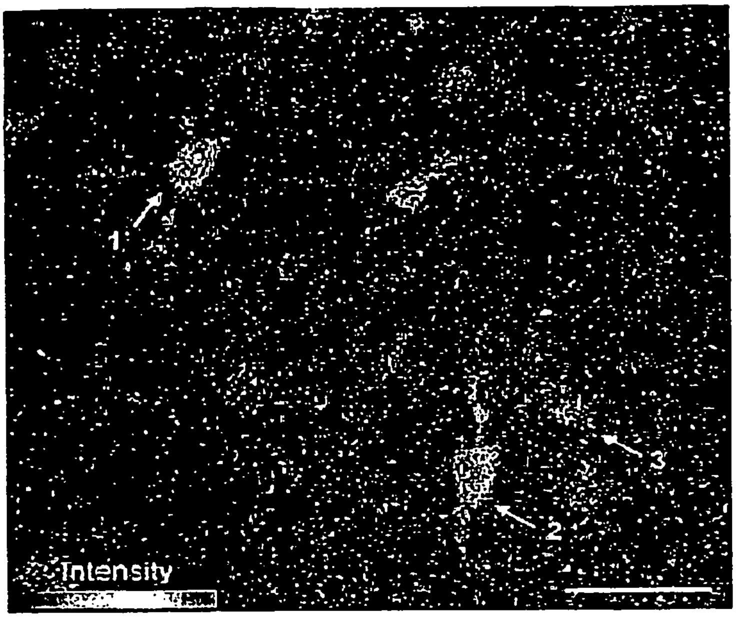


(a)

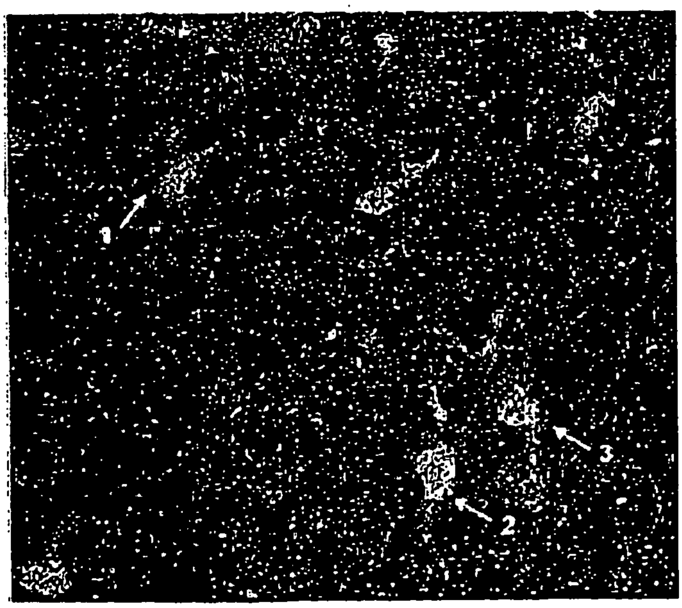


(b)

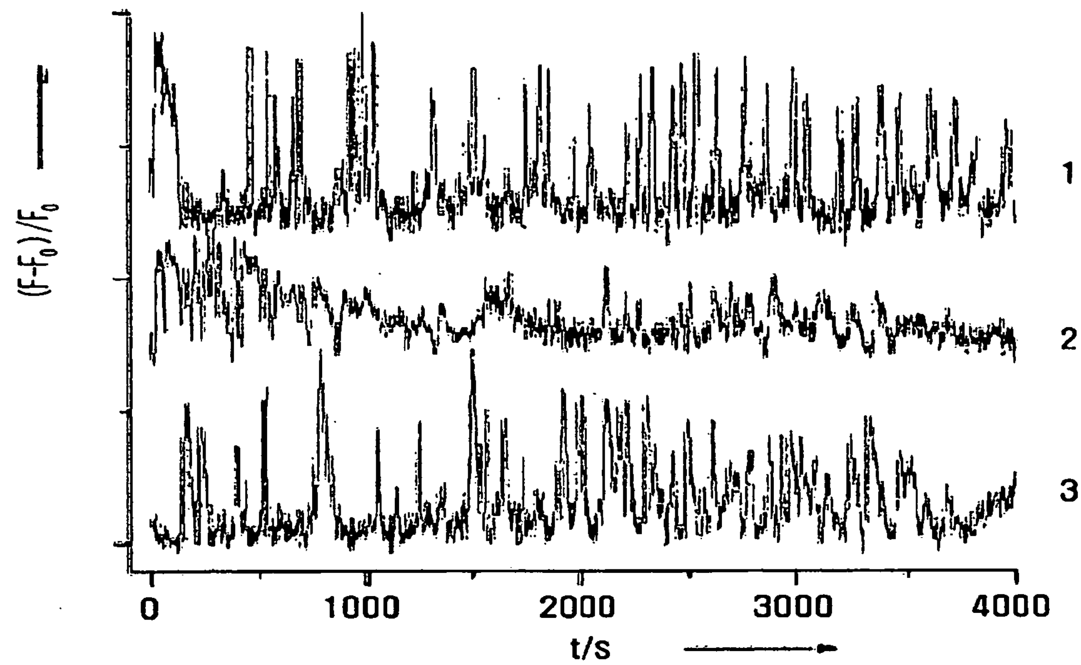


(c)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006024833 A


### Non-patent literature cited in the description


- **HWAN MYUNG KIM et al.** Environment-Sensitive Two-Photon Pobe for Intercellular Free Magnesium Ions in Live tissue. *Angew. Chem. Int. Ed.,* 2007, vol. 46 (19), 3460-3469
- **HWAN MYUNG KIM et al.** A Two-Photon Fluorescent probe for Calcium Waves in Living. *Angew. Chem. Int. Ed.,* 2007, vol. 46 (39), 7445-7448
- **R. PETHIG ; M. KUHN ; R. PAYNE ; E. ADLER ; T.-H. CHEN ; L. F. JAFFE.** *Cell Calcium,* 1989, vol. 10, 491-498
- **H. M. KIM ; C. JUNG ; B. R. KIM ; S.-Y. JUNG ; J. H. HONG ; Y.-G. KO ; K. J. LEE ; B. R. CHO.** *Angew. Chem. Int. Ed.,* 2007, vol. 46, 3460-3463
- **J. N. DEMAS ; G. A. CROSBY.** *J. Phys. Chem.,* 1971, vol. 75, 991-1024
- **J. R. LONG ; R. S. DRAGO.** *J. Chem. Ed.,* 1982, vol. 59, 1037
- **K. HIROSE.** *J. Incl. Phenom. Macrocycl. Chem.,* 2001, vol. 39, 193
- **J. R. LONG ; R. S. DRAGO.** *J. Chem. Educ.,* 1982, vol. 59, 1037-1039
- **C. M. MATIAS ; N. C. MATOS ; M. ARIF ; J. C. DIONISIO ; M. E. QUINTA-FERREIRA.** *Biol. Res.,* 2006, vol. 39, 521-530
- **M. E. QUINTA-FERREIRA ; C. M. MATIAS.** *Brain Res.,* 2004, vol. 1004, 52-60
- **J. Y. KOH ; S. W. SUH ; B. J. GWAG ; Y. Y. HE ; C. Y. HSU ; D. W. CHOI.** *Science,* 1996, vol. 272, 1013-1016
- **H. R. PARRI ; T. M. GOULD ; V. CRUNELLI.** *Nat. Neurosci.,* 2001, vol. 4, 803-812